# EUROPÄISCHE PATENTSCHRIFT

(11) **EP 1 497 279 B1**
(45) Veröffentlichungstag und Bekanntmachung des Hinweises auf die Patenterteilung: **09.03.2011**
(21) Anmeldenummer: 03720455.9
(22) Anmeldetag: 11.04.2003
(51) Int. Cl.: C07D 401/06, A61K 31/404, A61P 43/00, C07D 405/12, C07D 209/42, C07D 471/04, C07D 417/12, C07D 401/12, C07D 401/14

(54) **SUBSTITUIERTE INDOLE UND DEREN VERWENDUNG ALS 5HT-WIEDERAUFNAHME INHIBITOREN UND ALS 5HT LIGANDEN**
SUBSTITUTED INDOLES AND THEIR USE AS 5HT-REUPTAKE INHIBITORS AND AS 5HT LIGANDS
INDOLES SUBSTITUES ET LEUR UTILISATION COMME INHIBITEURS DE RECAPTURE 5HT ET COMME LIGANDS 5HT

(30) Priorität: 16.04.2002 DE 10217006
(43) Veröffentlichungstag der Anmeldung: 19.01.2005
(73) Patentinhaber: Merck Patent GmbH, 64293 Darmstadt (DE)
(72) Erfinder: SCHADT, Oliver, 63517 Rodenbach (DE); BÖTTCHER, Henning, 64287 Darmstadt (DE); LEIBROCK, Joachim, 64319 Pfungstadt (DE); SCHIEMANN, Kai, 64342 Seeheim-Jugenheim (DE); HEINRICH, Timo, 64823 Gross-Umstadt (DE); HÖLZEMANN, Günter, 64342 Seeheim-Jugenheim (DE); VAN AMSTERDAM, Christoph, 64295 Darmstadt (DE); BARTOSZYK, Gerd, 64331 Weiterstadt (DE); SEYFRIED, Christoph, 64342 Seeheim-Jugenheim (DE)
(86) Internationale Anmeldenummer: PCT/EP2003/003806
(87) Internationale Veröffentlichungsnummer: WO 2003/087086

(56) Entgegenhaltungen:
- EP-A- 0 496 222
- EP-A- 0 709 384
- EP-A- 0 952 154
- WO-A-96/03400
- Beilstein Datenbank, Beilstein Registry Nummern 121327, 395771, 395821, 3540022, 3541195, 3542111, 6329287, 6329720, 6644293, 7812825, 7814418, 7827556, 8764267, 8765991, 8767774.
- Beilstein Datenbank, Beilstein Registry Nummern 4802458, 140357.

## Beschreibung

Die Erfindung betrifft substituierte Indole der Formel Ia
Verbindungen der Formel la 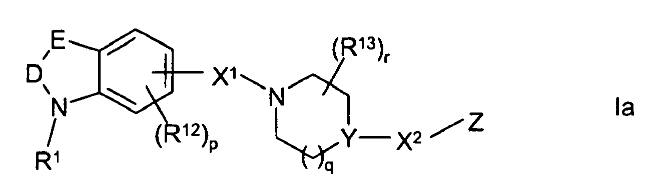 worin
- R¹: für H, A oder SO₂A,
- A: für geradkettiges Alkyl mit 1 bis 4 C-Atomen oder verzweigtes Alkyl mit 3 bis 6 C-Atomen, und
- D-E: für R²C=CR⁴ steht, worin R² ausgewählt ist unter H, A und Cycloalkyl mit 3 bis 7 C-Atomen, und R⁴ für Hal, CH₂Hal, CH(Hal)₂, C(Hal)₃, NO₂, (CH₂)ₙCN, (CH₂)ₙCOOR⁶, (CH₂)ₙCON(R⁶)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙS(O)_{w}A, (CH₂)ₙOOCR⁶ (CH₂)ₙCOR⁶, (CH₂)ₙCO(CH₂)ₘAr, (CH₂)ₙCO(CH₂)ₘHet, (CH₂)ₙCOO(CH₂)ₘAr, (CH₂)ₙCOO(CH₂)ₘHet, (CH₂)ₙOR⁶, (CH₂)ₙO(CH₂)ₘAr, (CH₂)ₙO(CH₂)ₘHet, (CH₂)ₙSR⁶ (CH₂)ₙS(CH₂)ₘAr, (CH₂)ₙS(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂)ₘAr, (CH₂)ₙN(R⁶)CO(CH₂)ₘAr, (CH₂)ₙCON(R⁶)(CH₂)ₘHet, (CH₂)ₙN(R⁶)CO(CH₂)ₘHet, (CH₂)ₙN(R⁶)₂, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, (CH₂)ₙN(COOR⁶)COOR⁶, (CH₂)ₙN(CONH₂)COOR⁶, (CH₂)nN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)nCHR⁶COR⁶, (CH₂)ₙCHR⁶COOR⁶ oder (CH₂)ₙCHR⁶CH₂OR⁶ steht, worin
- Het: für einen unsubstituierten oder einfach oder mehrfach durch A, Hal, NO₂, CN, OR⁶, N(R⁶)_{2,} COOR⁶, CON(R⁶)_{2,} NR⁶COR⁶, NR⁶CON(R⁶)₂, NR⁶SO₂A, COR⁶, SO₂N(R⁶)_{2,} S(O)_{w}A und/oder OOCR⁶ substituierten, gesättigten, ungesättigten oder aromatischen mono- oder bicyclischen heterocyclischen Rest steht,
- Ar: für einen unsubstituierten oder einfach oder mehrfach durch A, Hal, NO₂, CN, OR⁶, N(R⁶)₂, COOR⁶, CON(R⁶)_{2,} NR⁶COR⁶, NR⁶CON(R⁶)₂, NR⁶SO₂A, COR⁶, SO₂N(R⁶)₂, S(O)_{w}A und/oder OOCR⁶ substituierten aromatischen Kohlenwas- serstofferest mit 6 bis 14 Kohlenstoffatomen steht,
- w: für 0, 1, 2 oder 3, und
- m: für 0, 1, 2, 3, 4 oder 5 steht und
- n: für 0, 1, 2 oder 3 steht;
- X¹: für (CHR⁷)_{g}, oder (CHR⁷)ₕ-Q-(CHR⁸)ₖ steht, worin
- Q: ausgewählt ist unter O, S, N-R⁶, (O-CHR⁷)_{g}, (CHR⁷-O)g, CR⁹=CR¹⁰, (O-CHR⁹CHR¹⁰)_{g}, (CHR⁹CHR¹⁰-O)_{g}, C=O, C=S, C=NR⁶, CH(OR⁶), C(OR⁶)(OR⁶), C(=O)O, OC(=O), OC(=O)O, C(=O)N(R⁶), N(R⁶)C(=O), OC(=O)N(R⁶), N(R⁶)C(=O)O, CH=N-O, CH=N-NR⁶, OC(O)NR⁶, NR⁶C(O)O, S=O, SO₂, SO₂NR⁶ und NR⁶SO₂,
- g: für 1, 2, 3, 4, 5 oder 6 steht,
- h: für 0, 1, 2, 3, 4, 5 oder 6 steht,
- k: für 1, 2 oder 3 steht, und
- R⁶: unabhängig ausgewählt ist unter H, A oder Cycloalkyl mit 3 bis 7 C-Atomen,
- R⁷, R⁸, R⁹ und R¹⁰: unabhängig ausgewählt sind unter für den für R² und R⁴ angegebenen Bedeutungen;
- Y: für CH, N, COR¹¹, CSR¹¹, einen unsubstituierten oder substi- tuierten spiro-verknüpften Carbocyclus mit 5 bis 7 Kohlen- stoffatomen oder einen unsubstituierten oder substituierten spiro-verknüpften Heterocyclus, ausgewählt unter Strukturen der Formeln 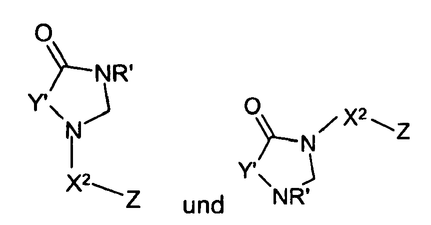 worin R' für H, A, (CH₂)ₙHet, (CH₂)ₙ Ar, Cycloalkyl mit 3 bis 7 C-Atomen oder SO₂A steht, und Y' für das spiro- verknüpfende C-Atom des spiro-verknüpften Heterocyclus steht, steht,
- R¹¹: für H, A, (CH₂)ₙHet, (CH₂)ₙAr oder Cycloalkyl mit 3 bis 7 C-Atomen steht,
- X²: für eine Bindung steht oder unabhängig unter den für X¹ an- gegebenen Bedeutungen ausgewählt ist, und bevorzugt für eine Bindung oder O, S, N-R⁷, CH₂ oder CH₂CH₂ steht,
- p, q, r: unabhängig voneinander für 0, 1, 2 oder 3
und
- Hal: für F, Cl, Br oder I steht, und
- R¹², R¹³: unabhängig voneinander ausgewählt sind unter den von H verschiedenen Bedeutungen von R⁴,
- Z: H bedeutet oder für einen gesättigten, ein- oder mehrfach ethylenisch ungesättigten Carbocyclus mit 5 bis 10 C- Atomen, einen aromatischen Carbocyclus, ausgewählt unter Phenyl und Naphthyl, oder einen gesättigten, ein- oder mehr- fach ethylenisch ungesättigten oder aromatischen Heterocyc- lus, ausgewählt unter Furanyl, Thiophenyl, Pyrrolyl, Imidazo- lyl, Pyrazolyl, Triazolyl, Tetrazolyl, Thiazolyl, Oxazolyl, Isoxa- zolyl, Oxadiazolyl, Oxopyridyl, Thiadiazolyl, Isothiazolyl, Pyri- dyl, Pyridazyl, Pyrazinyl, Pyrimidyl, Chinolinyl, Isochinolinyl, Benzofuranyl, Benzothiophenyl, Benzo[1,4]dioxoinyl, 2,3-Dihydrobenzo[1,4]dioxinyl, Benzothiadiazolyl, Chromenyl, 2-Oxo-chromenyl, Indolyl und Indazolyl, steht ,wobei der Car- bocyclus oder Heterocyclus ein- oder mehrfach substituiert sein kann, wobei die Substituenten unabhängig voneinander ausgewählt sind unter, A, Cycloalkyl mit 3 bis 7 C-Atomen, Hal, CH₂Hal, CH(Hal)₂, C(Hal)₃, NO₂, (CH₂)ₙCN, (CH₂)ₙN(R⁶)₂, (CH₂)ₙN(R⁶)Ar, (CH₂)ₙN(R⁶)Het, (CH₂)ₙN(Ar)₂, (CH₂)ₙN(Het)₂, (CH₂)ₙCOOR⁶, (CH₂)ₙCOOAr, (CH₂)ₙCOOHet, (CH₂)nCON(R⁶)2, (CH₂)ₙCON(R⁶)Ar, (CH₂)ₙCON(R⁶)Het, (CH₂)nCON(Ar)2, (CH₂)ₙCON(Het)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙSO₂NR⁶(CH₂)ₘAr, (CH₂)ₙSO₂NR⁶(CH₂)ₘHet, (CH₂)ₙS(O)_{w}R⁶ (CH₂)ₙS(O)_{w}Ar, (CH₂)S(O)_{w}Het, (CH₂)ₙOOR⁶ (CH₂)ₙHet, (CH₂)ₙAr, (CH₂)ₙCOR⁶ (CH₂)ₙCO(CH₂)ₘAr, (CH₂)ₙCO(CH₂)ₘHet, (CH₂)ₙCOO(CH₂)ₘAr, (CH₂)ₙCOO(CH₂)ₘHet, (CH₂)ₙOR⁶, (CH₂)ₙO(CH₂)ₘAr,(CH₂)ₙO(CH₂)ₘHet, (CH₂)ₙSR⁶ (CH₂)ₙS(CH₂)ₘAr, (CH₂)ₙS(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂)ₘAr, (CH₂)ₙN(R⁶)CO(CH₂)ₘAr, (CH₂)ₙCON(R⁶)(CH₂)ₘHet, (CH₂)ₙN(R⁶)CO(CH₂)ₘHet, CH=N-OA, CH₂CH=N-OA, (CH₂)ₙNHOA, (CH₂)ₙCH=N-Het, (CH₂)ₙOCOR⁶ (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, (CH₂)ₙN(R⁶)CH₂CH₂OR, (CH₂)ₙN(R⁶)CH₂CH₂OCF₃ (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, CH=CHCH₂NR⁶Het, CH=CHCH₂N(R⁶)₂, CH=CHCH₂OR , (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶, (CH₂)ₙCHR⁶CH₂OR⁶, (CH₂)ₙOCN und (CH₂)ₙNCO,
sowie die pharmazeutisch verwendbaren Salze, Solvate und Stereoisomere und Mischungen davon.

Benzylpiperidinderivate mit hoher Affinität zu Bindungsstellen von Aminosäure-Rezeptoren sind z.B. aus der EP 0 709 384 A1 bekannt.

Die EP 0 496 222 A1 beschreibt Indol-3-yl-Derivate und ihre Salze, die Wirkungen auf das Zentralnervensystem haben sollen.

Der Erfindung lag die Aufgabe zugrunde, neue Verbindungen mit wertvollen Eigenschaften aufzufinden, insbesondere solche, die ein verbessertes Wirkprofil aufweisen, zum Beispiel eine höhere Wirksamkeit, eine höhere Selektivität oder ein breiteres Anwendungsprofil und/oder geringere Nebenwirkungen. Die neue Verbindungen sollen vorzugsweise einfach und kostengünstig herstellbar sein und sich insbesondere zur Herstellung von Arzneimitteln eignen.

Überraschend wurde gefunden, dass die Aufgabe durch die Verbindungen der Formel Ia gelöst wird. Insbesondere wurde gefunden, daß die Verbindungen der Formel Ia und ihre Salze bei guter Verträglichkeit sehr wertvolle pharmakologische Eigenschaften besitzen.

Die erfindungsgemäßen Verbindungen zeigen besondere Wirkungen auf das Zentralnervensystem, vor allem 5HT-Wiederaufnahme hemmende und 5 HTₓ-agonistische und/oder -antagonistische Wirkungen, wobei unter HTₓ vorzugsweise HT_{LA}, HT_{1D}, HT_{2A} und/oder HT_{2C} zu verstehen ist.

Da die Verbindungen die Serotonin-Wiederaufnahme hemmen, eignen sie sich insbesondere als Antipsychotika, Neuroleptika, Antidepressiva, Anxiolytika und/oder Antihypertonika. Die Verbindungen zeigen serotonin- agonistische und -antagonistische Eigenschaften. Sie hemmen die Bindung von tritiierten Serotoninliganden an hippocampale Rezeptoren (Cossery et al., European J. Pharmacol. 140 (1987), 143-155) und die synaptosomale Serotoninwiederaufnahme (Sherman et al., Life Sci. 23 (1978), 1863-1870). Außerdem treten Veränderungen der DOPA-Akkumulation im Striatum und der 5-HT-Akkumulation in verschiedenen Gehirnregionen auf (Seyfried et al., European J. Pharmacol. 160 (1989), 31-41). Die 5-HT_{1A}-antagonistische Wirkung wird in vitro z. B. durch Hemmung der durch 8-OH-DPAT-verursachten Aufhebung der elektrisch induzierten Kontraktion des Meerschweinchenileums nachgewiesen (Fozard und Kilbinger, Br. J. Pharmacol. 86 (1985) 601P). Ex-vivo dient zum Nachweis der 5-HT_{1A}-antagonistischen Wirkung die Hemmung der durch 8-OH-DPAT verminderten 5-HTP-Akkumulation (Seyfried et al., European J. Pharmacol. 160 (1989), 31-41) und die Antagonisierung der durch 8-OH-DPAT- induzierten Effekte im Ultraschallvokalisationstest (DeVry, Psychpharmacol. 121 (1995), 1-26). Zum ex-vivo Nachweis der Serotoninwiederaufnahmehemmung kann die synaptosomale Aufnahmehemmung (Wong et al., Neuropsychopharmacol. 8 (1993), 23-33) und der p-Chloramphetaminantagonismus (Fulleret al., J. Pharmacol. Exp. Ther. 212 (1980), 115-119) herangezogen werden. Weiterhin treten analgetische und blutdrucksenkende Wirkungen auf.

Die Verbindungen sind daher geeignet für die Behandlung von Schizophrenie, kognitiven Defiziten, Angst, Depressionen, Übelkeit, tardiver Dyskinesien, Magen-Darm-Trakt-Störungen, Lernstörungen, altersabhängigen Erinnerungsstörungen, Psychosen und zur positiven Beeinflussung von Zwangsverhalten (OCD) und Essstörungen (z. B. Bulimie). Sie zeigen Wirkungen auf das Zentralnervensystem, vor allem zusätzliche 5-HT_{1A}-agonistische und 5-HT-Reuptake hemmende Wirkungen. Ebenso eignen sie sich zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson.

Die Verbindungen der Formel la eignen sich daher sowohl in der Veterinär- als auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Sind sie jedoch auch geeignet als Arzneimittelwirkstoffe für Anxiolytika, Antidepressiva, Antipsychotika, Neuroleptika, Antihypertonika und/oder zur positiven Beeinflussung von Zwangsverhalten, Schlafstörungen, tardiver Dyskinesien, Lernstörungen, altersabhängiger Erinnerungsstörungen, Essstörungen wie Bulimie und/oder Sexualfunktionsstörungen.

Besonders bevorzugt zeigen die erfindungsgemäßen Verbindungen eine hohe Bioverfügbarkeit und/oder sind in der Lage sind, den Serotoninspiegel im Gehirn deutlich zu erhöhen.

Gegenstand der vorliegenden Erfindung sind daher erfindungsgemäße Verbindungen als Arzneimittel oder Arzneimittelwirkstoff.

Gegenstand der vorliegenden Erfindung ist daher Verwendung der erfindungsgemäßen Verbindungen zur Herstellung eines Medikaments zur Prophylaxe und/oder Therapie von Erkrankungen, bei denen 5HT eine Rolle spielt.

Diese Erkrankungen sind vorzugsweise ausgewählt unter Depressionen, Schlaganfall, cerebrale Ischämien, extrapyramidal-motorischer Nebenwirkungen von Neuroleptika sowie des Morbus Parkinson, Alzheimer Krankheit, amyotrophe Lateralsklerose, Hirn- und Rückenmarkstraumata, Zwangsverhalten, Schlafstörungen, tardiver Dyskinesien, Lernstörungen, altersabhängiger Erinnerungsstörungen, Essstörungen wie Bulimie und/oder Sexualfunktionsstörungen.

Ferner zeigen die erfindungsgemäßen Verbindungen vorzugsweise eine besonders hohe Affinität zu Bindungsstellen von Aminosäure-Rezeptoren, insbesondere zur Ifenprodil-Bindungsstelle am NMDA-Rezeptor (NMDA = N-Methyl-D-aspartat), die die Polyamin-Bindungsstelle allosterisch moduliert.

Der Bindungstest für [³H]-lfenprodit kann nach der Methode von Schoemaker et al., Eur. J. Pharmacol. 176, 249-250 (1990) durchgeführt werden. Die Verbindungen eignen sich zur Behandlung von neurodegenerativen Erkrankungen einschließlich cerebrovaskulären Krankheiten. Ebenso können die neuen Verbindungen als Analgetikum oder Anxiolytikum sowie zur Behandlung von Epilepsie, Schizophrenie, der Alzheimer-, der Parkinson- bzw. der Huntington-Krankheit, cerebralen Ischämien oder Infarkten verwendet werden. Ferner eignen sie sich zur Behandlung von Psychosen, bedingt durch überhöhte Aminosäurespiegel.

Der [³H]-CGP-39653-Bindungstest für die Glutamat-Bindungsstelle des NMDA-Rezeptors kann beispielsweise nach der Methode von M.A.Stills et al., beschrieben in Eur. J. Pharmacol. 192, 19-24 (1991), durchgeführt werden. Der Test für die Glycin-Bindungsstelle des NMDA-Rezeptors ist durchführbar nach der Methode von M.B. Baron et al., beschrieben in Eur. J. Pharmacol. 206, 149-154 (1991).

Die Wirkung gegen Morbus Parkinson, d.h. die Potenzierung des L-DOPAinduzierten kontralateralen Drehens bei hemiparkinsonischen Ratten, ist nach der Methode von U. Ungerstedt und G.W. Arbuthnott, Brain Res. 24, 485 (1970) nachweisbar.

Besonders geeignet ist die Verbindung zur Behandlung oder Prophylaxe von Schlaganfällen sowie zum Schutz vor und zur Behandlung von Hirnödemen und Unterversorgungszuständen des Zentralnervensystems, vor allem Hypoxie oder Anoxie.

Die genannten Wirkungen können außerdem nach den Methoden nachgewiesen oder überprüft werden, wie sie in den folgenden Literaturstellen beschrieben sind:
J.W. McDonald, F.S. Silverstein und M.V. Johnston, Eur. J. Pharmacol. 140, 359 (1987); R. Gill, A.C. Foster und G.N. Woodruff, J. Neurosci. 7, 3343 (1987); J.B. Bederson et al., Stroke, 17, 472-476 (1986); S. Brint et al., J. Cereb. Blood Flow Metab. 8, 474-485 (1988).

Aus den nachfolgend aufgeführten Literaturstellen sind verschiedene Antagonisten bekannt, die verschiedene Bindungsstellen des NMDA-Rezeptors blockieren können:
W. Danysz, C.G. Parsons, I. Bresink und G. Quack, Drug, News & Perspectives 8, 261 (1995), K.R. Gee, Exp. Opin. Invest. Drugs 3, 1021 (1994) und J.J. Kulagowski und L.L. Iversen, J. Med. Chem. 37, 4053 (1994).

lfenprodil und Eliprodil der Formeln IV bzw. V können den NMDA-Rezeptor blockieren, indem sie eine Wechselwirkung mit der modulatorischen Polyamin-Bindungsstelle eingehen (C.J. Carter, K.G.Lloyd, B. Zivkovic und B. Scatton, J. Pharmacol. Exp. Ther. 253, 475 (1990)).

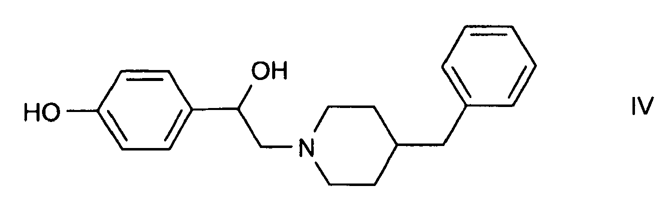

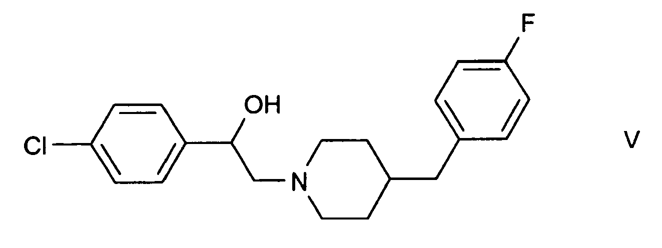

Da Ifenprodil und Eliprodil mit der Polyamin-Bindungsstelle am NMDA-Rezeptor wechselwirken, kann die antagonistische Aktivität der erfindungsgemäßen Verbindungen in einem Spermin-stimulierten [³H]MK-801 (Dizocilpine) - Bindungstest ermittelt werden.

In der Gegenwart von Sättigungskonzentrationen von Glycin und NMDA, kann Spermin noch die Bindung von MK-801 erhöhen, die durch Ifenprodil, Eliprodil und ganz besonders wirksam durch die erfindungsgemäßen Verbindungen inhibiert wird.

Zusätzlich können die erfindungsgemäßen Verbindungen in einem [³H]GABA (y-Aminobuttersäure) - Freisetzungstest, analog J. Dreijer, T. Honoré und A. Schousboe, J. Neurosci. 7, 2910 (1987), der als in-vitro-Modell die antagonistische Funktion in der Zelle beschreibt, getestet werden.

Gegenstand der Offenbarung sind demgemäß die Verbindungen der Formel la nach Anspruch 1 und/oder ihre physiologisch unbedenklichen Salze als Antagonisten an Rezeptoren von exzitatorischen Aminosäuren, wie z.B. Glutaminsäure bzw. deren Salze.

Gegenstand der Offenbarung sind die Verbindungen der Formel Ia nach Anspruch 1 und ihre physiologisch unbedenklichen Salze und Solvate als Glycin-Transporter-Inhibitor.

Gegenstand der Erfindung sind insbesondere die Verbindungen der Formel la nach Anspruch 1 und/oder ihrer unbedenklichen Salze, als exzitatorische Aminosäuren-Antagonisten zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

Gegenstand der Erfindung ist auch die Verwendung der Verbindungen der Formel la nach Anspruch 1 und/oder ihrer physiologisch unbedenklichen Salze zur Herstellung eines Arzneimittels zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

Die Verbindungen der Formel la können als Arzneimittelwirkstoff in der Human- und Veterinärmedizin eingesetzt werden.

Gegenstand der Erfindung ist ferner ein Verfahren zur Herstellung von Verbindungen der Formel la nach Anspruch 1 sowie ihrer Salze, dadurch gekennzeichnet, daß man
a) eine Verbindung der Formel II 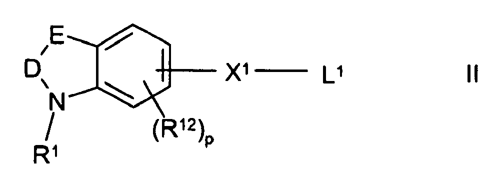 worin
   - L¹: Cl, Br, I, OH, eine reaktionsfähig veresterte OH-Gruppe, ausge- wählt unter Alkylsulfonyloxy-Gruppen mit 1-6 C-Atomen und Arylsulfonyloxy-Gruppen mit 6-10 C-Atomen, oder eine Diazoni- umgruppe bedeutet und R¹, D, E, R¹², p und X¹ die in Anspruch 1 angegebenen Bedeutungen haben,
b) mit einer Verbindung der Formel III umsetzt, 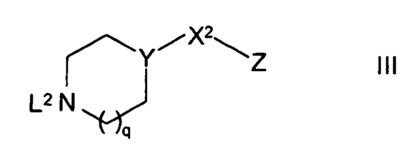 worin
   L² H oder ein Metallion bedeutet und q, Y, X² und Z die in Anspruch 1 angegebenen Bedeutungen haben,
   und gegebenenfalls
c) die erhaltene Verbindung der Formel Ia durch Behandeln mit einer Säure in eines ihrer Salze überführt.

Besonders bevorzugt ist die Verbindung der Formel VI ausgewählt unter Verbindungen der Formeln 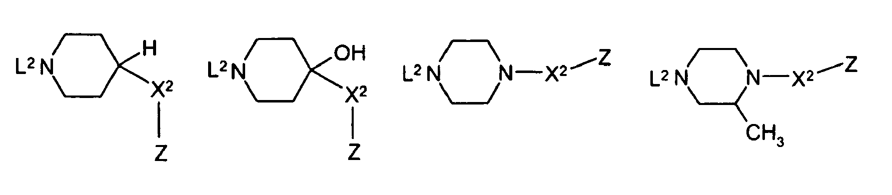 und 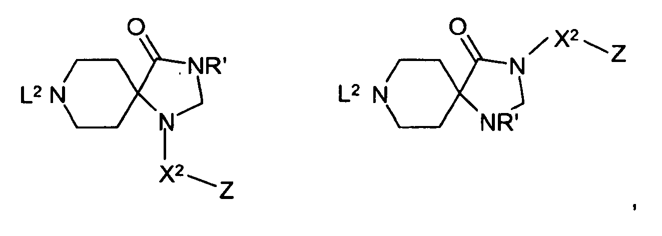 oder den Thioamiden davon,
worin L², X² und Z wie vorstehend und nachstehend definiert sind und R' für H, A, (CH₂)ₙHet, (CH₂)ₙAr, Cycloalkyl mit 3 bis 7 C-Atomen oder SO₂A steht.

Das erfindungsgemäße Verfahren kann im Sinne einer Eintopfreaktion durchgeführt werden, d. h. auf Isolierungs- und/oder Reinigungsschritte wird so weit wie möglich verzichtet und nur das gewünschte Endprodukt, d. h. in der Regel eine erfindungsgemäße Verbindung oder ein pharmazeutisch verwendbares Derivat davon, gereinigt und/oder isoliert. Alternativ kann nach jedem der genannten Reaktionsschritte ein Reinigungs- und/oder Isolierungsschritt durchgeführt werden. Auch gemischte Formen der vorstehend beschriebenen Verfahrensweisen sind denkbar. Geeignete Reinigungs- und Isolierungsschritte sind dem Fachmann bekannt, z. B. aus Houben-Weyl, Methoden der organischen Chemie, Georg-Thieme-Verlag, Stuttgart.

Gegenstand der Erfindung sind insbesondere diejenigen Verbindungen der Formel Ia, in denen mindestens einer der genannten Reste eine der vorstehend angegebenen bevorzugten Bedeutungen hat.

Im Rahmen der vorliegenden Erfindung bedeutet Alkyl einen linearen oder verzweigten Alkylrest, vorzugsweise einen unverzweigten Alkylrest, der 1, 2, 3, 4, 5, 6, 7, 8, 9 oder 10 C-Atome, vorzugsweise 1, 2, 3, 4 oder 5 C-Atome aufweist und ein- oder mehrfach mit Halogen (Hal), z. B. perfluoriert, sein kann. Wenn ein Alkylrest mit Halogen substituiert ist, weist er vorzugsweise, abhängig von der Anzahl der Kohlenstoffatome des Alkylrests, 1-, 2, 3, 4 oder 5 Halogenatome auf. So kann beispielsweise eine Methylgruppe (Alkylrest mit 1 Kohlenstoffatom) 1-, 2- oder 3-fach mit Halogen substituiert sein, und eine Ethylgruppe (Alkylrest mit 2 Kohlenstoffatomen) 1-, 2-, 3-, 4- oder 5-fach mit Halogen substituiert sein.
Für Alkylgruppen mit mehr als 2 Kohlenstoffatomen gilt vorzugsweise das gleiche wie für Ethylgruppen. Besonders bevorzugt steht Alkyl für Methyl, Ethyl, Trifluormethyl, Pentafluorethyl oder Propyl, weiterhin bevorzugt für Isopropyl, Butyl, Isobutyl, sek.-Butyl oder tert.-Butyl, aber auch für n-Pentyl, neo-Pentyl, Isopentyl oder Hexyl.

Der Ausdruck "Alkenyl" umfasst vorzugsweise ein- oder mehrfach ethylenisch ungesättigte, geradkettige oder verzweigte Kohlenwasserstoffereste mit 2 bis 10 und insbesondere 3 bis 6 Kohlenstoffatomen, und insbesondere Allyl, 2- oder 3-Butenyl, Isobutenyl, sek.-Butenyl, ferner bevorzugt ist 4-Pentenyl, iso-Pentenyl oder 5-Hexenyl.

Der Ausdruck "Alkoxy" steht vorzugsweise für Reste der Formel -O-Alkyl, worin Alkyl die vorstehend genannte Bedeutung hat, oder, wenn zwei Alkoxyreste an benachbarte (vicinale) Kohlenstoffatome gebunden sind, für -O-Alkylen-O-, worin Alkylen die vorstehend genannte Bedeutung hat. Bevorzugte Alkoxyreste der Formel -O-Alkyl sind Methoxy, Ethoxy und Propoxy. Bevorzugte Alkoxyreste der Formel -O-Alkylen-O- sind -O-CH₂-O-, -O-CH₂CH₂-O- und -O-CH₂CH₂CH₂-O-.

Der Ausdruck "Alkoxyalkyl" umfaßt vorzugsweise geradkettige Reste der Formel CᵤH₂ᵤ₊₁-O-(CH₂)ᵥ, worin u und v jeweils unabhängig voneinander 1 bis 6 bedeuten. Besonders bevorzugt ist u = 1 und v = 1 bis 4.

Der Ausdruck "Aryl" umfasst vorzugsweise einen unsubstituierten oder ein- oder mehrfach substituierten Benzolring, z.B. einen unsubstituierten oder substituierten Phenylrest oder ein unsubstituiertes oder ein- oder mehrfach substituiertes System aus Benzolringen, wie zum Beispiel Anthracen-, Phenanthren- oder Napthalen-Ringsysteme. Beispiele für geeignete Substituenten umfassen Alkyl-, Alkoxy-, Oxo-, Hydroxy-, Mercapto-, Amino-, Nitro-, Cyano- und Halogen-Reste.

Der Ausdruck "Aryl" umfasst vorzugsweise ein unsubstituiertes oder ein ein- oder mehrfach substituiertens aromatisches Ringsystem, z. B. einen unsubstituierten oder substituierten Phenylrest oder ein unsubstituiertes oder ein- oder mehrfach substituiertes System aus Benzolringen, wie zum Beispiel Anthracen-, Phenanthren- oder Napthalen-Ringsysteme. Beispiele für geeignete Substituenten umfassen Alkyl-, Alkoxy-, Oxo-, Hydroxy-, Mercapto-, Amino-, Nitro-, Cyano- und Halogen-Reste.

Der Ausdruck "Aralkyl" umfasst vorzugsweise einen Arylrest wie obenstehend definiert, verbunden mit einem Alkylrest wie obenstehend definiert. Beispiele für geeignete Aralkylreste umfassen, sind aber nicht beschränkt auf, Benzyl, Phenylpropyl, Phenylbutyl und dergleichen.

Ar steht vorzugsweise für einen unsubstituierten oder ein- oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten Arylrest und insbesondere für unsubstituiertes oder wie vorstehend substituiertes Phenyl, Naphthyl oder Biphenyl.

Het ist vorzugsweise ein unsubstituierter oder einfach oder mehrfach durch A, Hal, NO₂, CN, OR⁶, N(R⁶)₂ COOR⁶, CON(R⁶)₂, NR⁶COR⁶, NR⁶CON(R^{s})₂, NR⁶SO₂A, COR⁶, SO₂NR⁶, S(O)_{w}A und/oder OOCR⁶ substituierten, gesättigter, ungesättigter oder aromatischer mono- oder bicyclischer heterocyclischer Rest. Bevorzugt bedeutet Het unsubstituierten oder wie vorstehend beschrieben substituierten Rest, ausgewählt unter 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-pyrazolidinyl, 1-Imidazolidinyl oder 1-(3-Methyl)-imidazolidinyl, Thiophen-2-yl oder Thiophen-3-yl, 2-, 3- oder 4-Pyridyl, 2-, 4- oder 5-Oxazolyl, 2-, 4-oder 5-Thiazolyl, Chinolinyl, Isochinolinyl, 2- oder 4-Pyridazyl, 2-, 4- oder 5-Pyrimidyl, 2- oder 3-Pyrazinyl.

Der Rest Z steht vorzugsweise für einen 5- oder 6-gliedrigen, mehrfach ethylenisch ungesättigten oder aromatischen Carbocyclus, der ein- oder mehrfach, vorzugsweise 1- bis 3-fach substituiert sein kann, wobei die Substituenten unabhängig voneinander ausgewählt sind unter den von H verschiedenen Bedeutungen von R⁴, oder bevorzugt ausgewählt sind unter A, insbesondere Alkyl mit 1 bis 6 Kohlenstoffatomen, Alkoxy mit 1 bis 6 Kohlenstoffatomen und Alkoxyalkyl mit 2 bis 6 Kohlenstoffatomen, Hal, insbesondere F und Cl, NO₂, OR⁶, N(R⁶)₂, CN, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ und C(NH)NOH. Beispiele für carbocyclische Reste Q sind Cyclopentadienyl, Cyclohexadienyl, Phenyl, Naphthyl, insbesondere 1-Naphthyl und 2-Naphthyl, und Biphenyl, die wie vorstehend/nachstehend beschrieben substituiert sein können. Bevorzugt als carbocyclischer Rest Z ist Phenyl und besonders bevorzugt substituiertes Phenyl, insbesondere 4-Alkylphenyl, wie 4-Tolyl (4-Methylphenyl), 4-Alkoxyphenyl, wie 4-Methoxyphenyl, 3,4-Dialkoxyphenyl, wie 3,4-Dimethoxyphenyl und 3,4-Methylendioxyphenyl, und 4-Halogenphenyl, wie 4-Fluorphenyl und 4-Chlorphenyl.

Alternativ steht der Rest Z vorzugsweise für einen 5- oder 6-gliedrigen, mehrfach ethylenisch ungesättigten oder aromatischen Heterocyclus, der 1 bis 4 Heteroatome, unabhängig voneinander ausgewählt unter N, O und S, enthalten kann und ein- oder mehrfach, vorzugsweise 1- bis 3-fach substituiert sein kann, wobei die Substituenten unabhängig voneinander ausgewählt sind unter den von H verschiedenen Bedeutungen von R⁴, oder bevorzugt ausgewählt sind unter A, insbesondere Alkyl mit 1 bis 6 Kohlenstoffatomen, Alkoxy mit 1 bis 6 Kohlenstoffatomen und Alkoxyalkyl mit 2 bis 6 Kohlenstoffatomen, Hal, insbesondere F und Cl, NO₂, OR⁶, N(R⁶)_{2,} CN, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ und C(NH)NOH. Beispiele für heterocyclische Reste Q sind Furanyl, Thiophenyl, Pyrrolyl, Imidazolyl, Pyrazolyl, Triazolyl, Tetrazolyl, Thiazolyl, Oxazolyl, Isoxazolyl, Oxadiazolyl, Oxopyridyl, Thiadiazolyl, Isothiazolyl, Pyridyl, Pyridazyl, Pyrazinyl, Pyrimidyl, Chinolinyl, Isochinolinyl, Benzofuranyl, Benzothiophenyl, Benzo[1,4]dioxoinyl, 2,3-Dihydrobenzo[1,4]dioxinyl, Benzothiadiazolyl, Chromenyl, 2-Oxo-chromenyl, Indolyl und Indazolyl, die wie vorstehend/nachstehend beschrieben substituiert sein können. Besonders bevorzugt als heterocyclischer Rest Z ist gegebenenfalls substituiertes Furanyl, Thiophenyl, Pyrrolyl, Pyridyl, Pyridazyl, Pyrazolyl, Pyrazinyl, Pyrimidyl, Benzofuranyl, 2-Oxo-chromenyl, Indolyl, Benzothiadiazolyl, Chinolinyl, 2,3-Dihydrobenzo[1,4]dioxinyl und Benzo[d]isothiazolyl.

Gegenstand der vorliegenden Erfindung sind bevorzugt Verbindungen der Formel la wie vorstehend beschrieben, worin
- R¹: H oder SO₂A bedeutet;
- D-E: für R²C=CR⁴ steht,
- R²: H, Alkyl mit 1 bis 3 C-Atomen, Alkoxyalkyl mit 2 bis 5 C- Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ bedeutet;
- R⁴: H, Alkyl mit 1 bis 3 C-Atomen, Alkoxyalkyl mit 2 bis 5 C- Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ und
- R⁶: H oder A bedeutet,
- n: für 0 oder 1 steht,
- X¹: (CHR⁷)_{g} bedeutet, worin g für 2, 3 oder 4 steht, oder Q-(CHR⁸)ₖ bedeutet, worin k für 1, 2 oder 3 steht und Q aus- gewählt ist unter O, S, N-R⁶, CONR⁶, C=O, C=S, S=O, SO₂, SO₂NR⁶ und NR⁶SO₂,
- R⁷, R⁸: unabhängig voneinander ausgewählt sind unter H und A,
- Y: für CH, COR¹¹ oder N steht,
- R¹¹: H oder A, und
- X²: CH₂, CH₂CH₂, HCOH, O, S, N-R⁶, CONR⁶, C=O oder eine Bindung bedeutet, und
- R¹²: unabhängig ausgewählt ist unter A, Hal, CN, NO₂, OR⁶, N(R⁶)₂, COOR⁶, CON(R⁶)₂, COR⁶, SO₂N(R⁶)₂ und S(O)_{w}A.
sowie deren Solvate und Salze.

Vorzugsweise ist die Summe aus n und m größer als Null.

In den Verbindungen der Formel Ia und den Verbindungen der Formel III ist die Gruppe X²-Z vorzugsweise ausgewählt unter den Gruppen 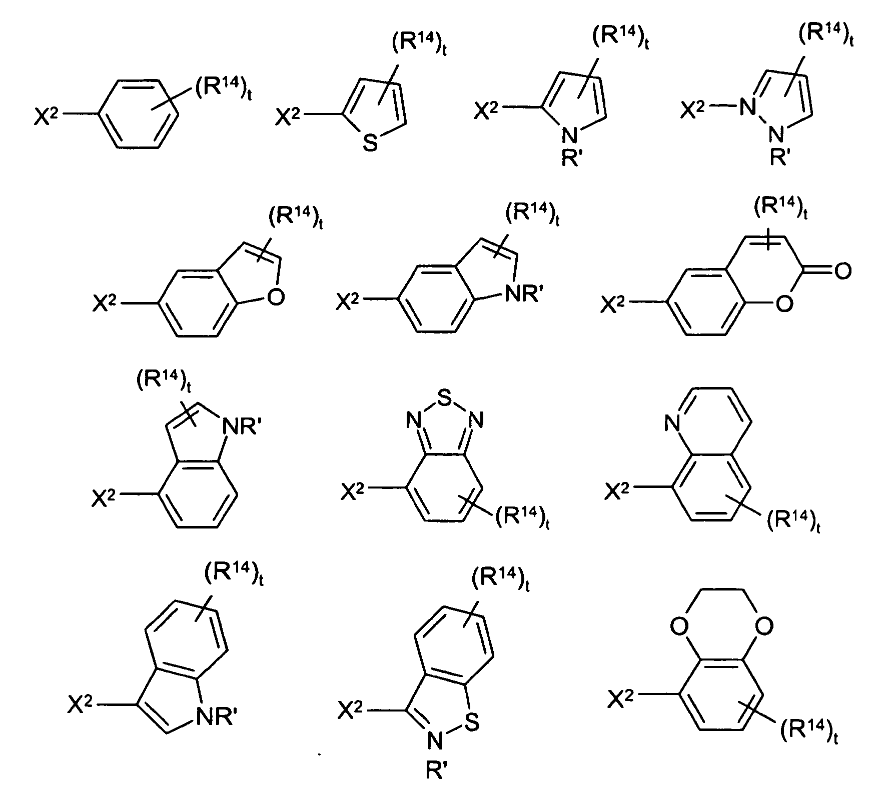 worin
- R¹⁴: unabhängig ausgewählt ist unter Hal, A, (CH₂)ₙHet, (CH₂)ₙAr, (CH₂)ₙCOO(CH₂)ₘAr, (CH₂)ₙCOO(CH₂)ₘHet, (CH₂)ₙOR⁶ (CH₂)ₙO(CH₂)ₘAr, (CH₂)ₙO(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)Ar, CH₂)ₙN(R⁶)SO₂(CH₂)ₘAr, (CH₂)ₘSO₂N(R⁶)(CH₂)ₘHet, (CH₂)ₙN(R⁶)SO₂(CH₂)ₘHet, (CH₂)ₙN(R⁶)₂, (CH₂)ₙNHOA, (CH₂)ₙ(R⁶)Het, (CH₂)ₙOCOR⁶, (CH₂)nOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, und insbesondere unabhängig vonein- ander für Hal, NO₂, OR⁶, N(R⁶)₂, CN, COOR⁶, CON(R⁶)₂, NR⁶COR⁶, NR⁶CON(R^{s})₂, NR⁶SO₂A, COR⁶, SO₂NR⁶, S(O)_{w}A, OOCR⁶ und/oder C(NH)NOH steht,
- w: für 0, 1, 2 oder 3,
- t: für 0, 1, 2, 3, 4 oder 5 und insbesondere 0, 1, 2 oder 3, und
- R': R' für H, A, (CH₂)ₙHet, (CH₂)ₙAr, Cycloalkyl mit 3 bis 7 C-Atomen oder SO₂A steht.

Wenn die Gruppe X²-Z für die Gruppe 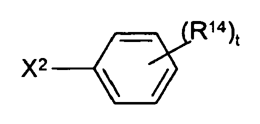 steht, ist sie vorzugsweise ausgewählt unter den Gruppen 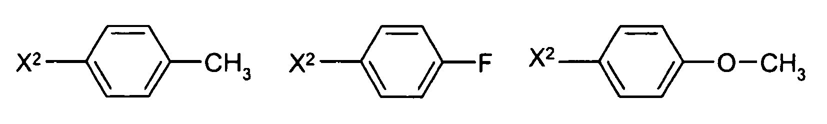 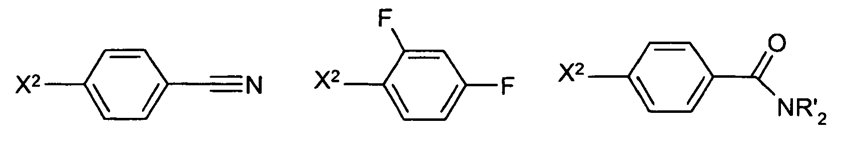 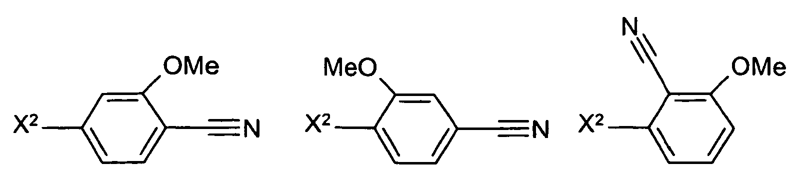 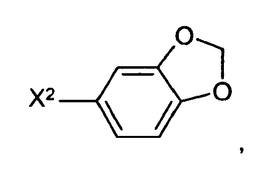 worin X² und R' wie vorstehend/nachstehend definiert ist.

In einer bevorzugten Ausführungsform der vorliegenden Erfindung steht n für 0 oder 1 und insbesondere für 0.

In einer besonders bevorzugten Ausführungsform der vorliegenden Erfindung steht n in den Resten R², R³, R⁴ und/oder R⁵, bevorzugt in den Resten R² und/oder R⁴ und insbesondere im Rest R⁴ für 0.

Einige bevorzugte Gruppen von Verbindungen können durch die folgenden Teilformeln Ia) bis In) ausgedrückt werden, die der Formel la entsprechen und worin die nicht näher bezeichneten Reste die vorstehend/nachstehend angegebene Bedeutung haben, worin jedoch

| | | |
|---|---|---|
| in Ia) | R¹ | H oder SO₂A und insbesondere Hoder SO₂-CH₃ bedeutet; |
| | | |
| in Ib) | R¹ | H oder SO₂A und insbesondere H oder SO₂-CH₃ bedeutet; |
| | D-E | für R²C=CR⁴ steht; |
| | | |
| in Ic) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen; |
| | | |
| in Id) | R¹ | H oder SO₂A und insbesondere H oder SO₂-CH₃ bedeutet; |
| | D-E | für R²C=CR⁴ steht. |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶ (CH₂)ₙCON(R⁶)₂ (CH₂)ₙSO₂N(R⁶)₂ oder (CH₂)ₙS(O)Q_{w}R⁶ bedeutet; |
| | | |
| in Ie) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ bedeutet und |
| | R⁶ | für H oder A steht; |
| | | |
| in If) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶, (CH₂)ₙCO_{N}(R⁶)₂ (CH₂)ₙSO₂N(R⁶)₂ oder (CH₂)ₙS(O)_{w}R⁶ und |
| | R⁶ | H oder A bedeutet, und |
| | n | für 0 oder 1 steht; |
| | | |
| in Ig) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht; |
| | | |
| in Ih) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)2 oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR⁶, C=O, C=S, S=O, SO₂, SO₂NR⁶ und NR⁶SO₂; |
| | | |
| in Ii) | R¹ | H oder SO₂A und insbesondere H oder SO₂-CH₃ bedeutet; |
| | D-E | für R²C=CR steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, |
| | | (CH₂)ₙCN, (CH₂)ₙCOOR⁶, (CH₂)ₙCON(R⁶)₂ (CH₂)ₙSO₂N(R⁶)₂oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR⁶, C=O, C=S, S=O, SO₂, SO₂NR⁶ und NR⁶SO₂, und |
| | R⁷, R⁸ | unabhängig voneinander ausgewählt sind unter H und A; |
| | | |
| in Ij) | R¹ | H oder SO₂A und insbesondere Hoder SO₂-CH₃ bedeutet; |
| | D-E | für R²C=CR⁴ steht, |
| | R ² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂. C(Hal)₃, NO₂. (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR , C=O, C=S, S=O, SO₂, SO₂NR⁶ und NR⁶SO₂, |
| | R⁷, R⁸ | unabhängig voneinander ausgewählt sind unter H und A, und |
| | Y | für CH, COR¹¹ oder N steht, |
| | | |
| in Ik) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR , (CH₂)ₙCON(R⁶)₂ (CH₂),SO₂N(R⁶)₂ oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1,2 oder 3 steht und Q ausgewählt ist unter O, S, N-R⁶, CONR⁶, C=O, C=S, S=O, SO₂, SO₂NR⁶ und NR⁶SO₂, |
| | R⁷, R⁸ | unabhängig voneinander ausgewählt sind unter H und A, |
| | Y | für CH, COR¹¹ oder N steht; und |
| | R¹¹ | H oder A bedeutet; |
| | | |
| in IL) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR , (CH₂)_{nCON}(R⁶)₂ (CH₂)ₙSO₂N(R⁶)₂ oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR⁶, C=O, C=S, S=O, SO₂, SO₂NR⁶ und NR⁶SO₂, |
| | R⁷, R⁸ | unabhängig voneinander ausgewählt sind unter H und A, |
| | Y | für CH, COR¹¹ oder N steht, |
| | R¹¹ | H oder A, und |
| | X² | CH₂, CH₂CH₂, HCOH, O, S, N-R⁶, CONR⁶, C=O oder eine Bindung bedeutet; |
| | | |
| in Im) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR , (CH₂)ₙCON(R⁶)₂ (CH₂)ₙSO₂N(R⁶)₂ oder (CH₂)ₙS(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR⁶, C=O, C=S, S=O, SO₂, SO₂NR⁶ und NR⁶SO₂, |
| | R⁷, R⁸ | unabhängig voneinander ausgewählt sind unter H und A, |
| | Y | für CH, COR¹¹ oder N steht, |
| | R¹¹ | H oder A, und |
| | X² | CH₂, CH₂CH₂, HCOH, O, S, N-R⁶, CONR⁶, C=O oder eine Bindung bedeutet, und |
| | R¹² | unabhängig ausgewählt ist unter A, Hal, CN, NO₂, OR⁶, N(R⁶)₂, COOR⁶ CON(R⁶)₂, COR⁶, SO₂N(R⁶)₂ und S(O)_{w}A; |
| | | |
| in In) | R¹ | H oder SO₂A und insbesondere H oder SO₂-CH₃ bedeutet; |
| | D-E | für R²C=CR⁴ steht, |
| | R² | H oder Alkyl mit 1 bis 3 C-Atomen bedeutet; |
| | R⁴ | H, Alkyl mit 1 bis 3 C-Atomen, Alkoxyalkyl mit 2 bis 5 C-Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)S(O)_{w}R⁶ und |
| | R⁶ | H oder A bedeutet, |
| | n | für 0 oder 1 steht, |
| | X¹ | (CHR⁷)_{g} bedeutet, worin g für 2, 3 oder 4 steht, oder Q-(CHR⁸)ₖ bedeutet, worin k für 1, 2 oder 3 steht und Q ausgewählt ist unter O, S, N-R⁶, CONR⁶, C=O, C=S, S=O, SO₂, SO₂NR⁶ und NR⁶SO₂, |
| | R⁷ R⁸ | unabhängig voneinander ausgewählt sind unter H und A, |
| | Y | für CH, COR¹¹, N oder einen unsubstituierten oder substituierten spiro-verknüpften 5-, 6- oder 7-gliedrigen Heterocyclus mit 1 bis 3 Heteroatomen, ausgewählt unter N, S oder O, steht, |
| | R¹¹ | H oder A, und |
| | X² | CH₂, CH₂CH₂, HCOH, O, S, N-R⁶, CONR⁶, C=O oder eine Bindung bedeutet, |
| | R¹² | unabhängig ausgewählt ist unter A, Hal, CN, NO₂, OR⁶, N(R⁶)₂, COOR ⁶ CON(R⁶)₂, COR⁶ SO₂N(R⁶)₂ und S(O)_{w}A, und |
| | R¹⁴ | unabhängig ausgewählt ist unter A, Hal, CN, NO₂, OR⁶, N(R⁶)₂, COOR ⁶ CON(R⁶)₂, COR⁶, SO₂N(R⁶)₂ und S(O)_{w}A. |

In einer bevorzugten Ausführungsform der vorliegenden Erfindung steht in den Teilformeln Ia) bis In) der Rest R² für H und R⁴ für H, A, insbesondere Alkyl mit 1 bis 4 C-Atomen oder O-Alkyl mit 1 bis 4 C-Atomen, Hal, insbesondere F oder Br, CN, NO₂ NH₂, CF₃, OCF₃ SO₂CH₃, COOR⁶ oder CON(R⁶)₂, besonders bevorzugt für CN. Im Rahmen dieser bevorzugten Ausführungsform steht R² insbesondere dann für H, wenn R⁴ für Hal, NO₂ NH₂, CF₃, OCF₃ SO₂CH₃, COOR⁶ oder CON(R⁶)₂ und insbesondere für CN steht. Im Rahmen dieser bevorzugten Ausführungsform weist die Verbindung der Formel Ia vorzugsweise einen oder zwei Substituenten R¹² und besonders bevorzugt keinen Substituenten R¹² auf. Im Rahmen dieser bevorzugten Ausführungsform weist die Verbindung der Formel Ia besonders bevorzugt vorzugsweise einen oder zwei Substituenten R¹³ und ganz besonders bevorzugt keinen Substituenten R¹³ auf. Im Rahmen dieser bevorzugten Ausführungsform weist die Verbindung der Formel la vorzugsweise keinen, einen oder zwei Substituenten R¹⁴ und insbesondere einen oder zwei Substituenten R¹⁴ auf. Wenn die Verbindung der Formel Ia einen oder zwei Substituenten R¹⁴ aufweist, sind diese vorzugsweise ausgewählt unter F, Cl, Br, I, CN, CF₃ und OCF₃ und insbesondere ausgewählt unter F, CF₃ und OCF₃.

In einer speziellen und bevorzugten Ausführungsform betrifft die vorliegende Erfindung eine Verbindung der Formel Ia und insbesondere eine Verbindung der Teilformeln Ia) bis In), worin D-E für R²C=CR⁴, R² für H oder Methyl und R⁴ für CN steht, sowie deren Solvate und Salze.

In einer weiteren speziellen und bevorzugten Ausführungsform betrifft die vorliegende Erfindung eine Verbindung der Formel Ia und insbesondere eine Verbindung der Teilformeln Ia) bis In), worin X¹ für CH₂CH₂ (d. h. für (CHR⁷)g, worin R⁷ für H und g für 2 steht), CH₂CH₂CH₂ (d. h. für (CHR⁷)_{g}, worin R⁷ für H und g für 3 steht) oder OCH₂CH₂ (d. h. für (CHR⁷)ₕ-Q-(CHR⁸)ₖ, worin h für 0, R⁸ für H und k für 2 steht) steht, sowie deren Solvate und Salze.

In einer weiteren speziellen und bevorzugten Ausführungsform betrifft die vorliegende Erfindung eine Verbindung der Formel Ia und insbesondere eine Verbindung der Teilformeln Ia) bis In), worin Y für CH, CHOH (d. h. für COR¹¹, worin R¹¹ für H steht) oder N und insbesondere für CH steht, sowie deren Solvate und Salze.

In einer weiteren speziellen und bevorzugten Ausführungsform betrifft die vorliegende Erfindung eine Verbindung der Formel la und insbesondere eine Verbindung der Teilformein Ia) bis In), worin Y einen unsubstituierten oder substituierten spiro-verknüpften Heterocyclus bedeutet, der ausgewählt ist unter Strukturen der Formeln 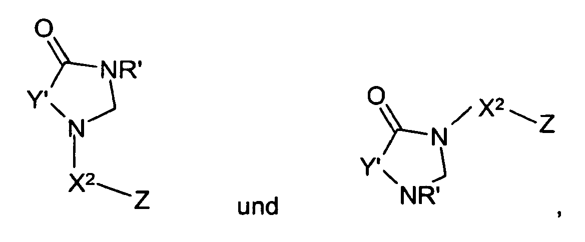 worin X², Z und R' wie vorstehend/nachstehend definiert sind und Y' für das spiro-verknüpfende C-Atom des spiro-verknüpften Heterocyclus steht. In dieser Ausführungsform steht X² besonders bevorzugt für eine Bindung. Insbesondere ist der spiro-verknüpfte Heterocyclus daher ausgewählt unter Strukturen der Formeln 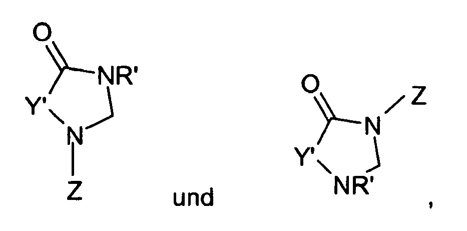 worin X², Z und R' wie vorstehend/nachstehend definiert sind und Y' für das spiro-verknüpfende C-Atom des spiro-verknüpften Heterocyclus steht.

In einer weiteren speziellen und bevorzugten Ausführungsform betrifft die vorliegende Erfindung eine Verbindung der Formel la und insbesondere eine Verbindung der Teilformeln Ia) bis In), worin X² für CH₂ (d. h. für (CHR⁷)g, worin R⁷ für H und g für 1 steht), CH₂CH₂ (d. h. für (CHR⁷)g, worin R⁷ für H und g für 2 steht), OCH₂ (d. h. für (CHR⁷)ₕ-Q-(CHR⁸)ₖ, worin Q für O, h für 0, R⁸ für H und k für 1 steht), O (d. h. für (CHR⁷)ₕ-Q-(CHR⁸)ₖ, worin Q für O und h und k für 0 steht), S (d. h. für (CHR⁷)ₕ-Q-(CHR⁸)ₖ, worin Q für S und h und k für 0 steht), C=O (d. h. für (CHR⁷)ₕ-Q-(CHR⁸)ₖ, worin Q für C=O und h und k für 0 steht), oder NH (d. h. für (CH^{R}7)ₕ-Q-(CHR⁸)ₖ, worin Q für N-R⁶, R⁶ für H und h und k für 0 steht) steht, besonders bevorzugt für CH₂, O, NH und CHOH und insbesondere für CH₂ oder O steht, sowie deren Solvate und Salze.

In einer weiteren speziellen und bevorzugten Ausführungsform betrifft die vorliegende Erfindung eine Verbindung der Formel Ia und insbesondere eine Verbindung der Teilformeln Ia) bis In), worin X² für eine chemische Bindung zwischen den Gruppen Y und Z steht. In dieser Ausführungsform steht somit die Gruppierung Y-X²-Z für die Gruppierung Y-Z.

In einer besonders bevorzugten Ausführungsform betrifft die vorliegende Erfindung eine Verbindung der Formel Ia und insbesondere eine Verbindung der Teilformeln Ia) bis In), welche die Merkmale einer oder mehrerer der vorstehend beschriebenen Ausführungsformen und insbesondere die Merkmale aller vorstehend beschriebenen Ausführungsformen oder die Merkmale aller vorstehend beschriebenen sich nicht gegenseitig ausschließenden Ausführungsformen umfasst.

In den Verbindungen der Formel Ia steht X¹, bezogen auf den Indol-Stickstoff, vorzugsweise in der 4-, 5- oder 6-Position, besonders bevorzugt 4-Position oder 6-Position und insbesondere in 4-Position des Indolrestes (gemäß der IUPAC-Nomenklatur für Indol-Systeme).

In den Verbindungen der Formel Ia stehen die Substituenten R¹⁴ vorzugsweise in ortho- und/oder para-Stellung, bezogen auf Z.

Gegenstand der vorliegenden Erfindung sind daher besonders bevorzugt Verbindungen der Formeln Iα, Iβ und Iχ 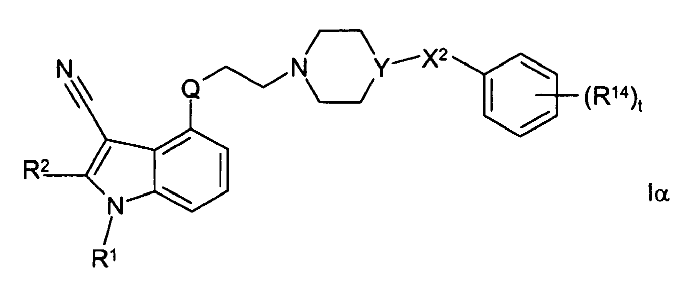 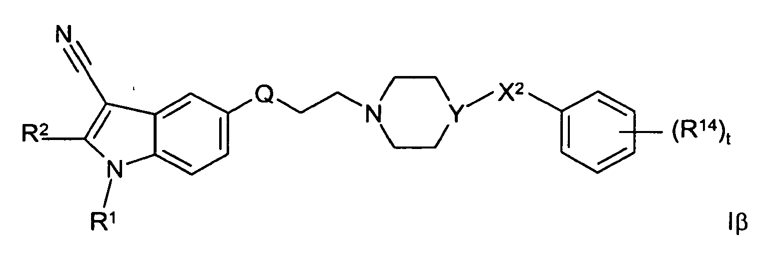 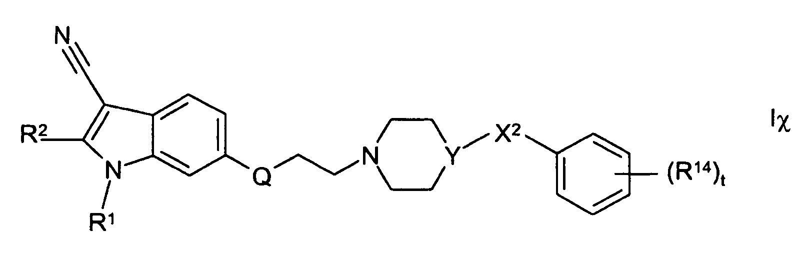 worin R¹, R², Q, Y, X², R¹⁴ und t wie vorstehend und insbesondere wie in vorstehenden Teilformeln Ia) bis In) und/oder den vorstehenden Ausführungsformen definiert sind, und Verbindungen der Formeln Iδ, Iε und Iζ 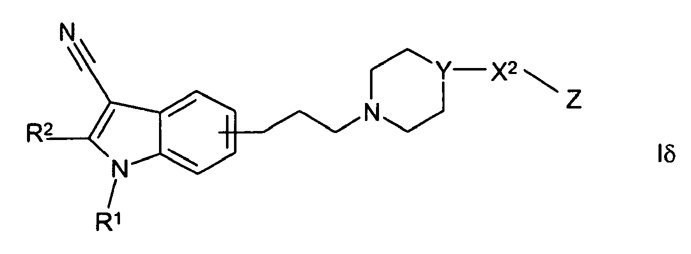 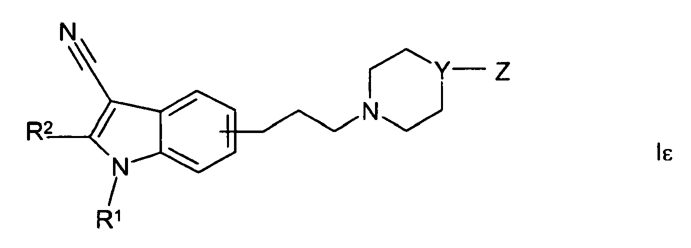 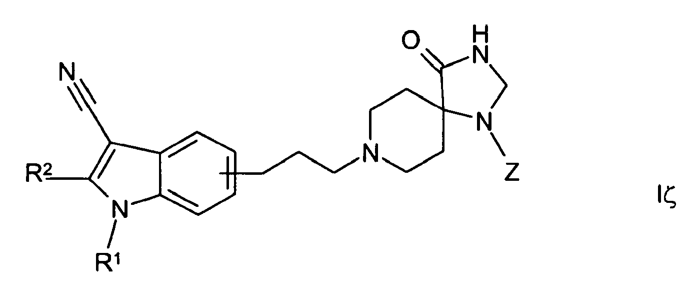 worin R¹, R², Y, X² und Z wie vorstehend und insbesondere wie in vorstehenden Teilformeln Ia) bis In) und/oder den vorstehenden Ausführungsformen definiert sind.

Besonders bevorzugt steht in den Verbindungen der Formeln Iα, Iβ und Iχ (R¹⁴)ₜ für einen Fluorsubstituenten in para-Stellung, für zwei Fluorsubstituenten, von denen einer in para- und einer in meta-Stellung, bezogen auf Z, steht, oder für CONH₂ in para-Stellung. Ferner steht in den Formeln Iα, Iβ und Iχ vorzugsweise R¹ und/oder R² für H, Q für CH₂ und Y für CH und X² für O oder CH₂.

Besonders bevorzugt steht in den Verbindungen der Formeln Iδ und Iε die Gruppe Y für N oder CH. Besonders bevorzugt steht in den Verbindungen der Formel Iζ die Gruppe Z für substiuiertes und insbesondere unsubstituiertes Phenyl.

Bevorzugt steht in den Verbindungen der Formel Iδ die Gruppierung Y-X²-Z für einen Rest der Formel 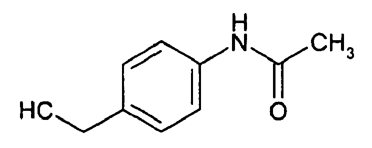 oder einen Rest der Formel

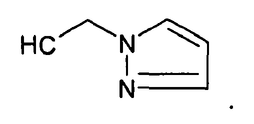

Besonders bevorzugt steht in den Verbindungen der Formel Iε die Gruppierung Y-Z für einen Rest der Formeln 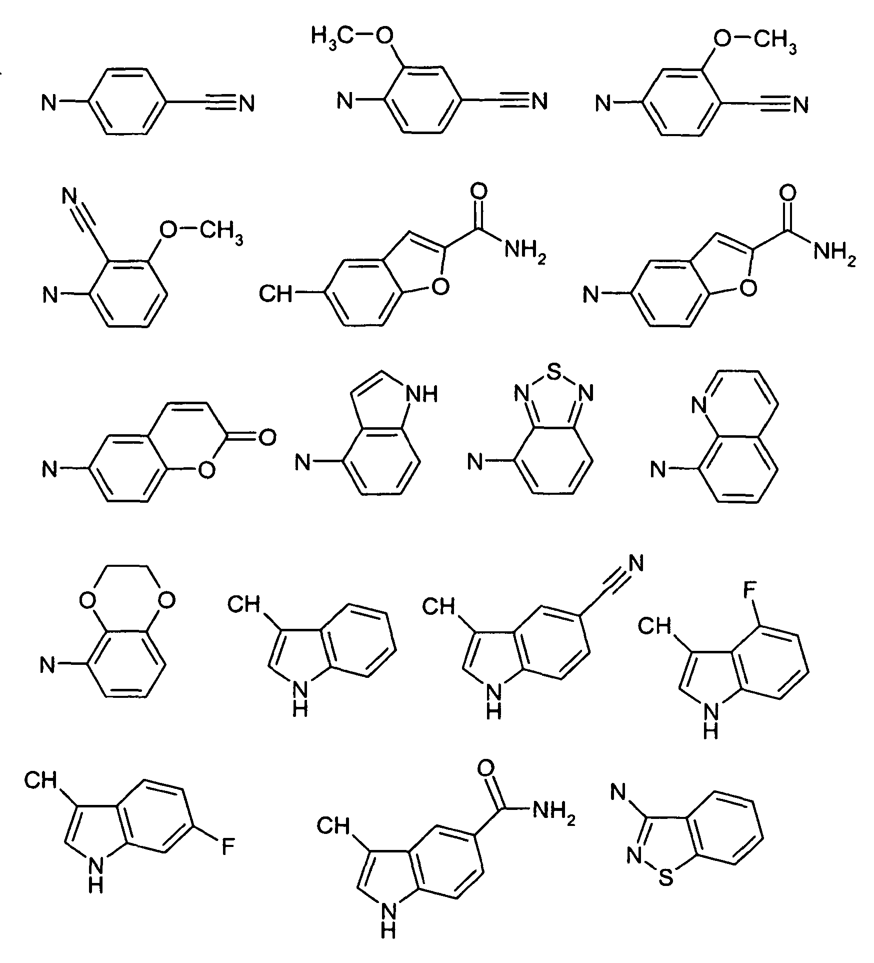 oder für einen Rest der Formeln

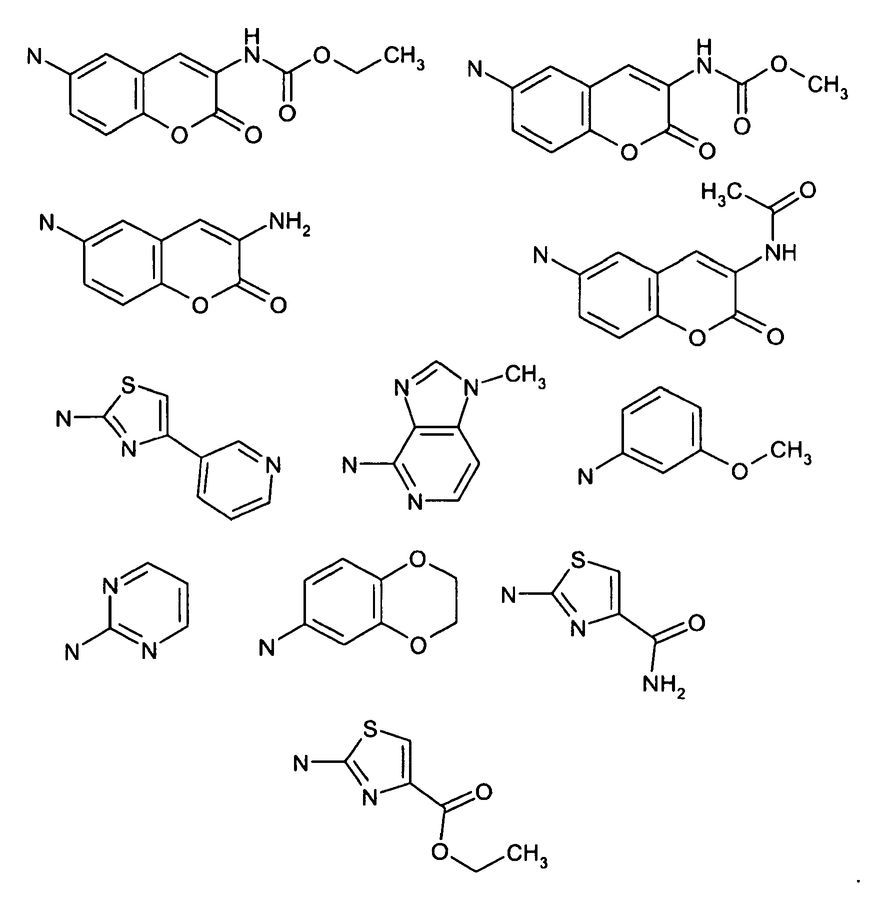

In einer ganz besonders bevorzugten Ausführungsform der vorliegenden Erfindung sind die Verbindungen der Formel Ia ausgewählt unter
a) 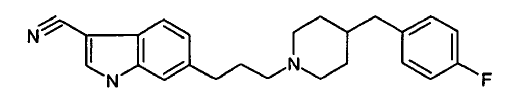 6-{3-[4-(4-Fluorbenzyl)-1-piperidyl]-propyl}-1H-indol-3-carbonitril;
b) 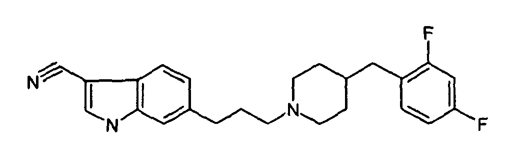 6-{3-[4-(2,4-Difluorbenzyl)-1-piperidyl]-propyl}-1H-indol-3-carbonitril;
c) 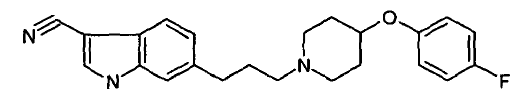 6-{3-(4-{4-Fluorphenoxy)-1-piperidyl]-propyl}-1H-indol-3-carbonitril;
d) 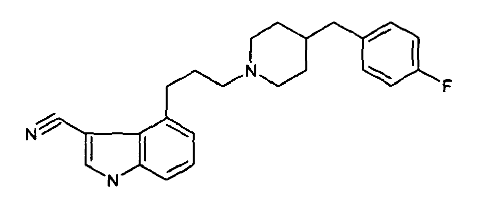 4-{3-[4-(4-Fluorbenzyl)-1-piperidyl]-propyl}-1H-indol-3-carbonitril;
e) 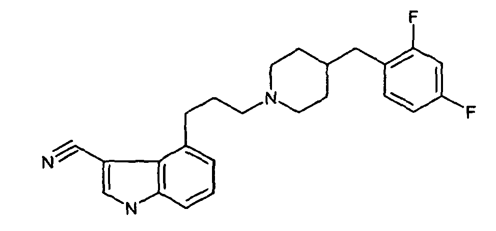 4-{3-[4-(2,4-Difluorbenzyl)-1-piperidyl]-propyl}-1H-indol-3-carbonitril;
f) 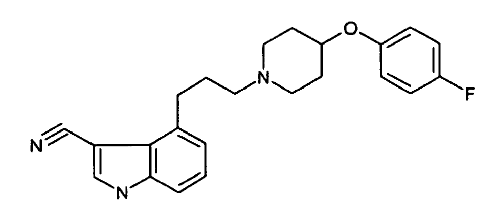 4-{3-[4-(4-Fluorphenoxy)-1-piperidyl]-propyl}-1H-indol-3-carbonitril;
g) 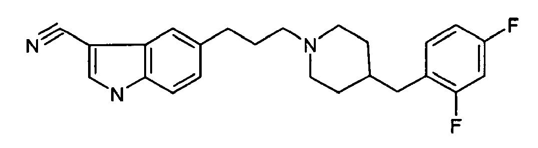 5-{3-[4-(2,4-Difluorbenzyl)-1-piperidyl]-propyl}-1H-indol-3-carbonitril;
h) 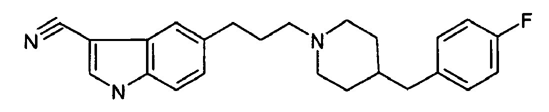 5-{3-[4-(4-Fluorbenzyl)-1-piperidyl]-propyl}-1H-indol-3-carbonitril;
i) 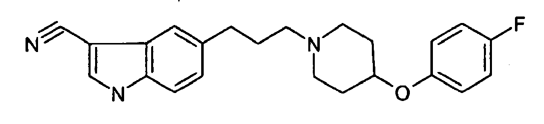 5-{3-[4-(4-Fluorphenoxy)-1-piperidyl]-propyl}-1H-indol-3-carbonitril;
j) 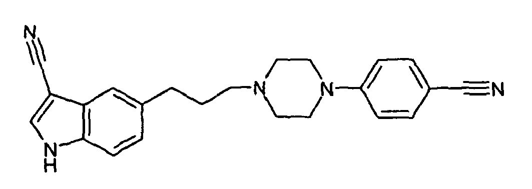 5-{3-[4-(4-Cyano-phenyl)-piperazin-1-yl]-propyl}-1H-indole-3-carbonitrile
k) 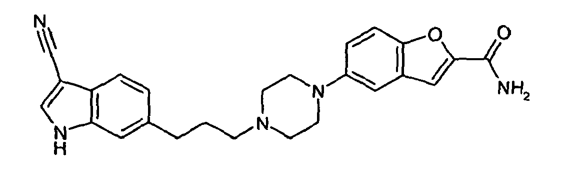 5-{4-[3-(3-Cyano-1H-indol-6-yl)-propyl]-piperazin-1-yl}-benzofuran-2-carboxylic acid amide
l) 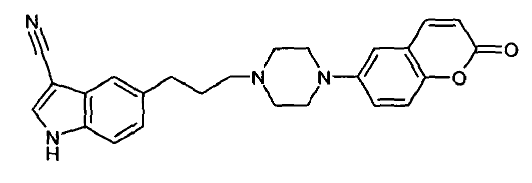 5-{3-[4-(2-Oxo-2H-chromen-6-yl)-piperazin-1-yl}-propyl}-1H-indole-3-carbonitrile
m) 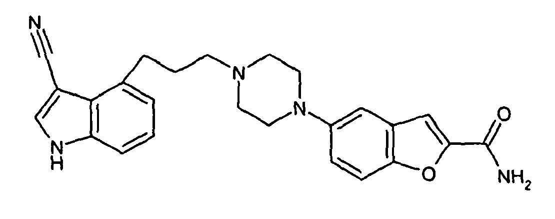 5-{4-[3-(3-Cyano-1H-indol-4-yl)-propyl]-piperazin-1-yl}-benzofuran-2-carboxylic acid amide
n) 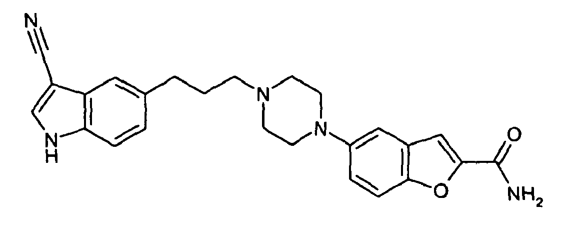 5-{4-[3-(3-Cyano-1H-indol-5-yl)-propyl]-piperazin-1-yl}-benzofuran-2-carboxylic acid amide
o) 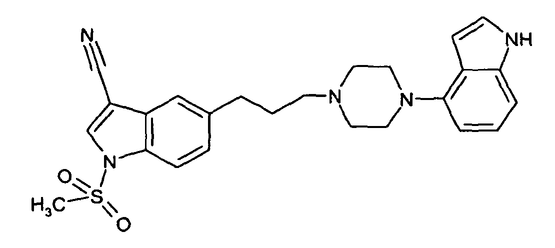 5-{3-[4-(1H-indol-4-yl)-piperazin-1-yl]-propyl}-1-methanesulfonyl-1H-indole-3-carbonitrile
p) 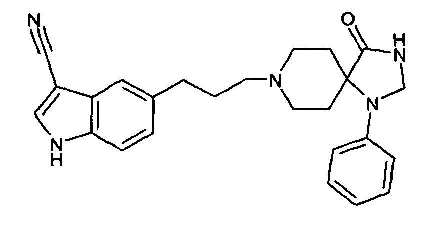 5-[3-(4-Oxo-1-phenyl-1,3,8-triaza-spiro[4.5]dec-8-yl)-propyl]-1H-indole-3-carbonitrile
q) 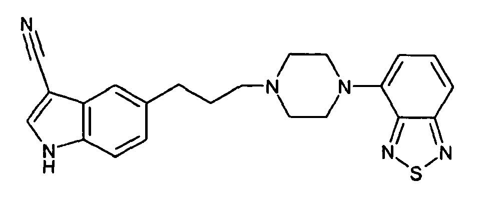 5-[3-(4-Benzo[1,2,5]thiadiazol-4-yl-piperazin-1-yl)-propyl]-1H-indole-3-carbonitrile
r) 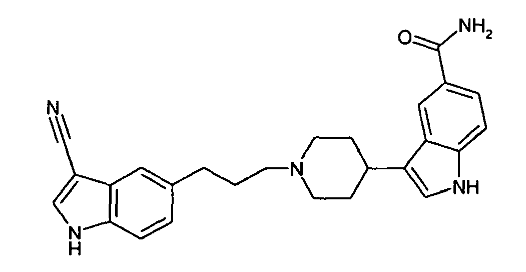 3-{1-[3-(3-Cyano-1H-indol-5-yl)-propyl]-piperidin-4-yl}-1H-indole-5-carboxylic acid amide
s) 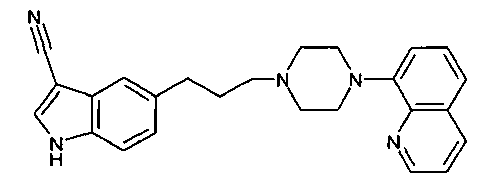 5-[3-(4-Quinolin-8-yl-piperazin-1-yl)-propyl]-1H-indole-3-carbonitrile
t) 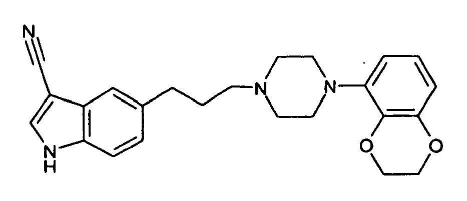 5-{3-[4-(2,3-Dihydro-benzo[1,4]diox)n-5-yl)-piperazin-1-yl]-propyl}-1H-indole-3-carbonitrile
u) 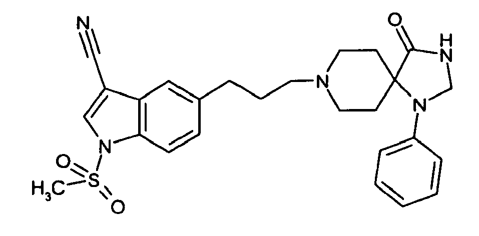 1-Methanesulfonyl-5-[3-(4-oxo-1-phenyl-1,3,8-triaza-spiro[4.5]dec-8-yl)-propyl]-1H-indole-3-carbonitrile
v) 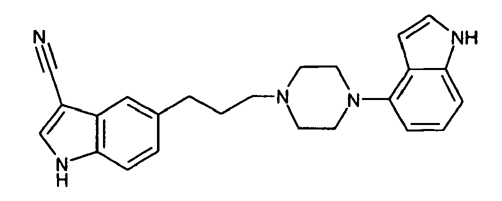 5-{3-[4-(1H-Indol-4-yl)-piperazin-1-yl]-propyl}-1H-indole-3-carbonitrile
w) 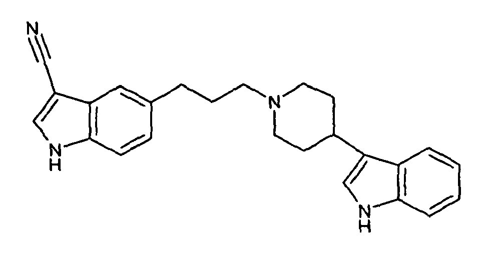 5-{3-[4-(1H-Indol-3-yl)-piperidin-1-yl]-propyl}-1H-indole-3-carbonitrile
x) 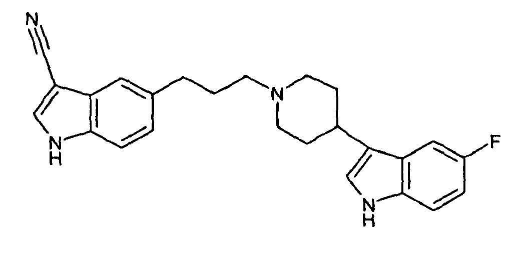 5-{3-[4-(5-Fluoro-1H-indol-3-yl)-piperidin-1-yl]-propyl}-1H-indole-3-carbonitrile
y) 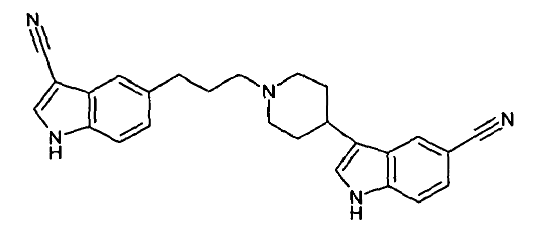 3-{1-[3-(3-Cyano-1H-indol-5-yl)-propyl]-piperidin-4-yl}-1H-indole-5-carbonitrile
z) 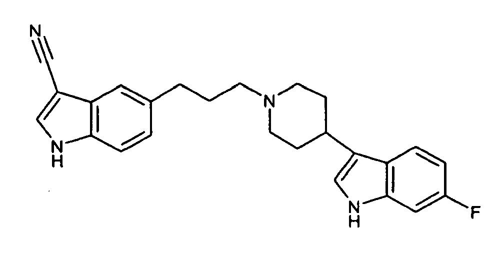 5-{3-[4-(6-Fluoro-1H-indol-3-yl)-piperidin-1-yl]-propyl}-1H-indole-3-carbonitrile
aa) 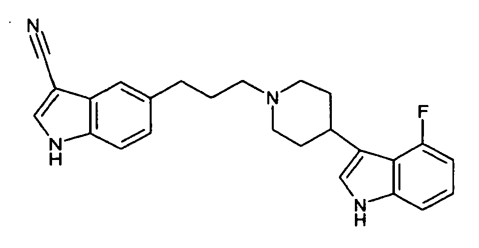 5-{3-[4-(4-Fluoro-1H-indol-3-yl)-piperidin-1-yl]-propyl}-1H-indole-3-carbonitrile
bb) 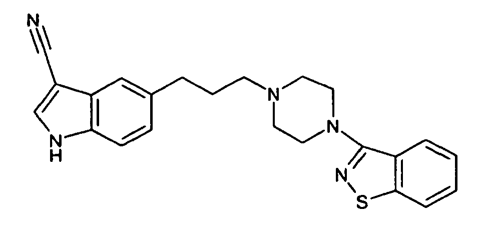 5-[3-(4-Benzo[d]isothiazol-3-yl-piperazin-1-yl)-propyl]-1H-indole-3-carbonitrile
cc) 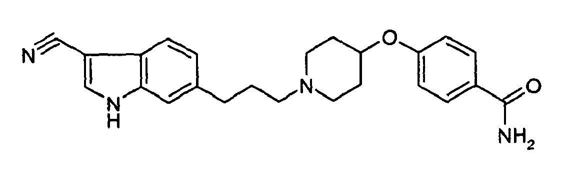 4-{1-[3-(3-Cyano-1H-indol-6-yl)-propyl]-piperidin-4-yloxy}-benzamide
dd) 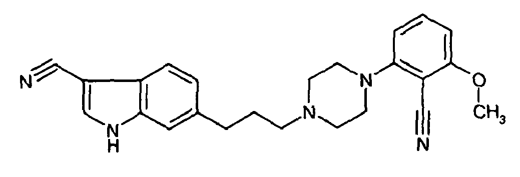 6-{3-[4-(2-Cyano-3-methoxy-phenyl)-piperazin-1-yl]-propyl}-1H-indole-3-carbonitrile
ee) 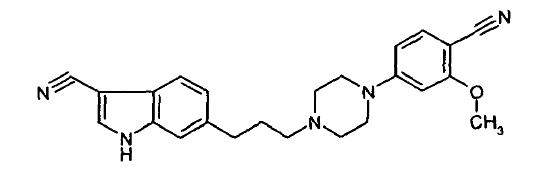 6-{3-[4-(4-Cyano-3-methoxy-phenyl)-piperazin-1-yl]-propyl]-1H-indole-3-carbonitrile
ff) 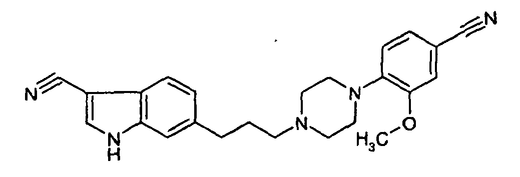 6-{3-[4-(4-Cyano-2-methoxy-phenyl)-piperazin-1-yl]-propyl]-1H-indole-3-carbonitrile
gg) 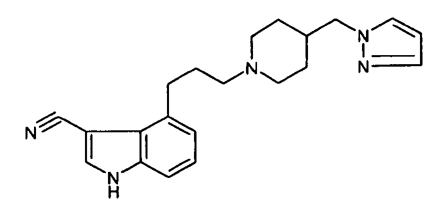 4-[3-(4-Pyrazol-1-ylmethyl-1-piperidyl)-propyl]-1H-indol-3-carbonitril
hh) 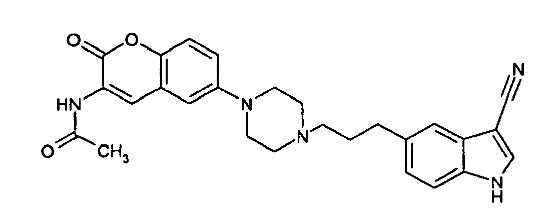 N-(6-{4-[3-(3-Cyano-1H-indol-5-yl)-propyl]-piperazin-1-yl}-2-oxo-2H-chromen-3-yl)-acetamid
ii) 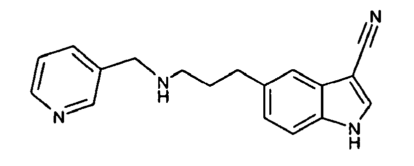 5-{3-[(Pyridin-3-ylmethyl)-amino]-propyl}-1H-indol-3-carbonitril
jj) 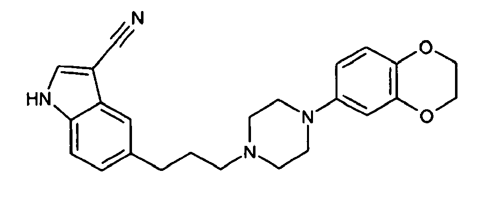 5-{3-[4-(2,3-Dihydro-benzo[1,4]dioxin-6-yl)-piperazin-1-yl]-propyl}-1H-indol-3-carbonitril
kk) 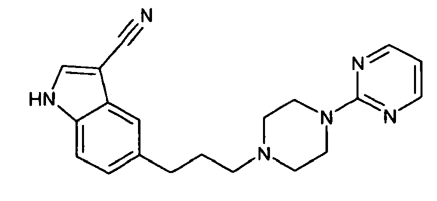 5-[3-(4-Pyrimidin-2-yl-piperazin-1-yl)-propyl]-1H-indol-3-carbonitril
ll) 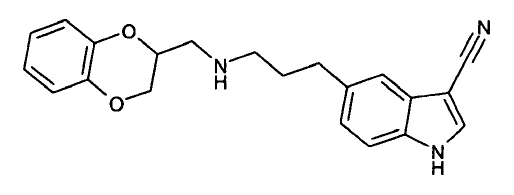 5-{3-[(2,3-Dihydro-benzo[1,4]dioxin-2-ylmethyl)-amino]-propyl}-1H-indol-3-carbonitril
mm) 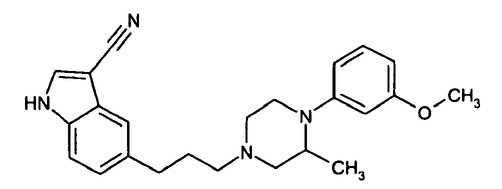 5-{3-[4-(3-Methoxy-phenyl)-3-methyl-piperazin-1-yl]-propyl}-1H-indol-3-carbonitril
nn) 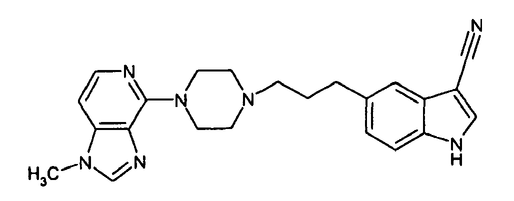 5-{3-[4-(1-Methyl-1H-imidazo[4,5-c]pyridin-4-yl)-piperazin-1-yl]-propyl}-1H-indol-3-carbonitril
oo) 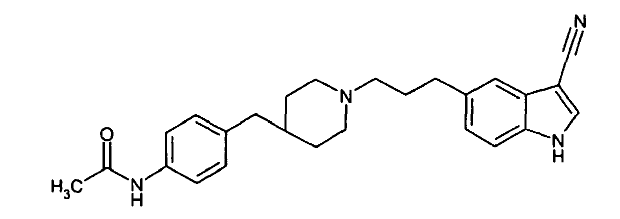 N-(4-{1-[3-(3-Cyano-1H-indol-5-yl)-propyl]-piperidin-4-ylmethyl}-phenyl)-acetamid
pp) 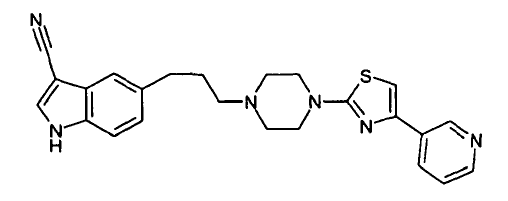 5-{3-[4-(4-Pyridin-3-yl-thiazol-2-yl)-piperazin-1-yl]-propyl}-1H-indol-3-carbonitril
qq) 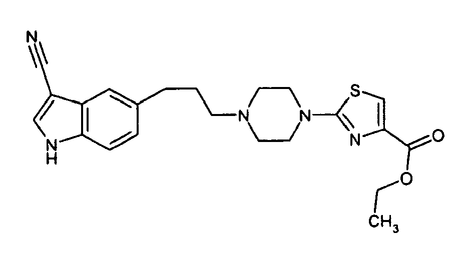 2-{4-[3-(3-Cyano-1H-indol-5-yl)-propyl]-piperazin-1-yl}-thiazol-4- carbonsäureethylester
rr) 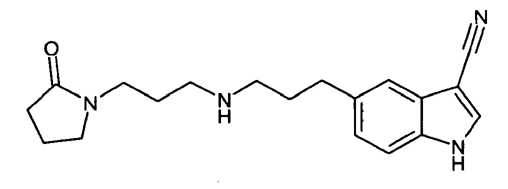 5-{3-[3-(2-Oxo-pyrrolidin-1-yl)-propylamino]-propyl}-1H-indole-3-carbonitril
ss) 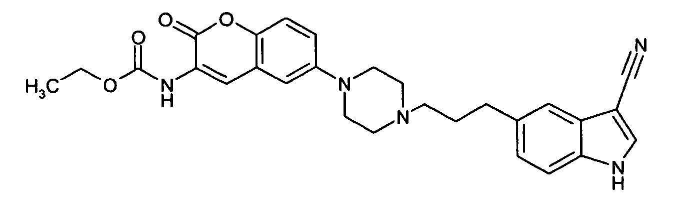 (6-{4-[3-(3-Cyano-1H-indol-5-yl)-propyl]-piperazin-1-yl}-2-oxo-2H-chromen-3-yl)- carbaminsäureethylester
tt) 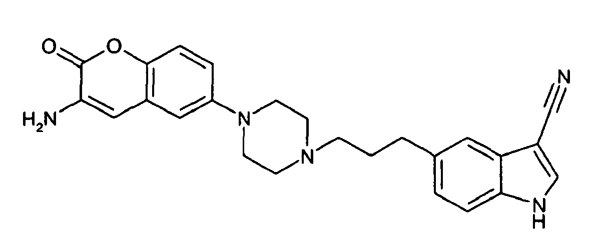 5-{3-[4-(3-Amino-2-oxo-2H-chromen-6-yl)-piperazin-1-yl]-propyl}-1H-indol-3-carbonitril
uu) 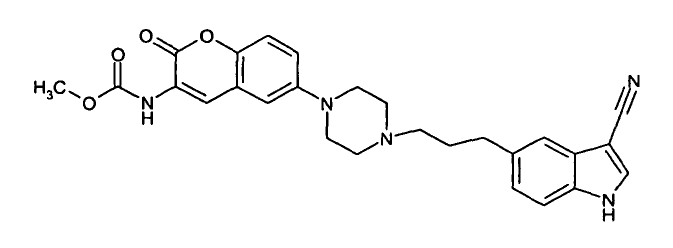 (6-{4-[3-(3-Cyano-1H-indol-5-yl)-propyl]-piperazin-1-yl}-2-oxo-2H-chromen-3-yl)- carbaminsäuremethylester
vv) 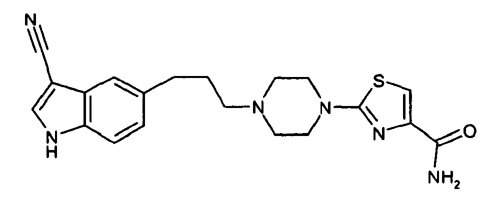 2-{4-[3-(3-Cyano-1H-indol-5-yl)-propyl]-piperazin-1-yl}-thiazol-4-carbonsäureamid
ww) 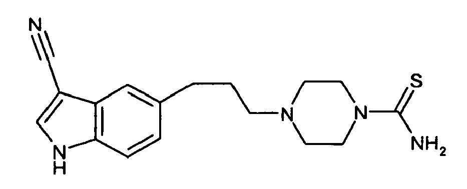 4-[3-(3-Cyano-1H-indol-5-yl)-propyl]-piperazin-1-thiocarbonsäureamid
sowie deren Salzen und Solvaten, bevorzugt deren physiologisch verträglichen Salzen und Solvaten und insbesondere deren physiologisch verträglichen Salzen.

Die erfindungsgemäßen Verbindungen können, abhängig von der Auswahl der vorstehend beschriebenen Substituenten und Reste, ein oder mehrere chirale Zentren, insbesondere ein oder mehrere chirale Kohlenstoffatome, aufweisen. Wenn eine erfindungsgemäße Verbindung definierter Zusammensetzung ein oder mehrere chirale Zentren aufweist, kann diese Verbindung definierter Zusammensetzung in unterschiedlichen Stereoisomeren vorliegen. Gegenstand der vorliegenden Erfindung sind alle möglichen solchen Stereoisomere erfindungsgemäßer Verbindungen, die sowohl als einzelne, stereochemisch einheitliche Verbindungen, als auch als Gemische zweier oder mehrerer stereochemisch einheitlicher Verbindungen vorliegen können. Im Falle von Gemischen zweier oder mehrerer Stereoisomere können die einzelnen Stereoisomere in unterschiedlichen oder gleichen Anteilen vorliegen. Bei Gemischen aus zwei Stereoisomeren, ihn gleichen Anteilen vorliegen und optische Antipoden darstellen, spricht man von racemischen Gemischen. Racemische Gemische von Verbindungen der Formel Ia sind ebenfalls Gegenstand der vorliegenden Erfindung.

Die Verbindungen der Formel la und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe, z. B. die Verbindungen der Formel 11 und/oder III können, falls erwünscht, auch in situ gebildet werden, so daß man sie aus dem Reaktionsgemisch nicht isoliert, sondern sofort weiter zu der Verbindung der Formel Ia umsetzt.

Die Verbindung der Formel Ia kann vorzugsweise erhalten werden, indem man Verbindungen der Formel II mit Benzylpiperidin, Phenoxypiperidin oder Derivaten davon, insbesondere 4-(4-Fluorobenzyl)-piperidin,4-(2,4-Difluorbenzyl)-piperidin oder 4-(4-Fluorphenoxy)-piperidin, umsetzt.
Die Ausgangsverbindungen der Formel II in der Regel neu. Sie können aber nach an sich bekannten Methoden hergestellt werden. Die Ausgangsverbindungen der Formel III sind entweder neu oder literaturbekannt bzw. handelsüblich. In jedem Fall können sie jedoch nach an sich bekannten Methoden hergestellt werden.

In den Verbindungen der Formel II bedeutet L¹ vorzugsweise Cl, Br, I, OH, eine reaktionsfähig abgewandelte OH-Gruppe, insbesondere eine reaktionsfähig veresterte OH-Gruppe, wie eine Alkylsulfonyloxy-Gruppe mit 1-6 C-Atomen (bevorzugt Methylsulfonyloxy) oder Arylsulfonyloxy-Gruppe mit 6-10 C-Atomen (bevorzugt Phenyl- oder p-Tolylsulfonyloxy), oder eine Diazoniumgruppe.

In den Verbindungen der Formel III bedeutet L² vorzugsweise H oder eine die Aminofunktion aktivierende Gruppe, beispielsweise ein Metallion. Geeignete Metallionen sind insbesondere Alkalimetall-, Erdalkalimetall- oder Aluminium-Ionen. Bevorzugt als Metallionen sind Alkalimetallionen, insbesondere Li, Na oder K. Bei mehrwertigen Metallionen bildet sich oft ein Komplex aus Metallion und zwei oder mehreren Verbindungen der Formel III, wobei der Komplex stöchiometrisch in der Regel so viele Verbindungen der Formel III umfasst, wie es der Wertigkeit des Metallion entspricht.

Die Umsetzung der Verbindungen der Formel II mit Verbindungen der Formel III erfolgt in der Regel in einem inerten Lösungsmittel, vorzugsweise in Gegenwart eines säurebindenden Mittels. Als säurebindende Mittel kommen alle in der organischen Synthesechemie üblichen Basen, sowohl anorganische als auch organische, bevorzugt organische Basen, in Betracht. Beispiele für geeignete organische Basen sind Triethylamin, Diisopropylamin (DIPEA), Dimethylanilin, Pyridin oder Chinolin. Auch der Zusatz einer anorganischen Base, wie beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, kann günstig sein.

Die Reaktionszeit liegt je nach den angewendeten Bedingungen zwischen einigen Minuten und 14 Tagen, die Reaktionstemperatur zwischen etwa -30° und 180°, normalerweise zwischen -20°und 140°, bevorzugt zwischen -10° und 130° und insbesondere zwischen etwa 0° und etwa 120°. In vielen Fällen ist es günstig, die Umsetzung einer Ausgangsverbindung der Formel II mit einer Ausgangsverbindung der Formel III bei vergleichsweise hohen Temperaturen durchzuführen, beispielsweise bei einer Temperatur im Bereich von 70° bis 130°, bevorzugt 80° bis 120°und insbesondere 90° bis 110°, z. B. bei etwa 100°. Bei einer Umsetzung in diesem Temperaturbereich ist es in vielen Fällen günstig, eine organische Base, wie Triethylamin oder Diisopropylamin, oder bevorzugt eine anorganische Base, wie Natriumhydroxid, Natriumcarbonat und insbesondere Natriumhydrogencarbonat, zu verwenden.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Die Ausgangsverbindungen der Formel II, insbesondere solche, bei denen X für (CHR⁷)₃ und vorzugsweise für einen Propylenrest, d. h. für CH₂CH₂CH₂ steht, lassen sich vorteilhaft in einer mehrstufigen Synthesesequenz herstellen, die von einer Verbindung der Formel VI 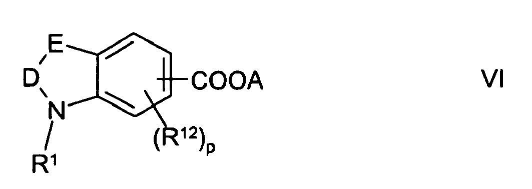 ausgeht.

In einem ersten Schritt wird die Estergruppe COOA der Verbindung der Formel VI zu einer Hydroxymethyl-Gruppe CH₂-OH reduziert, beispielsweise mit Hydriden, vorzugsweise komplexen Hydriden, wie Lithiumaluminiumhydrid. Man erhält so ein Hydroxymethyl-Derivat der Formel VII, 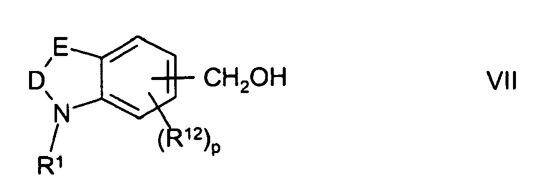 welches in einem zweiten Schritt mit einem Oxidationsmittel, wie beispielsweise Braunstein (MnO₂), in ein Aldehyd-Derivat der Formel VIII 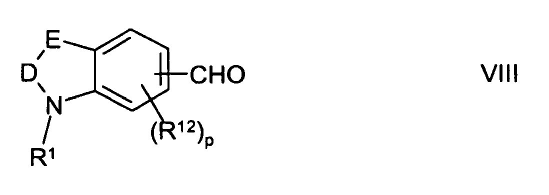 überführt werden kann. Das Aldehyd-Derivat der Formel VIII kann in einem dritten Reaktionsschritt mit einem Essigsäure-Derivat CH₃-COOA, vorzugsweise Ethyacetat und insbesondere Methylacetat, kondensiert werden (Aldolkondensation). Die Kondensation wird vorzugsweise unter basischen Bedingungen durchgeführt, beispielsweise mit Alkoholaten, wie Natriummethanolat oder Natriumethanolat, als Base. Man erhält in diesem dritten Schritt ein Acrylat-Derivat der Formel IX, 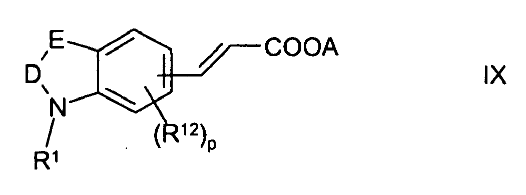 welches in einem vierten Schritt zu einer Verbindung der Formel X 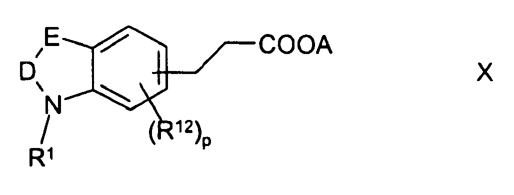 hydriert werden kann, z. B. in einer Wasserstoff-Atmosphäre in Gegenwart eines Platinmetall-Katalysators, wie beispielsweise Palladium/Kohlenstoff. Die Verbindung der Formel X kann anschließend in einem fünften Schritt zu einem Alkohol-Derivat der Formel XI 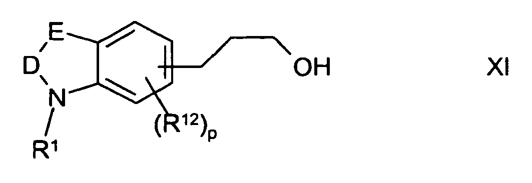 reduziert werden. Als Reduktionsmittel für diesen Schritt kommen beispielsweise Metall- oder Borhydride und insbesondere komplexe Hydride, wie Lithiumaluminiumhydrid, und sogenannte deaktivierte komplexe Hydride, wie LiAl(OR)ₓH₄₋ₓ, worin X für 1,2 oder 3 und R für Alkylreste mit 1 bis von Kohlenstoffen steht. Ein für diese Reduktion geeignetes deaktiviertes komplexes Hydrid ist unter dem Namen Vitride handelsüblich. Der Einsatz der so genannten deaktivierten komplexen Hydride ist insbesondere dann vorteilhaft, wenn man eine selektive Reduktion der COOA-Gruppe der Verbindung X zu einer Alkohol-Gruppe gemäß Formel XI in Gegenwart von anderen anderen, durch übliche Hydride, wie Lithiumaluminiumhydrid, reduzierbaren Gruppen, wie beispielsweise Nitril-Gruppen, erreichen möchte. Die Verbindung XI ist ein Beispiel für eine Verbindung der Formel II, worin X für CH₂CH₂CH₂ und L¹ für OH steht. Vorzugsweise überführt man eine Verbindung der Formel XI in eine Verbindung der Formel XII, 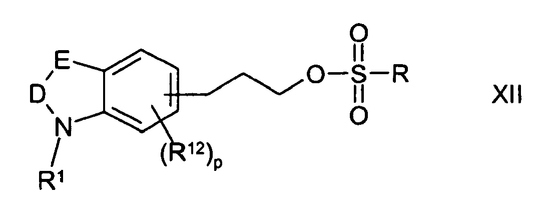 worin die OH-Gruppe reaktionsfähig verestert ist, beispielsweise durch Umsetzung mit Alkyl- oder Arylsulfonylchloriden, vorzugsweise in Gegenwart einer Base, wie beispielsweise Alkylaminen und insbesondere Triethylamin oder Diisopropylamin. Die Verbindungen der Formel XI oder vorzugsweise der Formel XII werden anschließend mit einer Verbindung der Formel III, worin L² vorzugsweise für H steht, zu einer Verbindung der Formel la umgesetzt, bevorzugt in Gegenwart einer Base, wie beispielsweise Alkalimetallhydroxiden, Alkalimetallcarbonaten und insbesondere Alkalimetallhydrogencarbonaten.

Zur Herstellung von Verbindungen der Formel Ia, worin D-E für R²C=CR⁴ und R² und/oder R⁴ für CN steht, ist es vorteilhaft, von Verbindungen der Formel VI auszugehen, in denen D-E für R²C=CR⁴ und R² und/oder R⁴ für H steht, und diese zunächst wie vorstehend beschrieben zu Verbindungen der Formeln VII, VII VIII und IX umzusetzen, in denen D-E für R²C=CR⁴ und R² und/oder R⁴ für H steht. Die Umwandlung von R² und/oder R⁴ von H in CN wird dann bevorzugt so durchgeführt, das man zunächst Dimethylformamid mit Phosphorylchlorid reagieren lässt, eine Verbindung der Formel IX, in der D-E für R²C=CR⁴ und R² und/oder R⁴ für H steht und insbesondere eine Verbindung der Formel IXa, in der D-E für R²C=CR⁴ und R² und R⁴ für H steht, zugibt, das Reaktionsgemisch vorzugsweise bei einer Temperatur zwischen 50 und 150 °C und insbesondere 80 bis 130 °C reagieren lässt, vorzugsweise für eine Zeit im Bereich von zehn Minuten bis zwei Stunden und insbesondere 30 bis 90 Minuten. Anschließend setzt man das Reaktionsgemisch mit Hydroxylammoniumchlorid (= Hydroxylamin-Hydrochlorid) um. Die Umsetzung mit Hydroxylammoniumchlorid wird vorzugsweise in einem Temperaturbereich wie vorstehend beschrieben und in einen Zeitraum von 2 bis 60 Minuten und insbesondere 5 bis 30 Minuten durchgeführt. Zur Aufarbeitung wird das Reaktionsgemisch vorzugsweise hydrolysiert und die Verbindung der Formel IX, worin D-E für R²C=CR⁴ und R² und/oder R⁴ für CN steht, oder, wenn man eine Verbindung der Formel IXa eingesetzt hat, die Verbindung der Formel IXb, worin D-E für R²C=CR⁴ und R² für H und R⁴ für CN steht, nach üblichen, dem Fachmann geläufigen Methoden isoliert.

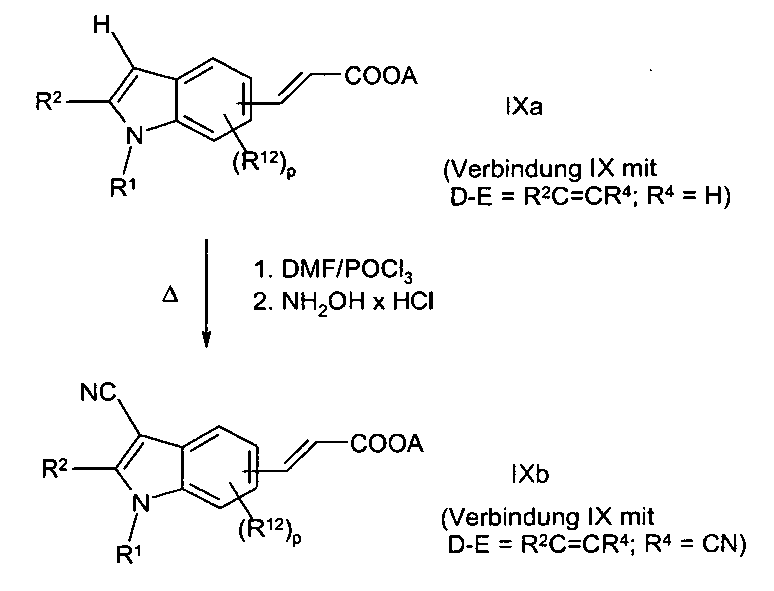

Das vorstehende Verfahren eignet sich insbesondere für die Umwandlung von Verbindungen der Formel IX, in denen D-E für R²C=CR⁴ und R² und R⁴ für H steht, in Verbindungen der Formel IX, in denen D-E für R²C=CR⁴, R² für H und R⁴ für CN steht.

Eine Base der Formel Ia kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und/oder Aufreinigung der Verbindungen der Formel Ia verwendet werden.

Die Erfindungsgemäßen Verbindungen können als Therapeutika, Diagnostika und/oder Kosmetika beziehungsweise zusammen mit einem oder mehreren von den erfindungsgemäßen Verbindungen verschiedenen Wirkstoffen und/oder Hilfsstoffen in Therapeutika, Diagnostika und oder Kosmetika verwendet werden. Üblicherweise werden die erfindungsgemäßen Verbindungen in Form von pharmazeutischen, diagnostischen und/oder kosmetischen Formulierungen eingesetzt. Solche Formulierungen und Verfahren zu ihrer Herstellung sind dem Fachmann bekannt.

Beispiele für solche Formulierungen sind wenigstens eine erfindungsgemäße Verbindung enthaltende Suspensionen, Emulsionen, Lösungen, Liposome, Salze, Pasten, bioabbaubare Polymere, Nanopartikel, Tabletten, beschichtete Tabletten, Dragees, Filmtabletten, Kapseln, Pillen, Granulate, Pulver, Aerosole, Tropfen oder Sprays.

Die erfindungsgemäßen Verbindungen bzw. Formulierungen, die wenigstens eine erfindungsgemäße Verbindung enthalten, können an Mensch oder Tier verabreicht werden, z. B. lokal oder systemisch und insbesondere oral, intravenös, intraperitoneal, subkutan, transdermal, nasal, buccal und/oder ionotophoretisch.

Gegenstand der Erfindung ist ferner die Verwendung der Verbindungen der Formel la und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend eine wirksame Menge mindestens einer der Verbindungen der Formel Ia und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.

Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Gegenstand der vorliegenden Erfindung ist daher ein Verfahren zur Herstellung pharmazeutischer Zubereitungen, das dadurch gekennzeichnet ist, daß man eine Verbindung der Formel Ia nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

Die Verbindungen der Formel Ia und/oder ihre physiologisch unbedenklichen Salze können als exzitatorische Aminosäure-Antagonist bei der Bekämpfung von Krankheiten, insbesondere zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen verwendet werden.

Gegenstand der vorliegenden Verbindung ist daher die Verwendung von Verbindungen der Formel Ia nach Anspruch 1 und/oder ihrer physiologisch unbedenklichen Salze oder Solvate zur Herstellung eines Arzneimittels zur Prophylaxe und/oder Behandlung von Schizophrenie, Depression, Demenz, Parkinsonschen Krankheit, Morbus Alzheimer, Lewy Bodies Dementia, Huntington, Tourette Syndrom, Angst, Lern- und Erinnerungseinschränkungen, neurodegenerativen Erkrankungen und anderen kognitiven Beeinträchtigungen, sowie Nikotinabhängigkeit und Schmerzen.

Dabei können die erfindungsgemäßen Verbindungen in der Regel in Analogie zu anderen bekannten Verbindungen mit ähnlichem Wirkprofil, wie z.B. Ifenprodil, verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt. Die orale Applikation ist besonders bevorzugt.

Die erfindungsgemäßen Verbindungen zeigen bei vergleichsweise leichter Herstellbarkeit ein vorteilhaftes Wirkprofil. So zeigen erfindungsgemäße Verbindungen in Rezeptorbindungstests eine Affinität zur Ifenprodil-Bindungsstelle des NMDA-Rezeptors bereits in nanomolaren Konzentrationen. Vorzugsweise zeichnen sich die erfindungsgemäßen Verbindungen darüber hinaus als Polyamin-Antagonisten mit bevorzugt selektiver Bindung am NR2B-Rezeptor der NMDA-Unterrezeptor-Klasse durch keine oder eine nur sehr geringe Verlängerung des QT-Segments im Elektrocardiogramm aus.

Die Verbindungen der Formel la und ihre pharmazeutisch verwendbaren Prodrugs, Derivate, Solvate, Stereoisomere und Salze eignen sich besonders bevorzugt zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesien, Parkinsonismus und/oder Psychosen und von Nebenwirkungen bei der Behandlung der Hypertonie (z. B. mit α- Methyldopa). Ferner können die Verbindungen vorteilhaft in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z. B. Migräne), insbesondere in der Geriatrie ähnlich wie gewisse Ergot-Alkaloide.

Ganz besonders bevorzugt können die erfindungsgemäßen Verbindungen auch als Therapeutika zur Bekämpfung der Folgen cerebraler Infarktgeschehen (apoplexia cerebri), wie Schlaganfall und cerebraler Ischämien und zur Behandlung von Hirn- und Rückenmarkstraumata eingesetzt werden.

Insbesondere sind sind die erfindungsgemäßen Verbindungen geeignet als Ärzneimittelwirkstoffe für Anxiolxytika, Antidepresiva, Antipsychotika, Neuroleptika, Antihypertonika und/oder zur positiven Beeinflussung von Zwangsverhalten (OCD), Schlafstörungen, tardiver Dyskinesien, Lernstörungen, altersabhängiger Erinnerungsstörungen, Essstörungen wie Bulimie und/oder Sexualfunktionsstörungen.

Dabei werden die erfindungsgemäßen Substanzen für die vorstehend genannten Indikationen/Anwendungsgebiete, bevorzugt für die in den zwei vorstehenden Absätzen genannten Indikationen/Anwendungsgebiete, in der Regel in Analogie zu bekannten, im Handel befindlichen Präparaten (z. B. Citalopram und Fluoxetine) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen liegen zwischen etwa 0,2 und 500 mg, insbesondere zwischen 0,2 und 50 mg pro Dosierungseinheit. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist für die vorstehend genannten Indikationen bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet gegebenenfalls die organische Phase, z. B. über Natriumsulfat, dampft die organische Phase ein und reinigt den erhaltenen Rückstand durch Chromatographie, z. B. an Kieselgel, und/oder durch Kristallisation.

### Beispiel 1

### Herstellung von 4-{3-[4-(Fluorbenzyl)-piperidin-1-yl]-propyl}-1H-indol-3-carbonitril (= EMD 432517)

1.1. Schritt a: Herstellung von 4-Hydroxymethylindol (**2**)
   Eine Suspension von LiAlH₄ in 100 ml Tetrahydrofuran wird unter Schutzgasatmosphäre (N₂) so mit einer Lösung von 25 g 4-Indolcarbonsäure-methylester (**1**) in 200 ml Tetrahydrofuran versetzt, dass die Temperatur des Reaktionsgemischs 40 °C nicht übersteigt. Nach beendeter Zugabe rührt man das Reaktionsgemisch noch etwa zwei Stunden bei Raumtemperatur (25 °C), bis der 4-Indolcarbonsäuremethylester vollständig umgesetzt ist. Anschließend hydrolysiert man das Reaktionsgemisch durch Zugabe von Eiswasser (etwa 100 ml) und filtriert das Hydrolysat über ein Bett aus Kieselgur ab. Nach destillativer Entfernung des Tetrahydrofurans wird die erhaltene wässrige Phase mit Ethylacetat extrahiert. Die erhaltene organische Phase wird über Magnesiumsulfat getrocknet und eingeengt. Man erhält 16,0 g (76% der Theorie) eines leicht beige gefärbten kristallinen Rückstands von 4-Hydroxymethylindol (**2**).
1.2. Schritt b: Herstellung von 4-Formylindol (**3**)
   Eine Lösung von 74 g 4-Hydroxymethylindol (**2**) in 3 Litern Dichlormethan wird unter Rühren langsam mit Braunstein (MnO₂) versetzt und anschließend 72 Stunden bei Raumtemperatur gerührt. Anschließend filtriert man das Reaktionsgemisch über Kieselgur ab und engt das Filtrat ein. Den erhaltenen kristallinen Rückstand verrührt man mit Cyclohexan und filtriert im Vakuum. Nach Trocknen erhält man 63 g (86% der Theorie) kristallines 4-Formylindol (**3**).
1.3. Schritt c: Herstellung von 3-(1H-Indol-4-yl)-acrylsäuremethylester (**4**)
   Zu 300 ml Tetrahydrofuran gibt man unter Rühren bei 5 °C unter Schutzgas (N₂) 48 g Natriummethanolat, kühlt die Suspension auf 0 °C, gibt bei 0 bis 3 °C Tropfenweise 70 ml Methylacetat zu und rührt weitere 45 Minuten bei dieser Temperatur. Anschließend gibt man unter Rühren eine Lösung aus 43 g 4-Formylindol (**3**) in 300 ml Tetrahydrofuran hinzu, wobei die Temperatur 3°C nicht übersteigt. Anschließend rührt man noch zwei Stunden bei Raumtemperatur. Zur Aufarbeitung wird das Reaktionsgemisch eingeengt, der Rückstand in Methylacetat gelöst und mit Wasser extrahiert. Die erhaltene organische Phase wird getrocknet, das Lösungsmittel im Vakuum entfernt und der Rückstand durch Chromatographie an Kieselgel gereinigt. Man erhält 35 g (59% der Theorie) eines leicht beige gefärbten kristallinen Rückstands von 3-(1H-Indol-4-yl)-acrylsäuremethylester (**4**).
1.4. Schritt d: Herstellung von Indol-Carbonitril **5**
   Zu 100 ml Dimethylformamid tropft man unter Kühlung im Eisbad Phosphorylchlorid, sodass die Temperatur des Gemisches etwa 20 bis 30 °C beträgt. Anschließend gibt man Tropfenweise bei Raumtemperatur eine Lösung von 25 g 3-(1H-Indol-4-yl)-acrylsäure in 100 ml Dimethylformamid zu wobei die Temperatur auf 60 °C ansteigt. Anschließend rührt man eine Stunde bei 125 °C. Danach gibt man eine warme Lösung von 17,2 g Hydroxylammoniumchlorid in 100 ml Dimethylformamid zu und rührt noch 15 Minuten bei 120 °C. Man kühlt das Reaktionsgemisch auf Raumtemperatur und tropft es unter Rühren auf Eiswasser, wobei beige gefärbte Kristalle ausfallen. Man rührt weitere zwei Stunden, filtriert ab und trocknet den kristallinen Rückstand über Nacht bei 120 °C im Vakuum. Man erhält 23,5 g (84 % der Theorie) eines leicht beige gefärbten kristallinen Rückstands von Indol-Carbonitril **5**.
1.5. Schritt e: Herstellung von Verbindung **6**
   5 g Indol-Carbonitril **5** werden unter Schutzgasatmosphäre (N₂) in Methanol gelöst, mit Palladium auf Aktivkohle (Pd/C) versetzt und bei Raumtemperatur unter Normaldruck mit Wasserstoff bis zur vollständigen Umsetzung hydriert. Zur Aufarbeitung wird das Reaktionsgemisch filtriert und das Filtrat im Vakuum vom Lösungsmittel befreit. Der Rückstand wird in Methylacetat aufgenommen und mit Wasser extrahiert. Die organische Phase wird abgetrennt, getrocknet und eingeengt. Der Rückstand wird durch Chromatographie an Kieselgel mit Ethylacetat als Eluenten gereinigt. Man erhält 4,2 g (84% der Theorie) beige gefärbten kristallinen Rückstand von Verbindung **6**.
1.6. Schritt f: Herstellung von Verbindung **7**
   Unter Schutzgasatmosphäre löst man 18,2 g von Verbindung 6 in 1000 ml Tetrahydrofuran und kühlt die Lösung auf 0 °C. Anschließend tropft man eine Lösung aus 60 ml Vitride in 60 ml Toluol so zu, dass die Temperatur im Bereich von 2 bis 8 °C bleibt und rührt weitere drei Stunden bei 5 °C. Während der Zugabe des Vitride kann sich ein Niederschlag bilden, welcher welcher sich jedoch in der Regel beim anschließenden Rühren wieder auflöst. Überschüssiges Vitride wird durch Zugabe von 250 ml Wasser hydrolysiert, wobei ein Niederschlag ausfallen kann. Man gibt weitere 200 ml Wasser und 300 ml Ethylacetat zu und lässt über Nacht stehen. Anschließend wird das Reaktionsgemisch über Kieselgur filtriert, der Rückstand verworfen und die Phasen des Filtrats getrennt. Die organische Phase wird im Vakuum vom Lösungsmittel befreit und durch Chromatographie an Kieselgel mit Ethylacetat als Eluenten gereinigt. Man erhält 13,5 g (84% der Theorie) beige gefärbten kristallinen Rückstand von Verbindung **7**.
1.7. Schritt g: Herstellung von Verbindung **8**
   Eine Lösung von 6 g der Verbindung **7** in 80 ml Dichlormethan und 80 ml Tetrahydrofuran wird auf 2 °C gekühlt. Man gibt 2,3 ml Methansulfonsäurechlorid zu, rührt Kurz und gibt anschließend bei 2 bis 5 °C 5,3 ml Triethylamin zu. Anschließend rührt man noch 2 Stunden bei 2 °C. Danach gießt man das Reaktionsgemisch auf Eiswasser, extrahiert die wässrige Phase und befreit die vereinigten organischen Phasen im Vakuum vom Lösungsmittel. Man erhält 7,6 g (91 % der Theorie) braun gefärbten kristallinen Rückstand von Verbindung **8**.
1.8. Schritt h: Herstellung von Verbindung **10**
   Eine Suspension von 0,50 Gramm der Verbindung **8** in 10 ml Acetonitril wird unter Rühren mit 0,40 Gramm 4-(4-Fluorbenzyl)-piperidin (**9**) und 0,42 Gramm Natriumhydrogencarbonat versetzt und 24 Stunden bei 100 °C gerührt. Anschließend kühlt man das Reaktionsgemisch auf Raumtemperatur und entfernt das Lösungsmittel im Vakuum. Der erhaltene Rückstand wird durch Chromatographie an Kieselgel gereinigt, in Aceton gelöst und mit etherischer Salzsäure versetzt. Der sich bildende kristalline Niederschlag wird durch Filtration abgetrennt und getrocknet. Man erhält 230 mg (35% der Theorie) der Verbindung **10** (4-{3-[4-(4-Fluorbenzyl)-1-piperidyl]-propyl}-1H-indol-3-carbonitril als Hydrochlorid.

Das nachfolgende Formelschema betrifft die Herstellung von 4-{3-[4-(4-Fluorbenzyl)-1-piperidyl]-propyl}-1H-indol-3-carbonitril gemäß Beispiel 1. Die Kleinbuchstaben a bis h auf den Reaktionspfeilen korrespondieren mit den vorstehend beschriebenen Reaktionsschritten a bis h.

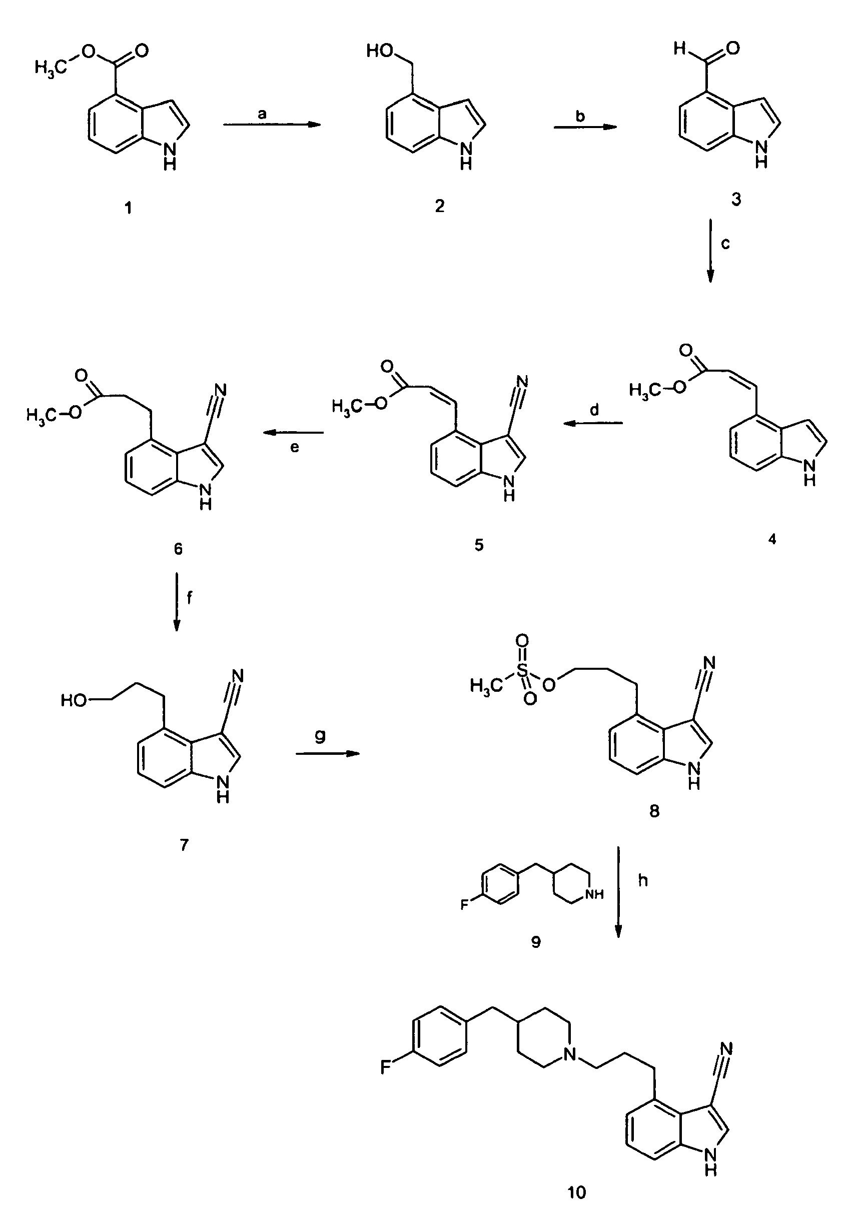

### Beispiel 2

### Verfahrensschritte a' bis g': Herstellung von Verbindung 18

Unter analoger Anwendung der in Beispiel 1 in den Reaktionsschritten a bis g durchgeführten Reaktionssequenz wurde gemäß nachstehenden Formelschema in den Reaktionsschritten a' bis h' die Verbindung **18** erhalten.

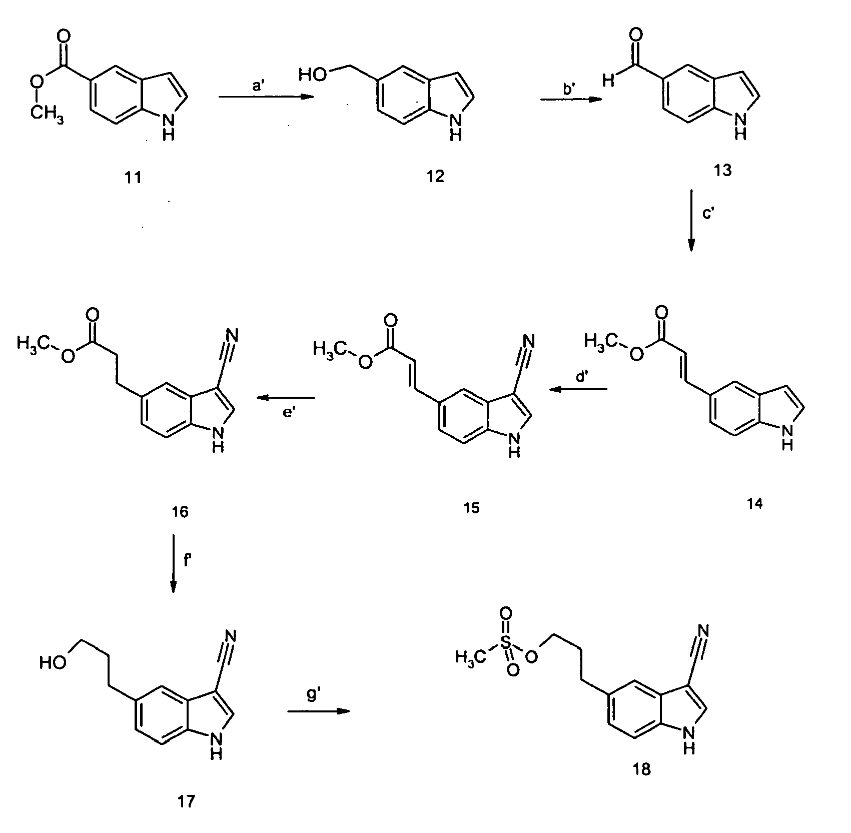

In Analogie zu Verfahrenschritt h von Beispiel 1 kann gemäß nachstehenden Bedingungen durch Umsetzung von Verbindung **18** mit Verbindung **19** 5-{4-[3-(3-Cyano-1H-indol-5-yl)-propyl]-piperazin-1-yl}-benzofuran-2-carbonsäureamid **20** erhalten werden:

### Schritt h': Herstellung von Verbindung 20

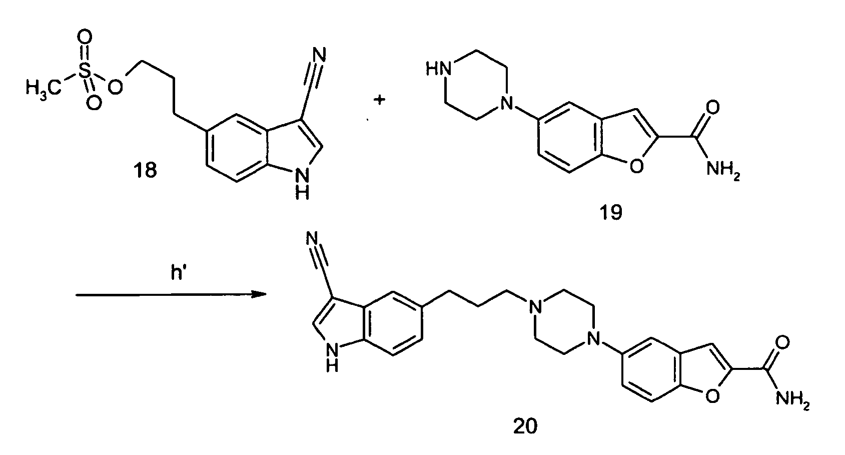

Eine Suspension von 1,00 Gramm der Verbindung **18** in 30 ml Acetonitril wird unter Rühren mit 0,90 g 5-Piperazin-1-yl-benzofuran-2-carbonsäureamid (**19**) und 1,4 g Ethyldiisopropylamin (DIPEA) versetzt und 18 Stunden unter Rückfluss gerührt. Anschließend kühlt man das Reaktionsgemisch auf Raumtemperatur, gibt Methanol (20 ml) zu und erhitzt weitere drei Stunden zum Rückfluß. Anschließend entfernt man das Lösungsmittel im Vakuum und reinigt den Rückstand durch Chromatographie an Kieselgel. Der verhaltene Rückstand wird in Aceton gelöst und mit etherischer Salzsäure versetzt. Der sich bildende kristalline Niederschlag wird durch Filtration abgetrennt und getrocknet. Man erhält 0,4 g (22% der Theorie) der Verbindung 5-{4-[3-(3-Cyano-1H-indol-5-yl)-propyl]-piperazin-1-yl}-benzofuran-2-carbonsäureamid **20** als Hydrochlorid.

### Beispiel 3

Analog zu dem in Beispiel 1 oder Beispiel 2 beschriebenen Verfahren können folgende Verbindungen erhalten werden:
5-{3-[4-(4-Cyano-phenyl)-piperazin-1-yl]-propyl}-1H-indol-3-carbonitril (**21**);
5-{4-[3-(3-Cyano-1H-indol-6-yl)-propyl]-piperazin-1-yl}-benzofuran-2-carbonsäureamid (**22**);
5-{3-[4-(2-Oxo-2H-chromen-6-yl)-piperazin-1-yl]-propyl}-1H-indol-3-carbonitril (**23**);
5-{4-[3-(3-Cyano-1H-indol-4-yl)-propyl]-piperazin-1-yl}-benzofuran-2-carbonsäureamid 24);
5-{3-[4-(1H-indo)-4-yl)-piperazin-1-yl]-propyl}-1-methansulfony)-1H-indol-3-carbonitril (**25**);
5-[3-(4-Oxo-1-phenyl-1,3,8-triaza-spiro[4.5]dec-8-yl)-propyl]-1H-indol-3-carbonitril (**26**);
5-[3-(4-Benzo[1,2,5]thiadiazol-4-yl-piperazin-1-yl)-propyl]-1H-indol-3-carbonitril (**27**);
3-{1-[3-(3-Cyano-1H-indol-5-yl)-propyl]-piperidin-4-yl}-1H-indole-5-carbonsäureamid (28);
5-[3-(4-Chinomin-8-yl-piperazin-1-yl)-propyl]-1H-indol-3-carbonitril (**29**);
5-{3-[4-(2,3-Dihydro-benzo[1,4]dioxin-5-yl)-piperazin-1-yl]-propyl}-1H-indol-3-carbonitril (30);
1-Methansulfonyl-5-[3-(4-oxo-1-phenyl-1,3,8-triaza-spiro[4.5]dec-8-yl)-propyl]-1H-indol-3-carbonitril (**31**);
5-{3-[4-(1H-Indol-4-yl)-piperazin-1-yl]-propyl}-1H-indol-3-carbonitril (**32**);
5-{3-[4-(1H-Indol-3-yl)-piperidin-1-yl]-propyl}-1H-indol-3-carbonitril (**33**);
5-{3-[4-(5-Fluor-1H-indol-3-yl)-piperidin-1-yl]-propyl}-1H-indol-3-carbonitril (**34**);
3-{1-[3-(3-Cyano-1H-indol-5-yl)-propyl]-piperidin-4-yl}-1H-indol-5-carbonitril (**35**);

5-{3-[4-(6-Fluor-1H-indol-3-yl)-piperidin-1-yl]-propyl}-1H-indol-3-carbonitril (**36**);
5-{3-[4-(4-Fluor-1H-indol-3-yl)-piperidin-1-yl]-propyl}-1H-indol-3-carbonitril (**37**);
5-[3-(4-Benzo[d]isothiazol-3-yl-piperazin-1-yl)-propyl]-1H-indol-3-carbonitril (38);
4-{1-[3-(3-Cyano-1H-indol-6-yl)-propyl]-piperidin-4-yloxy}-benzamid (**39**);
6-{3-[4-(2-Cyano-3-methoxy-phenyl)-piperazin-1-yl]-propyl}-1H-indol-3-carbonitril (**40**);
6-{3-[4-(4-Cyano-3-methoxy-phenyl)-piperazin-1-yl]-propyl}-1H-indol-3-carbonitril (**41**);
6-{3-[4-(4-Cyano-2-methoxy-phenyl)-piperazin-1-yl]-propyl}-1H-indol-3-carbonitril (**42**);
4-[3-(4-Pyrazol-1-ylmethyl-1-piperidyl)-propyl]-1H-indol-3-carbonitril (**43**);
6-{3-[4-(4-Fluorbenzyl)-1-piperidyl]-propyl}-1H-indol-3-carbonitril (**44**);
6-{3-[4-(2,4-Difluorbenzyl)-1-piperidyl]-propyl}-1H-indol-3-carbonitril (**45**);
6-{3-[4-(4-Fluorphenoxy)-1-piperidyl]-propyl}-1H-indol-3-carbonitril (**46**);
4-{3-[4-(2,4-Difluorbenzyl)-1-piperidyl]-propyl}-1H-indol-3-carbonitril (**47**);
4-{3-[4-(4-Fluorphenoxy)-1-piperidyl]-propyl}-1H-indol-3-carbonitril (**48**);
5-{3-[4-(4-Fluorphenoxy)-1-piperidyl]-propyl}-1H-indol-3-carbonitril (**49**);
5-{3-[4-(4-Fluorbenzyl)-1-piperidyl]-propyl}-1H-indol-3-carbonitril (**50**);
5-{3-(4-(2,4-Difluorbenzyl)-1-piperidyl]-propyl}-1H-indol-3-carbonitril (**51**).

Die Verbindungen können durch HPLC-Chromatographie gereinigt und/oder charakterisiert werden. Die Charakterisierung der Verbindungen über die Retensionszeit (Rₜ) kann auf einer Säule 3µ Silica-Rod mit einem 210 Sekunden Gradienten von 20 bis 100 % Wasser/Acetonitril/0,01% Trifluoressigsäure bei einem Fluss von 2,2 ml/Minute und einer Detektion bei einer Wellenlänge von 220 Nanometer erfolgen.

Physikalische Konstanten und analytische Daten (Massenspektrometrische Daten (HPLC-MS)) der wie vorstehend synthetisierten Verbindungen 20-43 sind in Tabelle I zusammengefasst.

**Tabelle I**

| **Verbindung** | **Struktur** | **MW g/mol** | **[M+H] HPLC-MS** | **Rₜ(HPLC) /min** |
|---|---|---|---|---|
| 20 | 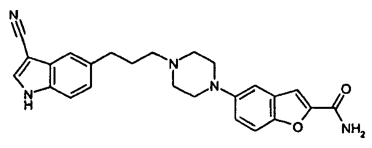 | | 429 | |
| 21 | 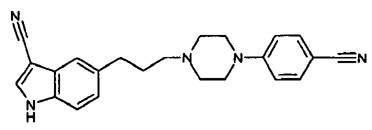 | | 370 | |
| 22 | 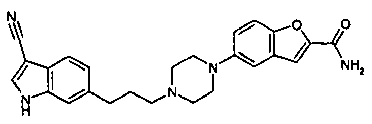 | | 429 | |
| 23 | 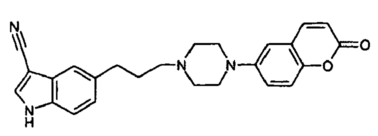 | | 413 | |
| 24 | 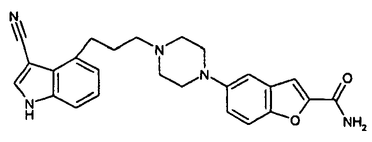 | | 429 | |
| 25 | 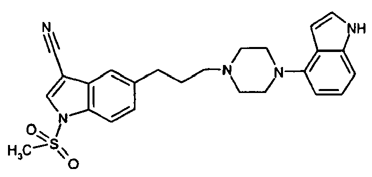 | | 463 | |
| 26 | 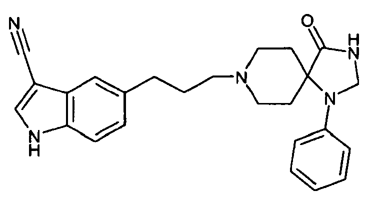 | | 415 | |
| 27 | 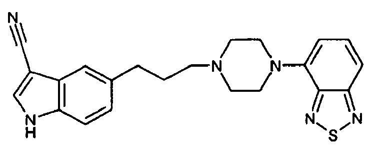 | | 404 | |
| 28 | 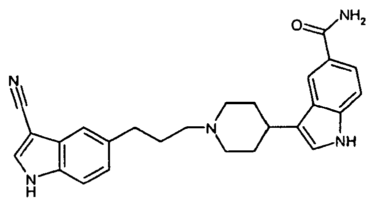 | | 426 | |
| 29 | 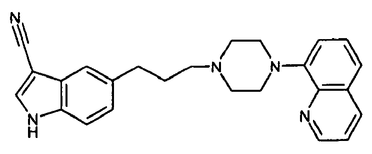 | | 397 | |
| 30 | 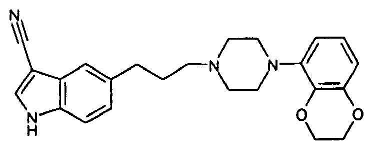 | | 403 | |
| 31 | 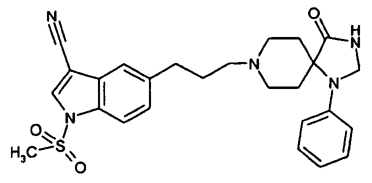 | | 493 | |
| 32 | 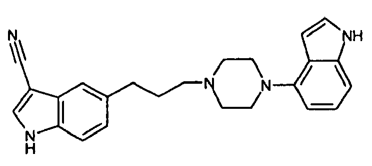 | | 384 | |
| 33 | 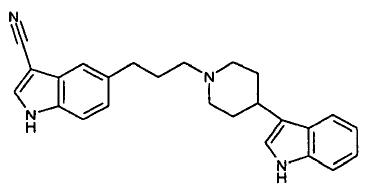 | | 384 | |
| 34 | 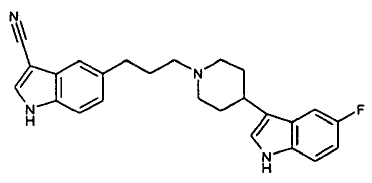 | | 401 | |
| 35 | 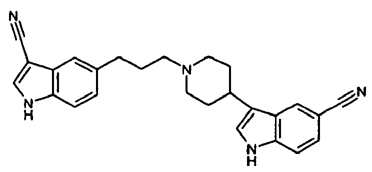 | | 409 | |
| 36 | 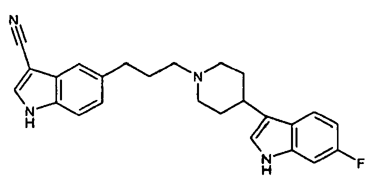 | | 401 | |
| 37 | 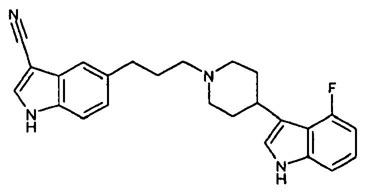 | | 401 | |
| 38 | 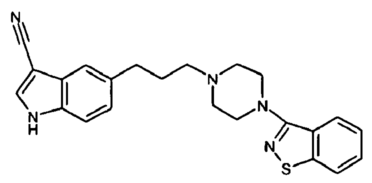 | | 403 | |
| 39 | 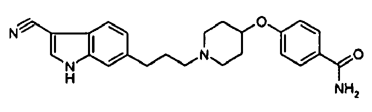 | | 403 | |
| 40 | 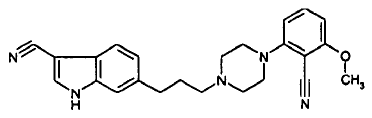 | | 400 | |
| 41 | 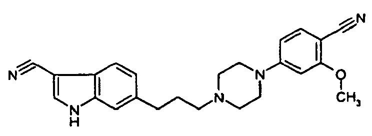 | | 400 | |
| 42 | 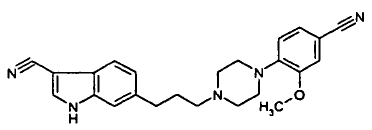 | | 400 | |
| 43 | 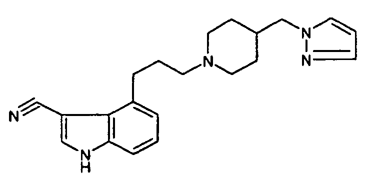 | | 348 | |

Physikalische Konstanten und analytische Daten (Massenspektrometrische Daten (FAB-MS) und Retensionszeiten (HPLC)) der wie vorstehend synthetisierten Verbindungen 10 und 44-51 sind in Tabelle II zusammengefasst.

**Tabelle II**

| **Verbindung** | **Struktur** | **MW g/mol** | **[M+H] HPLC-MS** | **Rₜ(HPLC) /min** |
|---|---|---|---|---|
| 40 | 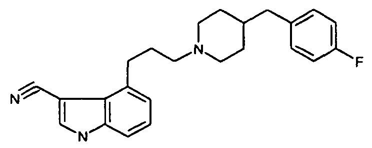 | 375.49 | 376.20 | 1.428 |
| 44 | 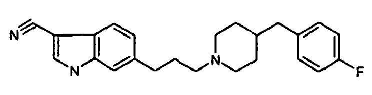 | 375.49 | 376 | 1.203 |
| 45 | 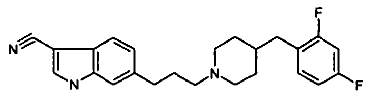 | 393.48 | 394 | 1.236 |
| 46 | 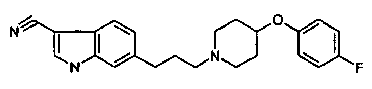 | 377.47 | 378 | 1.172 |
| 47 | 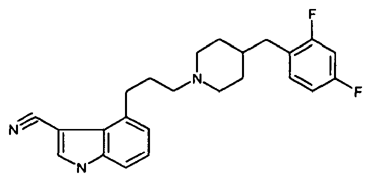 | 393.48 | 394 | 1.443 |
| 48 | 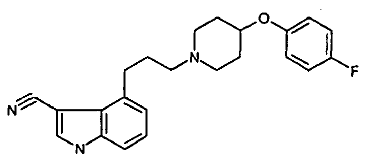 | 377.47 | 378 | 1.347 |
| 49 | 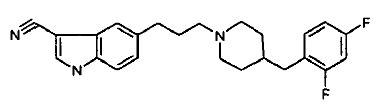 | | 394 | |
| 50 | 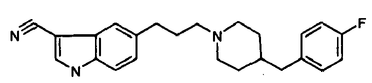 | | 376 | |
| 51 | 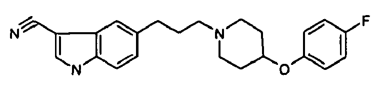 | | 378 | |

Die nachfolgenden Beispiele betreffen pharmazeutische Zubereitungen:

### Beispiel A: Injektionsgläser

Eine Lösung von 100 g des Wirkstoffes der Formel la und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g des Wirkstoffes der Formel la mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g des Wirkstoffes der Formel Ia, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg des Wirkstoffes der Formel la mit 99,5 g Vaseline unter aseptischen Bedingungen.

### Beispiel E: Tabletten

Ein Gemisch von 1 kg Wirkstoff der Formel Ia, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel Ia werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel Ia in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel Ia in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel Ia 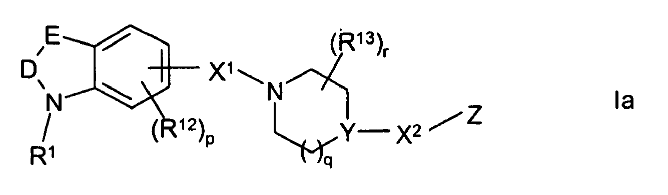 worin
R¹ für H, A oder SO₂A,
A für geradkettiges Alkyl mit 1 bis 4 C-Atomen oder verzweigtes Alkyl mit 3 bis 6 C-Atomen, und
D-E für R²C=CR⁴ steht, worin R² ausgewählt ist unter H, A und Cycloalkyl mit 3 bis 7 C-Atomen, und R⁴ für Hal, CH₂Hal, CH(Hal)₂, C(Hal)₃, NO₂, (CH₂)ₙCN, (CH₂)nCOOR⁶, (CH₂)ₙCON(R⁶)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙS(O)_{w}A, (CH₂)ₙOOCR⁶, (CH₂)ₙCOR⁶, (CH₂)ₙCO(CH₂)ₘAr, (CH₂)ₙCO(CH₂)ₘHet, (CH₂)ₙCOO(CH₂)ₘAr, (CH₂)ₙCOO(CH₂)ₘHet, (CH₂)nOR⁶, (CH₂)nO(CH₂)ₘAr, (CH₂)ₙO(CH₂)ₘHet, (CH₂)ₙSR⁶, (CH₂)ₙS(CH₂)ₘAr, (CH₂)ₙS(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂)ₘAr, (CH₂)ₙN(R⁶)CO(CH₂)ₘAr, (CH₂)ₙCON(R⁶)(CH₂)mHet, (CH₂)ₙN(R⁶)CO(CH₂)ₘHet, (CH₂)ₙN(R⁶)₂, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₙAr, (CH₂)ₙNR⁶COO(CH₂)ₘHet, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶ oder (CH₂)ₙCHR⁶CH₂OR⁶ steht, worin
Het für einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)₂, S(O)_{w}A und/oder OOCR⁶ substituierten, gesättigten, ungesättigten oder aromatischen mono- oder bicyclischen heterocyclischen Rest steht,
Ar für einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)₂, S(O)_{w}A und/oder OOCR⁶ substituierten aromatischen Kohlenwas- serstofferest mit 6 bis 14 Kohlenstoffatomen steht,
w für 0, 1, 2 oder 3, und
m für 0, 1, 2, 3, 4 oder 5 steht und
n für 0, 1, 2 oder 3 steht;
X¹ für (CHR⁷)_{g}, oder (CHR⁷)ₕ-Q-(CHR⁸)ₖ steht, worin
Q ausgewählt ist unter O, S, N-R⁶, (O-CHR⁷)_{g}, (CHR⁷-O)_{g}, CR⁹=CR¹⁰, (O-CHR⁹CHR¹⁰)_{g}, (CHR⁹CHR¹⁰-O)_{g}, C=O, C=S, C=NR⁶, CH(OR⁶), C(OR⁶)(OR⁶), C(=O)O, OC(=O), OC(=O)O, C(=O)N(R⁶), N(R⁶)C(=O), OC(=O)N(R⁶), N(R⁶)C(=O)O, CH=N-O, CH=N-NR⁶, OC(O)NR⁶, NR⁶C(O)O, S=O, SO₂, SO₂NR⁶ und NR⁶SO₂,
g für 1, 2, 3, 4, 5 oder 6 steht,
h für 0, 1, 2, 3, 4, 5 oder 6 steht,
k für 1, 2 oder 3 steht, und
R⁶ unabhängig ausgewählt ist unter H, A oder Cycloalkyl mit 3 bis 7 C-Atomen,
R⁷, R⁸, R⁹ und R¹⁰ unabhängig ausgewählt sind unter für den für R² und R⁴ angegebenen Bedeutungen;
Y für CH, N, COR¹¹, CSR¹¹, einen unsubstituierten oder substi- tuierten spiro-verknüpften Carbocyclus mit 5 bis 7 Kohlen- stoffatomen oder einen unsubstituierten oder substituierten spiro-verknüpften Heterocyclus, ausgewählt unter Strukturen der Formeln 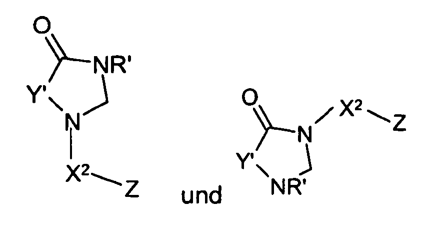 worin R' für H, A, (CH₂)ₙHet, (CH₂)ₙAr, Cycloalkyl mit 3 bis 7 C-Atomen oder SO₂A steht, und Y' für das spiro- verknüpfende C-Atom des spiro-verknüpften Heterocyclus steht, steht,
R¹¹ für H, A, (CH₂)ₙHet, (CH₂)ₙAr oder Cycloalkyl mit 3 bis 7 C-Atomen steht,
X² für eine Bindung steht oder unabhängig unter den für X¹ an- gegebenen Bedeutungen ausgewählt ist, und bevorzugt für eine Bindung oder O, S, N-R⁷, CH₂ oder CH₂CH₂ steht,
p, q, r unabhängig voneinander für 0, 1, 2 oder 3
und
Hal für F, Cl, Br oder I steht, und
R¹², R¹³ unabhängig voneinander ausgewählt sind unter den von H verschiedenen Bedeutungen von R⁴,
Z H bedeutet oder für einen gesättigten, ein- oder mehrfach e- thylenisch ungesättigten Carbocyclus mit 5 bis 10 C-Atomen, einen aromatischen Carbocyclus, ausgewählt unter Phenyl und Naphthyl, oder einen gesättigten, ein- oder mehrfach e- thylenisch ungesättigten oder aromatischen Heterocyclus, ausgewählt unter Furanyl, Thiophenyl, Pyrrolyl, Imidazolyl, Pyrazolyl, Triazolyl, Tetrazolyl, Thiazolyl, Oxazolyl, Isoxazolyl, Oxadiazolyl, Oxopyridyl, Thiadiazolyl, Isothiazolyl, Pyridyl, Py- ridazyl, Pyrazinyl, Pyrimidyl, Chinolinyl, Isochinolinyl, Benzo- furanyl, Benzothiophenyl, Benzo[1,4]dioxoinyl, 2,3-Dihydrobenzo[1,4]dioxinyl, Benzothiadiazolyl, Chromenyl, 2-Oxo-chromenyl, Indolyl und Indazolyl, steht, wobei der Car- bocyclus oder Heterocyclus ein- oder mehrfach substituiert sein kann, wobei die Substituenten unabhängig voneinander ausgewählt sind unter, A, Cycloalkyl mit 3 bis 7 C-Atomen, Hal, CH₂Hal, CH(Hal)₂, C(Hal)₃, NO₂, (CH₂)ₙCN, (CH₂)ₙN(R⁶)₂, (CH₂)ₙN(R⁶)Ar, (CH₂)ₙN(R⁶)Het, (CH₂)ₙN(Ar)₂, (CH₂)ₙN(Het)₂, (CH₂)ₙCOOR⁶, (CH₂)ₙCOOAr, (CH₂)ₙCOOHet, (CH₂)ₙCON(R⁶)₂, (CH₂)ₙCON(R⁶)Ar, (CH₂)ₙCON(R⁶)Het, (CH₂)ₙCON(Ar)₂, (CH₂)ₙCON(Het)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙSO₂NR⁶(CH₂)ₘAr, (CH₂)ₙSO₂NR⁶(CH₂)ₘHet, (CH₂)ₙS(O)_{w}R⁶, (CH₂)ₙS(O)_{w}Ar, (CH₂)ₙS(O)_{w}Het, (CH₂)ₙOOCR⁶, (CH₂)ₙHet, (CH₂)ₙAr, (CH₂)ₙCOR⁶, (CH₂)ₙCO(CH₂)ₘAr, (CH₂)ₙCO(CH₂)ₘHet, (CH₂)ₙCOO(CH₂)ₘAr, (CH₂)ₙCOO(CH₂)ₘHet, (CH₂)ₙOR⁶, (CH₂)ₙO(CH₂)ₘAr, (CH₂)ₙO(CH₂)ₘHet, (CH₂)ₙSR⁶, (CH₂)ₙS(CH₂)ₘAr, (CH₂)ₙS(CH₂)ₘHet, (CH₂)ₙN(R⁶)(CH₂)ₘAr, (CH₂)nN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂)ₘAr, (CH₂)ₙN(R⁶)CO(CH₂)ₘAr, (CH₂)ₙCON(R⁶)(CH₂)ₘHet, (CH₂)ₙN(R⁶)CO(CH₂)ₘHet, CH=N-OA, CH₂CH=N-OA, (CH₂)ₙNHOA, (CH₂)ₙCH=N-Het, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₙHet, (CH₂)ₙNR⁶COOR⁶, (CH₂)ₙNR⁶COO(CH₂)ₘAr, (CH₂)ₙNR⁶COO(CH₂)ₘHet, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, CH=CHCH₂NR⁶Het, CH=CHCH₂N(R⁶)₂, CH=CHCH₂OR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶, (CH₂)ₙCHR⁶CH₂OR⁶, (CH₂)ₙOCN und (CH₂)ₙNCO,
sowie die pharmazeutisch verwendbaren Salze, Solvate und Stereoisomere und Mischungen davon.

2. Verbindungen der Formel Ia nach Anspruch 1, worin
R¹ H oder SO₂A bedeutet;
D-E für R²C=CR⁴ steht,
R² H, Alkyl mit 1 bis 3 C-Atomen, Alkoxyalkyl mit 2 bis 5 C- Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ bedeutet;
R⁴ H, Alkyl mit 1 bis 3 C-Atomen, Alkoxyalkyl mit 2 bis 5 C- Atomen, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ oder (CH₂)ₙS(O)_{w}R⁶ und
R⁶ H oder A bedeutet,
n für 0 oder 1 steht,
X¹ (CHR⁷)_{g} bedeutet, worin g für 2, 3 oder 4 steht, oder Q-(CHR⁸)ₖ bedeutet, worin k für 1, 2 oder 3 steht und Q aus- gewählt ist unter O, S, N-R⁶, CONR⁶, C=O, C=S, S=O, SO₂, SO₂NR⁶ und NR⁶SO₂,
R⁷, R⁸ unabhängig voneinander ausgewählt sind unter H und A,
Y für CH, COR¹¹ oder N steht,
R¹¹ H oder A, und
X² CH₂, CH₂CH₂, HCOH, O, S, N-R⁶, CONR⁶, C=O oder eine Bindung bedeutet, und
R¹² unabhängig ausgewählt ist unter A, Hal, CN, NO₂, OR⁶, N(R⁶)₂, COOR⁶, CON(R⁶)₂, COR⁶, SO₂N(R⁶)₂ und S(O)_{w}A.

3. Verbindungen nach Anspruch 1 oder 2, worin die Gruppe X²-Z ausgewählt ist unter den Gruppen 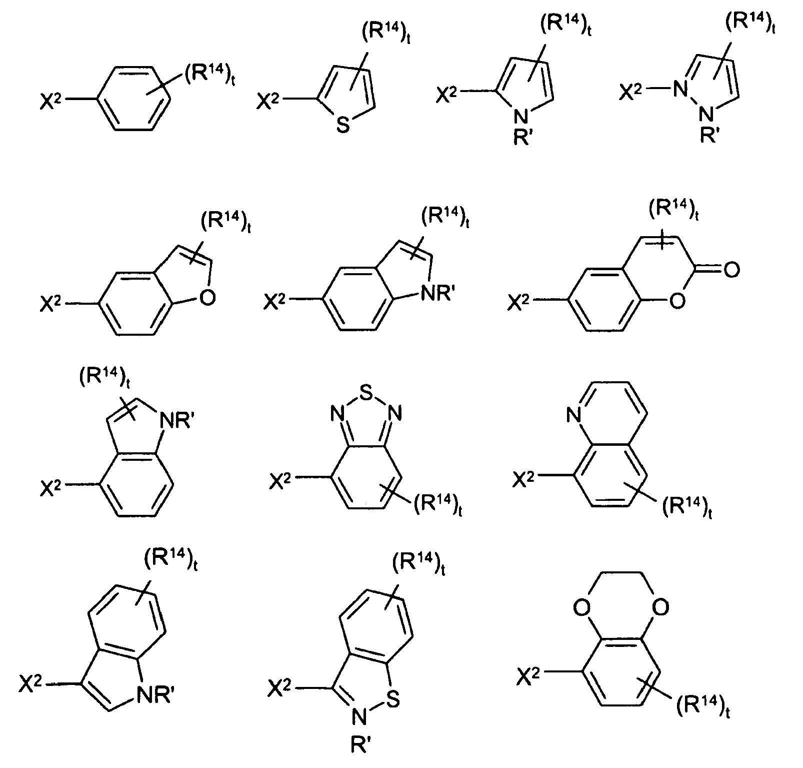 worin
R¹⁴ unabhängig ausgewählt ist unter Hal, A, (CH₂)ₙHet, (CH₂)ₙAr, (CH₂)ₙCOO(CH₂)ₘAr, (CH₂)ₙCOO(CH₂)ₘHet, (CH2)ₙOR⁶, (CH₂)ₙO(CH₂)ₘAr, (CH₂)ₙO(CH₂)ₘHet, (CH₂)ₙN(R⁶)(CH₂)mAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙN(R⁶)₂, (CH₂)ₙNHOA, (CH₂)ₙ(R⁶)Het, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, steht,
t für 0, 1, 2 oder 3, und
R' R' für H, A, (CH₂)ₙHet, (CH₂)ₙAr, Cycloalkyl mit 3 bis 7 C- Atomen oder SO₂A steht.

4. Verbindungen nach Anspruch 1 oder 2, worin die Gruppe X²-Z ausgewählt ist unter den Gruppen 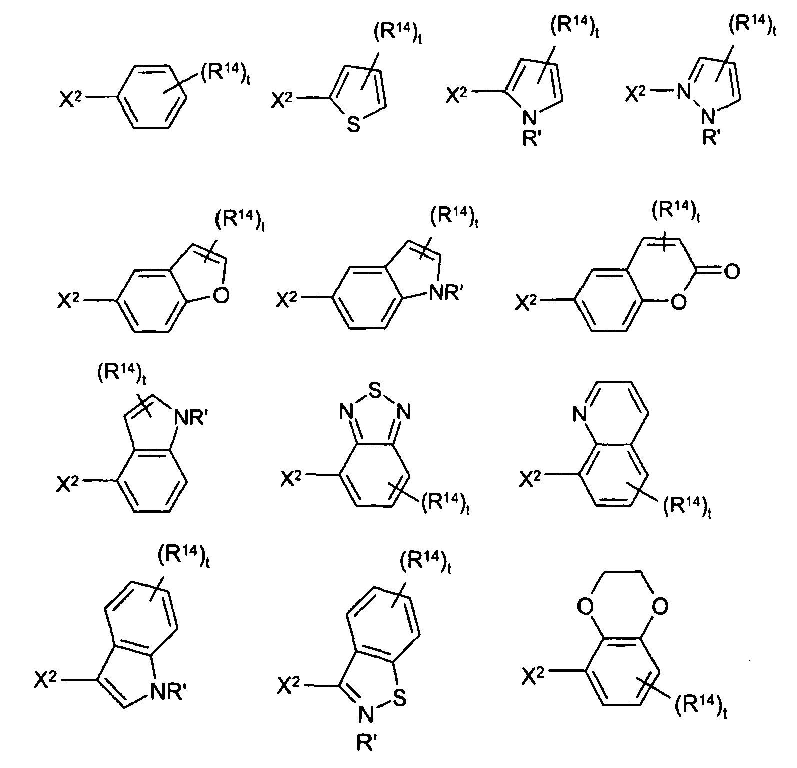 worin
R¹⁴ unabhängig voneinander für Hal, NO₂, OR⁶, N(R⁶)₂, CN, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ und/oder C(NH)NOH steht,
w für 0, 1, 2 oder 3,
t für 0, 1, 2 oder 3, und
R' R' für H, A, (CH₂)ₙHet, (CH₂)ₙAr, Cycloalkyl mit 3 bis 7 C- Atomen oder SO₂A steht.

5. Verbindungen nach Anspruch 1, 2, 3 oder 4, ausgewählt unter
a) 6-{3-[4-(4-Fluorbenzyl)-1-piperidyl]-propyl}-1H-indol-3-carbonitril;
b) 6-{3-[4-(2,4-Difluorbenzyl)-1-piperidyl]-propyl}-1H-indol-3-carbonitril;
c) 6-{3-[4-(4-Fluorphenoxy)-1-piperidyl]-propyl}-1H-indol-3-carbonitril;
d) 4-{3-[4-(4-FLuorbenzyl)-1-piperidyl]-propyl}-1H-indol-3-carbonitril;
e) 4-{3-[4-(2,4-Difluorbenzyl)-1-piperidyl]-propyl}-1H-indol-3-carbonitril;
f) 4-{3-[4-(4-Fluorphenoxy)-1-piperidyl]-propyl}-1H-indol-3-carbonitril;
g) 5-{3-[4-(4-Fluorphenoxy)-1-piperidyl]-propyl}-1H-indol-3-carbonitril;
h) 5-{3-[4-(4-Fluorbenzyl)-1-pipendyl]-propyl}-1H-indol-3-carbonitril;
i) 5-{3-[4-(2,4-Difluorbenzyl)-1-piperidyl]-propyl}-1H-indol-3-carbonitril;
j) 5-{3-[4-(4-Cyano-phenyl)-piperazin-1-yl]-propyl}-1H-indol-3-carbonitril;
k) 5-{4-[3-(3-Cyano-1H-indol-6-yl)-propyl]-piperazin-1-yl}-benzofuran-2-carbonsäureamid;
l) 5-{3-[4-(2-Oxo-2H-chromen-6-yl)-piperazin-1-yl]-propyl}-1H-indol-3-carbonitril;
m) 5-{4-[3-(3-Cyano-1H-indol-4-yl)-propyl]-piperazin-1-yl}-benzofuran-2-carbonsäureamid;
n) 5-{4-[3-(3-Cyano-1H-indol-5-yl)-propyl]-piperazin-1-yl}-benzofuran-2-carbonsäureamid
o) 5-{3-[4-(1H-Indol-4-yl)-piperazin-1-yl]-propyl}-1-methansulfonyl-1H-indol-3-carbonitril;
p) 5-[3-(4-Oxo-1-phenyl-1,3,8-triaza-spiro[4.5]dec-8-yl)-propyl]-1H-indol-3-carbonitril;
q) 5-[3-(4-Benzo[1,2,5]thiadiazol-4-yl-piperazin-1-yl)-propyl]-1H-indol-3-carbonitril;
r) 3-{1-[3-(3-Cyano-1H-indol-5-yl)-propyl]-piperidin-4-yl}-1H-indole-5-carbonsäureamid;
s) 5-[3-(4-Chinolin-8-yl-piperazin-1-yl)-propyl]-1H-indol-3-carbonitril;
t) 5-{3-[4-(2,3-Dihydro-benzo[1,4]dioxin-5-yl)-piperazin-1-yl]-propyl}-1H-indol-3-carbonitril;
u) 1-Methansulfonyl-5-[3-(4-oxo-1-phenyl-1,3,8-triaza-spiro[4.5]dec-8-yl)-propyl]-1H-indol-3-carbonitril;
v) 5-{3-[4-(1H-Indol-4-yl)-piperazin-1-yl]-propyl}-1H-indol-3-carbonitril;
w) 5-{3-[4-(1H-Indol-3-yl)-piperidin-1-yl]-propyl}-1H-indol-3-carbonitril;
x) 5-{3-[4-(5-Fluor-1H-indol-3-yl)-piperidin-1-yl]-propyl}-1H-indol-3-carbonitril;
y) 3-{1-[3-(3-Cyano-1H-indol-5-yl)-propyl]-piperidin-4-yl}-1H-indol-5-carbonitril;
z) 5-{3-[4-(6-Fluor-1H-indol-3-yl)-piperidin-1-yl]-propyl}-1H-indol-3-carbonitril;
aa) 5-{3-[4-(4-Fluor-1H-indol-3-yl)-piperidin-1-yl]-propyl}-1H-indol-3-carbonitril;
bb) 5-[3-(4-Benzo[d]isothiazol-3-yl-piperazin-1-yl)-propyl]-1H-indol-3-carbonitril;
cc) 4-{1-[3-(3-Cyano-1H-indol-6-yl)-propyl]-piperidin-4-yloxy}-benzamid;
dd) 6-{3-[4-(2-Cyano-3-methoxy-phenyl)-piperazin-1-yl]-propyl}-1H-indol-3-carbonitril;
ee) 6-{3-[4-(4-Cyano-3-methoxy-phenyl)-piperazin-1-yl]-propyl}-1H-indol-3-carbonitril;
ff) 6-{3-[4-(4-Cyano-2-methoxy-phenyl)-piperazin-1-yl]-propyl}-1H-indol-3-carbonitril;
gg) 4-[3-(4-Pyrazol-1-ylmethyl-1-piperidyl)-propyl]-1H-indol-3-carbonitril,
hh) N-(6-{4-[3-(3-Cyano-1H-indol-5-yl)-propyl]-piperazin-1-yl}2-oxo-2H-chromen-3-yl)-acetamid,
ii) 5-{3-[(Pyridin-3-ylmethyl)-amino]-propyl}-1H-indol-3-carbonitril,
jj) 5-{3-[4-(2,3-Dihydro-benzo[1,4]dioxin-6-yl)-piperazin-1-yl]-propyl}-1H-indol-3-carbonitril,
kk) 5-[3-(4-Pyrimidin-2-yl-piperazin-1-yl)-propyl]-1H-indol-3-carbonitril,
ll) 5-{3-[4-(3-Methoxy-phenyl)-3-methyl-piperazin-1-yl]-propyl}-1H-indol-3-carbonitril,
mm) 5-{3-[4-(1-Methyl-1H-imidazo[4,5-c]pyridin-4-yl)-piperazin-1-yl]-propyl}-1H-indol-3-carbonitril,
nn) N-(4-{1-[3-(3-Cyano-1H-indol-5-yl)-propyl]-piperidin-4-ylmethyl}-phenyl)-acetamid,
oo) 5-{3-[4-(4-Pyridin-3-yl-thiazol-2-yl)-piperazin-1-yl]-propyl}-1H-indol-3-carbonitril,
pp) 2-{4-[3-(3-Cyano-1H-indol-5-yl)-propyl]-piperazin-1-yl}-thiazol-4-carbonsäureethylester,
qq) 5-{3-[3-(2-Oxo-pyrrolidin-1-yl)-propylamino]-propyl}-1H-indole-3-carbonitril,
rr) 5-{3-[4-(3-Amino-2-oxo-2H-chromen-6-yl)-piperazin-1-yl]-propyl}-1H-indol-3-carbonitril,
ss) (6-{4-[3-(3-Cyano-1H-indol-5-yl)-propyl]-piperazin-1-yl}-2-oxo-2H-chromen-3-yl)- carbaminsäuremethylester,
tt) 2-{4-[3-(3-Cyano-1H-indol-5-yl)-propyl]-piperazin-1-yl}-thiazol-4-carbonsäureamid,
uu) 4-[3-(3-Cyano-1H-indol-5-yl)-propyl]-piperazin-1-thiocarbonsäureamid;
sowie deren Salzen und Solvaten.

6. Verfahren zur Herstellung von Verbindungen der Formel Ia nach Anspruch 1 sowie ihrer Salze, **dadurch gekennzeichnet, daß** man
a) eine Verbindung der Formel II 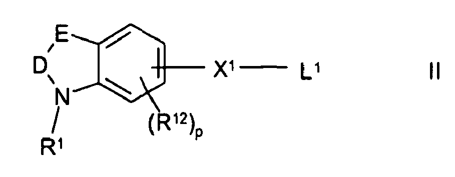 worin
L¹ Cl, Br, I, OH, eine reaktionsfähig veresterte OH-Gruppe, ausge- wählt unter Alkylsulfonyloxy-Gruppen mit 1-6 C-Atomen und Arylsulfonyloxy-Gruppen mit 6-10 C-Atomen, oder eine Diazoni- umgruppe bedeutet und R¹, D, E, R¹², p und X¹ die in Anspruch 1 angegebenen Bedeutungen haben,
b) mit einer Verbindung der Formel III umsetzt, 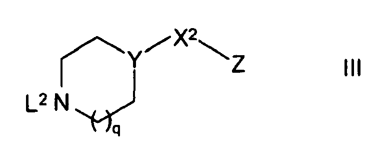 worin
L² H oder ein Metallion bedeutet und q, Y, X² und Z die in Anspruch 1 angegebenen Bedeutungen haben,
und gegebenenfalls
c) die erhaltene Verbindung der Formel Ia durch Behandeln mit einer Säure in eines ihrer Salze überführt.

7. Pharmazeutische Zubereitung, **gekennzeichnet durch** einen Gehalt an mindestens einer Verbindung der Formel Ia nach Anspruch 1 und/oder einem ihrer physiologisch unbedenklichen Salze und/oder eines ihrer Solvate.

8. Verbindungen der Formel Ia nach Anspruch 1 und ihre physiologisch unbedenklichen Salze und Solvate als Arzneimittel.

9. Verwendung der Verbindungen der Formel Ia nach Anspruch 1 zur Herstellung eines Medikaments zur Prophylaxe und/oder Therapie von Erkrankungen, bei denen 5HT eine Rolle spielt, **dadurch gekennzeichnet, dass** die Erkrankungen ausgewählt sind aus der Gruppe umfassend Depression, Schlaganfall, cerebrale Ischämien, extrapyramidal-motorischer Nebenwirkungen von Neuroleptika sowie des Morbus Parkinson, Alzheimer Krankheit, amyotrophe Lateralsklerose, Hirn- und Rückenmarkstraumata, Zwangsverhalten, Schlafstörungen, tardiver Dyskinesien, Lernstörungen, altersabhängiger Erinnerungsstörungen, Essstörungen und Sexualfunktionsstörungen.

10. Verwendung von Verbindungen der Formel Ia nach Anspruch 1 und/- oder ihre physiologisch unbedenklichen Salze oder Solvate zur Herstellung eines Arzneimittels zur Prophylaxe und/oder Behandlung von Schizophrenie, Depression, Demenz, Parkinsonschen Krankheit, Morbus Alzheimer, Lewy Bodies Dementia, Huntington, Tourette Syndrom, Angst, Lern- und Erinnerungseinschränkungen, neurodegenerativen Erkrankungen, kognitiven Beeinträchtigungen, sowie Nikotinabhängigkeit und Schmerzen.

11. Verbindungen der Formel Ia nach Anspruch 1 und/oder von deren physiologisch unbedenklichen Salzen zur Verwendung bei der Bekämpfung von cerebrovaskulären Krankheiten, Epilepsie, Schizophrenie, der Alzheimer-, der Parkinson- bzw. der Huntington-Krankheit, cerebralen Ischämien, Infarkten oder Psychosen.

12. Verfahren zur Herstellung pharmazeutischer Zubereitungen, **dadurch gekennzeichnet, daß** man eine Verbindung der Formel Ia nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

13. Verwendung von Verbindungen der Formel II 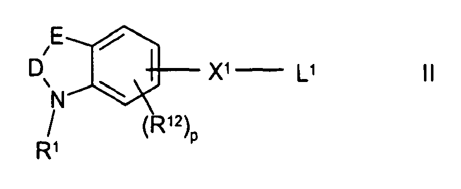 worin
L¹ Cl, Br, I, OH, eine reaktionsfähig veresterte OH-Gruppe, ausge- wählt unter Alkylsulfonyloxy-Gruppen mit 1-6 C-Atomen und Arylsulfonyloxy-Gruppen mit 6-10 C-Atomen, oder eine Diazoni- umgruppe bedeutet und R¹, D, E, R¹², p und X¹ die in Anspruch 1 angegebenen Bedeutungen haben, zur Herstellung von Ver- bindungen der Formel Ia nach Anspruch 1.

14. Verwendung von Verbindungen der Formel III 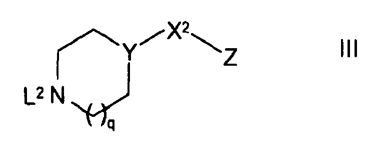 worin
L² H oder ein Metallion bedeutet und q, Y, X² und Z die in An- spruch 1 angegebenen Bedeutungen haben, zur Herstellung von Verbindungen der Formel Ia nach Anspruch 1.

## Claims

1. Compounds of the formula Ia 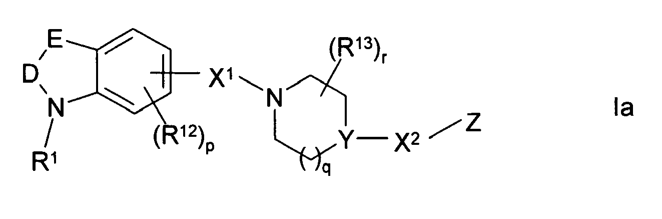 in which
R¹ stands for H, A or SO₂A,
A stands for straight-chain alkyl having 1 to 4 C atoms or branched alkyl having 3 to 6 C atoms, and
D-E stands for R²C=CR⁴, in which R² is selected from H, A and cycloalkyl having 3 to 7 C atoms, and R⁴ stands for Hal, CH₂Hal, CH(Hal)₂, C(Hal)₃, NO₂, (CH₂)ₙCN, (CH₂)ₙCOOR⁶, (CH₂)ₙCON(R⁶)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙS(O)_{w}A, (CH₂)ₙOOCR⁶, (CH₂)ₙCOR⁶, (CH₂)ₙCO(CH₂)ₘAr, (CH₂)ₙCO(CH₂)ₘHet, (CH₂)ₙCOO(CH₂)ₘAr, (CH₂)ₙCOO(CH₂)ₘHet, (CH₂)ₙOR⁶ (CH₂)ₙO(CH₂)ₘAr, (CH₂)ₙO(CH₂)ₘHet, (CH₂)ₙSR⁶ (CH₂)ₙS(CH₂)ₘAr, (CH₂)ₙS(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂)ₘAr, (CH₂)ₙN(R⁶)CO(CH₂)ₘAr, (CH2)ₙCON(R⁶)(CH₂)ₘHet, (CH₂)ₙN(R⁶)CO(CH₂)ₘHet, (CH₂)ₙN(R⁶)₂, (CH₂)ₙOCOR⁶ (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶ ,(CH₂)ₙN(CH₂CONH₂)CONH₂, (CH₂)ₙCHR⁶COR⁶, (CH₂)ₙCHR⁶COOR⁶ or (CH₂)ₙCHR⁶CH₂OR⁶, in which
Het stands for a saturated, unsaturated or aromatic mono- or bicyclic heterocyclic radical which is unsubstituted or mono- substituted or polysubstituted by A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)₂, S(O)_{w}A and/or OOCR⁶,
Ar stands for an aromatic hydrocarbon radical having 6 to 14 carbon atoms which is unsubstituted or monosubstituted or polysubstituted by A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)₂, S(O)_{w}A and/or OOCR⁶,
w stands for 0, 1, 2 or 3, and
m stands for 0, 1, 2, 3, 4 or 5, and
n stands for 0, 1, 2 or 3,
X¹ stands for (CHR⁷)_{g} or (CHR⁷)ₕ-Q-(CHR⁸)ₖ, in which
Q is selected from O, S, N-R⁶, (O-CHR⁷)_{g}, (CHR⁷-O)_{g}, CR⁹=CR¹⁰, (O-CHR⁹CHR¹⁰)_{g}, (CHR⁹CHR¹⁰-O)_{g}, C=O, C=S, C=NR⁶, CH(OR⁶), C(OR⁶)(OR⁶), C(=O)O, OC(=O), OC(=O)O, C(=O)N(R⁶), N(R⁶)C(=O), OC(=O)N(R⁶), N(R⁶)C(=O)O, CH=N-O, CH=N-NR⁶, OC(O)NR⁶, NR⁶C(O)O, S=O, SO₂, SO₂NR⁶ and NR⁶SO₂,
g stands for 1, 2, 3, 4, 5 or 6,
h stands for 0, 1, 2, 3, 4, 5 or 6,
k stands for 1, 2 or 3, and
R⁶ is selected, independently, from H, A or cycloalkyl having 3 to 7 C atoms,
R⁷, R⁸, R⁹ and R¹⁰ are selected, independently, from the meanings indicated for R² and R⁴,
Y stands for CH, N, COR¹¹, CSR¹¹, an unsubstituted or substi- tuted spiro-linked carbocycle having 5 to 7 carbon atoms or an unsubstituted or substituted spiro-linked heterocycle selected from structures of the formulae 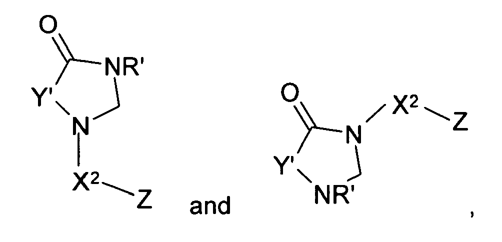 in which R' stands for H, A, (CH₂)ₙHet, (CH₂)ₙAr, cycloalkyl having 3 to 7 C atoms or SO₂A, and Y' stands for the spiro- linking C atom of the spiro-linked heterocycle,
R¹¹ stands for H, A, (CH₂)ₙHet, (CH₂)ₙAr or cycloalkyl having 3 to 7 C atoms,
X² stands for a bond or is selected, independently, from the meanings indicated for X¹, and preferably stands for a bond or O, S, N-R⁷, CH₂ or CH₂CH₂,
p, q, r, independently of one another, stand for 0, 1, 2 or 3
and
Hal stands for F, Cl, Br or I, and
R¹², R¹³ are selected, independently of one another, from the mean- ings of R⁴ other than H,
Z denotes H or stands for a saturated, mono- or polyethyleni- cally unsaturated carbocycle having 5 to 10 C atoms, an aromatic carbocycle selected from phenyl and naphthyl, or a saturated, mono- or polyethylenically unsaturated or aromatic heterocycle selected from furanyl, thiophenyl, pyrrolyl, imida- zolyl, pyrazolyl, triazolyl, tetrazolyl, thiazolyl, oxazolyl, isoxa- zolyl, oxadiazolyl, oxopyridyl, thiadiazolyl, isothiazolyl, pyridyl, pyridazyl, pyrazinyl, pyrimidyl, quinolinyl, isoquinolinyl, benzo- furanyl, benzothiophenyl, benzo-1,4-dioxinyl, 2,3-dihydro- benzo-1,4-dioxinyl, benzothiadiazolyl, chromenyl, 2-oxochro- menyl, indolyl and indazolyl, where the carbocycle or hetero- cycle may be mono- or polysubstituted, where the substitu- ents are selected, independently of one another, from A, cycloalkyl having 3 to 7 C atoms, Hal, CH₂Hal, CH(Hal)₂, C(Hal)₃, NO₂, (CH₂)ₙCN, (CH₂)ₙN(R⁶)₂, (CH₂)ₙN(R⁶)Ar, (CH₂)ₙN(R⁶)Het, (CH₂)ₙN(Ar)₂, (CH₂)ₙN(Het)₂, (CH₂)ₙCOOR⁶, (CH₂)ₙCOOAr, (CH₂)ₙCOOHet, (CH₂)ₙCON(R⁶)₂, (CH₂)ₙCON(R⁶)Ar, (CH₂)ₙCON(R⁶)Het, (CH₂)ₙCON(Ar)₂, (CH₂)ₙCON(Het)₂, (CH₂)nNR⁶COR⁶, (CH₂)ₙNR⁶CON(R⁶)₂, (CH₂)ₙNR⁶SO₂A, (CH₂)nSO₂N(R⁶)₂, (CH₂)ₙSO₂NR⁶(CH₂)ₘAr, (CH₂)ₙSO₂NR⁶(CH₂)ₘHet, (CH₂)ₙS(O)_{w}R⁶, (CH₂)ₙS(O)_{w}Ar, (CH₂)ₙS(O)_{w}Het, (CH₂)ₙOOCR⁶, (CH₂)ₙHet, (CH₂)ₙAr, (CH₂)ₙCOR⁶, (CH₂)ₙCO(CH₂)mAr, (CH₂)ₙCO(CH₂)ₘHet, (CH₂)ₙCOO(CH₂)ₘAr, (CH₂)ₙCOO(CH₂)ₘHet, (CH₂)ₙOR⁶ (CH₂)ₙO(CH₂)ₘAr, (CH₂)ₙO(CH₂)ₘHet, (CH₂)ₙSR⁶, (CH₂)ₙS(CH2)ₘAr, (CH₂)ₙS(CH₂)ₘHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)ₘHet, (CH₂)ₙCON(R⁶)(CH₂₎mAr, (CH₂)ₙN(R⁶)CO(CH₂)ₘAr, (CH₂)ₙCON(R⁶)(CH₂)ₘHet, (CH₂)ₙN(R⁶)CO(CH₂)ₘHet, CH=N-OA, CH₂CH=N-OA, (CH₂)ₙNHOA, (CH₂)ₙCH=N-Het, (CH₂)ₙOCOR⁶ (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)ₘAr, (CH₂)ₙNR⁶COO(CH₂)ₘHet, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHet, (CH₂)ₙN(R⁶)CH₂Het, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, CH=CHCH₂NR⁶Het, CH=CHCH₂N(R⁶)₂, CH=CHCH₂OR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶, (CH₂)ₙCHR⁶CH₂OR⁶, (CH₂)ₙOCN and (CH₂)ₙNCO,
and the pharmaceutically usable salts, solvates and stereoisomers and mixtures thereof.

2. Compounds of the formula Ia according to Claim 1 in which
R¹ denotes H or SO₂A,
D-E stands for R²C=CR⁴,
R² denotes H, alkyl having 1 to 3 C atoms, alkoxyalkyl having 2 to 5 C atoms, Hal, CH₂Hal, CH(Hal)₂, C(Hal)₃, NO₂, (CH₂)ₙCN, (CH₂)ₙCOOR⁶, (CH₂)ₙCON(R⁶)₂, (CH₂)ₙSO₂N(R⁶)₂ or (CH₂)ₙS(O)_{w}R⁶,
R⁴ denotes H, alkyl having 1 to 3 C atoms, alkoxyalkyl having 2 to 5 C atoms, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ or (CH₂)ₙS(O)_{w}R⁶ and
R⁶ denotes H or A,
n stands for 0 or 1,
X¹ denotes (CHR⁷)_{g}, in which g stands for 2, 3 or 4, or denotes Q-(CHR⁸)ₖ, in which k stands for 1, 2 or 3 and Q is selected from O, S, N-R⁶, CONR⁶, C=O, C=S, S=O, SO₂, SO₂NR⁶ and NR⁶SO₂,
R⁷, R⁸ are selected, independently of one another, from H and A,
Y stands for CH, COR¹¹ or N,
R¹¹ denotes H or A, and
X² denotes CH₂, CH₂CH₂, HCOH, O, S, N-R⁶, CONR⁶, C=O or a bond,and
R¹² is selected, independently, from A, Hal, CN, NO₂, OR⁶, N(R⁶)₂, COOR⁶, CON(R⁶)₂, COR⁶, SO₂N(R⁶)₂ and S(O)_{w}A.

3. Compounds according to Claim 1 or 2 in which the group X²-Z is selected from the groups 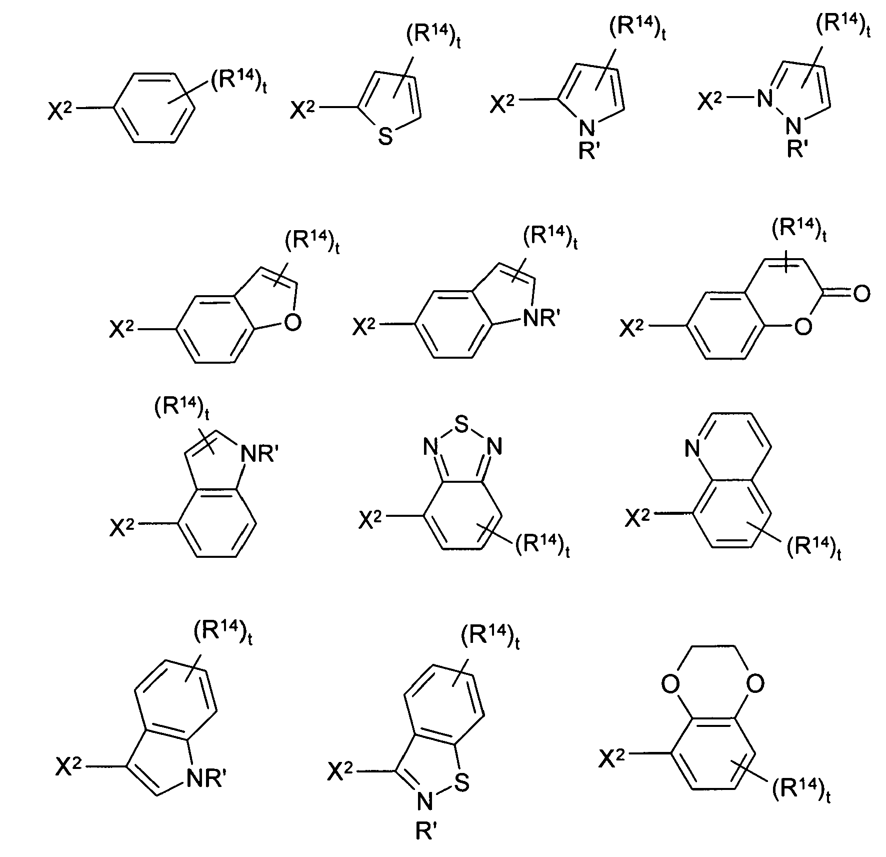 in which
R¹⁴ is selected independently from Hal, A, (CH₂)ₙHet, (CH₂)ₙAr, (CH₂)ₙCOO(CH₂)ₘAr, (CH₂)ₙCOO(CH₂)ₘHet, (CH₂)ₙOR⁶, (CH₂)ₙO(CH₂)ₘAr, (CH₂)ₙO(CH₂)mHet, (CH₂)ₙN(R⁶)(CH₂)ₘAr, (CH₂)ₙN(R⁶)(CH₂)ₘHet, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHet, (CH₂)ₙN(R⁶)SO₂(CH₂)mHet, (CH₂)ₙN(R⁶)₂, (CH₂)ₙNHOA, (CH₂)ₙ(R⁶)Het, (CH₂)ₙOCOR⁶ (CH₂)ₙOC(O)N(R⁶)₂, (CH₂)ₙOC(O)NR⁶(CH₂)ₘAr, (CH₂)ₙOC(O)NR⁶(CH₂)ₘHet, (CH₂)ₙNR⁶COOR⁶, (CH₂)ₙNR⁶COO(CH₂)mAr, (CH₂₎ₙNR⁶COO(CH2)ₘHet,
t stands for 0, 1, 2 or 3, and
R' stands for H, A, (CH₂)ₙHet, (CH₂)ₙAr, cycloalkyl having 3 to 7 C atoms or SO₂A.

4. Compounds according to Claim 1 or 2 in which the group X²-Z is selected from the groups 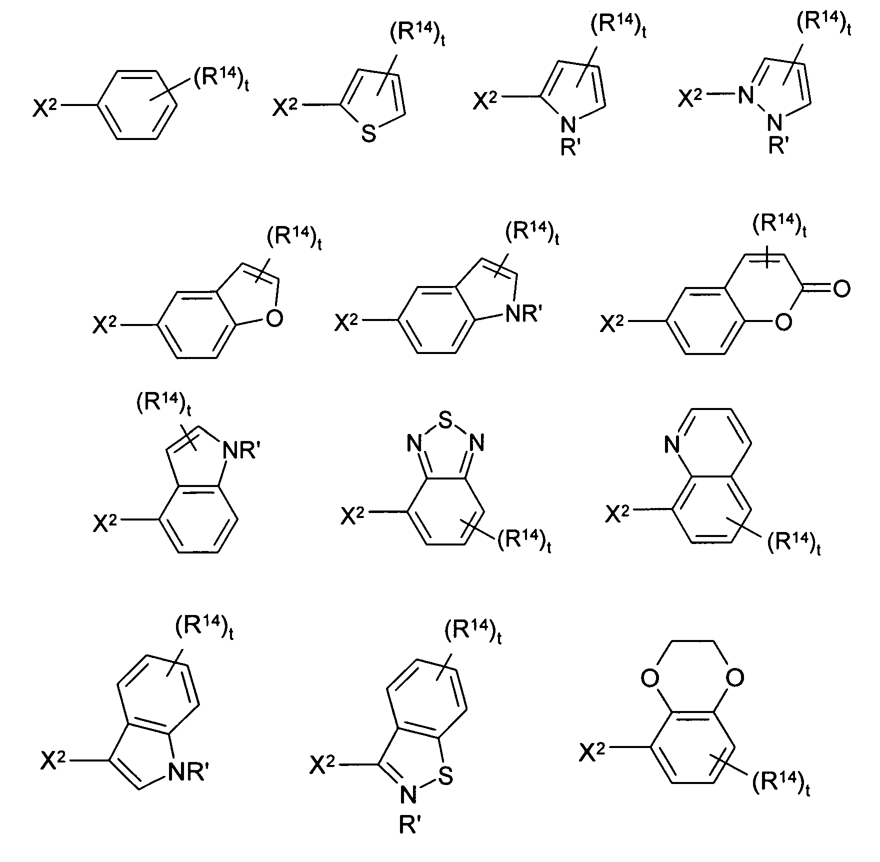 in which
R¹⁴ stands, independently of one another, for Hal, NO₂, OR⁶, N(R⁶)₂, CN, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ and/or C(NH)NOH,
w stands for 0, 1, 2 or 3,
t stands for 0, 1, 2 or 3, and
R' stands for H, A, (CH₂)ₙHet, (CH₂)ₙAr, cycloalkyl having 3 to 7 C atoms or SO₂A.

5. Compounds according to Claim 1, 2, 3 or 4, selected from
a) 6-{3-[4-(4-fluorobenzyl)-1-piperidyl]propyl}-1H-indole-3-carbonitrile;
b) 6-{3-[4-(2,4-difluorobenzyl)-1-piperidyl]propyl}-1H-indole-3-carbonitrile;
c) 6-(3-[4-(4-fluorophenoxy)-1-piperidyl]propy}-1H-indole-3-carbonitrile;
d) 4-{3-[4-(4-fluorobenzyl)-1-piperidyl]propyl}-1H-indole-3-carbonitrile;
e) 4-{3-[4-(2,4-difluorobenzyl)-1-piperidyl]propyl}-1H-indole-3-carbonitrile;
f) 4-{3-[4-(4-fluorophenoxy)-1-piperidyl]propyl}-1H-indole-3-carbonitrile;
g) 5-{3-[4-(4-fluorophenoxy)-1-piperidyl]propyl}-1H-indole-3-carbonitrile;
h) 5-{3-[4-(4-fluorobenzyl)-1-piperidyl]propyl}-1H-indole-3-carbonitrile;
i) 5-{3-[4-(2,4-difluorobenzyl)-1-piperidyl]propyl}-1H-indole-3-carbonitrile;
j) 5-{3-[4-(4-cyanophenyl)piperazin-1-yl]propyl)-1H-indole-3-carbonitrile;
k) 5-{4-[3-(3-cyano-1H-indol-6-yl)propyl]piperazin-1-yl}benzofuran-2-carboxamide;
l) 5-{3-[4-(2-oxo-2H-chromen-6-yl)piperazin-1-yl]propyl}-1H-indole-3-carbonitrile;
m) 5-{4-[3-(3-cyano-1H-indol-4-yl)propyl]piperazin-1-yl}benzofuran-2-carboxamide;
n) 5-{4-[3-[3-cyano-1H-indol-5-yl)propyl]piperazin-1-yl}benzofuran-2-carboxamide;
o) 5-{3-[4-(1H-indol-4-yl)piperazin-1-yl]propyl}-1-methanesulfonyl-1H-indole-3-carbonitrile;
p) 5-[3-(4-oxo-1-phenyl-1,3,8-triazaspiro[4.5]dec-8-yl)propyl]-1H-indole-3-carbonitrile;
q) 5-[3-(4-benzo-1,2,5-thiadiazol-4-ylpiperazin-1-yl)propyl]-1H-indole-3-carbonitrile;
r) 3-{1-[3-(3-cyano-1H-indol-5-yl)propyl]piperidin-4-yl}-1H-indole-5-carboxamide;
s) 5-[3-(4-quinolin-8-ylpiperazin-1-yl)propyl]-1H-indole-3-carbonitrile;
t) 5-{3-[4-(2,3-dihydrobenzo-1,4-dioxin-5-yl)piperazin-1-yl]propyl}-1H-indole-3-carbonitrile;
u) 1-methanesulfonyl-5-[3-(4-oxo-1-phenyl-1,3,8-triazaspiro[4.5]-dec-8-yl)propyl]-1H-indole-3-carbonitrile;
v) 5-{3-[4-(1H-indol-4-yl)piperazin-1-yl]propyl}-1H-indole-3-carbonitrile;
w) 5-{3-[4-(1H-indol-3-yl)piperidin-1-yl]propyl}-1H-indole-3-carbonitrile;
x) 5-{3-[4-(5-fluoro-1H-indol-3-yl)piperidin-1-yl]propyl}-1H-indole-3-carbonitrile;
y) 3-{1-[3-(3-cyano-1H-indol-5-yl)propyl]piperidin-4-yl}-1H-indole-5-carbonitrile;
z) 5-{3-[4-(6-fluoro-1H-indol-3-yl)piperidin-1-yl]propyl}-1H-indole-3-carbonitrile;
aa) 5-{3-[4-(4-fluoro-1H-indol-3-yl)piperidin-1-yl]propyl}-1H-indole-3-carbonitrile;
bb) 5-[3-(4-benzo[d]isothiazol-3-ylpiperazin-1-yl)propyl]-1H-indole-3-carbonitrile;
cc) 4-{1-[3-(3-cyano-1H-indol-6-yl)propyl]piperidin-4-yloxy}benzamide;
dd) 6-{3-[4-(2-cyano-3-methoxyphenyl)piperazin-1-yl]propyl}-1H-indole-3-carbonitrile;
ee) 6-{3-[4-(4-cyano-3-methoxyphenyl)piperazin-1-yl]propyl}-1H-indole-3-carbonitrile;
ff) 6-{3-[4-(4-cyano-2-methoxyphenyl)piperazin-1-yl]propyl}-1H-indole-3-carbonitrile;
gg) 4-[3-(4-pyrazol-1-ylmethyl-1-piperidyl)propyl]-1H-indole-3-carbonitrile;
hh) N-(6-{4-[3-(3-cyano-1H-indol-5-yl)propyl]piperazin-1-yl}-2-oxo-2H-chromen-3-yl)acetamide;
ii) 5-{3-[(pyridin-3-ylmethyl)amino]propyl}-1H-indole-3-carbonitrile;
jj) 5-{3-[4-(2,3-dihydrobenzo-1,4-dioxin-6-yl)piperazin-1-yl]propyl}-1H-indole-3-carbonitrile;
kk) 5-[3-(4-pyrimidin-2-yl)piperazin-1-yl)propyl]-1H-indole-3-carbonitrile;
ll) 5-{3-[4-(3-methoxyphenyl)-3-methylpiperazin-1-yl]propyl}-1H-indole-3-carbonitrile;
mm) 5-{3-[4-(1-methyl-1H-imidazo[4,5-c]pyridin-4-yl)piperazin-1-yl]-propyl}-1H-indole-3-carbonitrile;
nn) N-(4-{1-[3-(3-cyano-1H-indol-5-yl)propyl]piperidin-4-ylmethyl}-phenyl)acetamide;
oo) 5-{3-[4-(4-pyridin-3-ylthiazol-2-yl)piperazin-1-yl]propyl}-1H-indole-3-carbonitrile;
pp) ethyl 2-{4-[3-(3-cyano-1H-indol-5-yl)propyl]piperazin-1-yl}thiazole-4-carboxylate;
qq) 5-{3-[3-(2-oxopyrrolidin-1-yl)propylamino]propyl)-1H-indole-3-carbonitrile;
rr) 5-{3-[4-(3-amino-2-oxo-2H-chromen-6-yl)piperazin-1-yl]propyl}-1H-indole-3-carbonitrile;
ss) methyl (6-{4-[3-(3-cyano-1H-indol-5-yl)propyl]piperazin-1-yl}-2-oxo-2H-chromen-3-yl)carbamate;
tt) 2-{4-[3-(3-cyano-1H-indol-5-yl)propyl]piperazin-1-yl}thiazole-4-carboxamide;
uu) 4-[3-(3-cyano-1H-indol-5-yl)propyl]piperazine-1-thiocarboxamide;
and salts and solvates thereof.

6. Process for the preparation of compounds of the formula Ia according to Claim 1 and salts thereof, **characterised in that**
a) a compound of the formula II 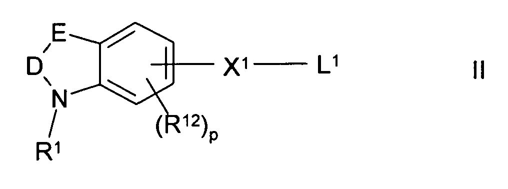 in which
L¹ denotes Cl, Br, I, OH, a reactively esterified OH group, selected from alkylsulfonyloxy groups having 1-6 C atoms and arylsul- fonyloxy groups having 6-10 C atoms, or a diazonium group, and R¹, D, E, R¹², p and X¹ have the meanings indicated in Claim 1,
b) is reacted with a compound of the formula III 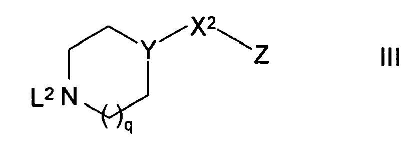 in which
L²denotes H or a metal ion, and q, Y, X² and Z have the meanings indicated in Claim 1,
and optionally
c) the resultant compound of the formula Ia is converted into one of its salts by treatment with an acid.

7. Pharmaceutical composition, **characterised by** a content of at least one compound of the formula Ia according to Claim 1 and/or one of its physiologically acceptable salts and/or one of its solvates.

8. Compounds of the formula Ia according to Claim 1 and physiologically acceptable salts and solvates thereof as medicaments.

9. Use of the compounds of the formula Ia according to Claim 1 for the preparation of a medicament for the prophylaxis and/or therapy of diseases in which 5HT plays a role, **characterised in that** the diseases are selected from the group comprising depression, strokes, cerebral ischaemia, extrapyramidal motor side effects of neuroleptics and of Parkinson's disease, Alzheimer's disease, amyotrophic lateral sclerosis, brain and spinal cord trauma, obsessive-compulsive disorder, sleeping disorders, tardive dyskinesia, learning disorders, age-related memory disorders, eating disorders and sexual dysfunctions.

10. Use of compounds of the formula Ia according to Claim 1 and/or physiologically acceptable salts or solvates thereof for the preparation of a medicament for the prophylaxis and/or treatment of schizophrenia, depression, dementia, Parkinson's disease, Alzheimer's disease, Lewy bodies dementia, Huntington's disease, Tourette's syndrome, anxiety, learning and memory impairments, neurodegenerative diseases, cognitive impairments, as well as nicotine dependence and pain.

11. Compounds of the formula Ia according to Claim 1 and/or physiologically acceptable salts thereof for use in combating cerebrovascular diseases, epilepsy, schizophrenia, Alzheimer's disease, Parkinson's disease or Huntington's disease, cerebral ischaemia, infarction or psychoses.

12. Process for the preparation of pharmaceutical compositions, **characterised in that** a compound of the formula Ia according to Claim 1 and/or one of its physiologically acceptable salts and/or one of its solvates is converted into a suitable dosage form together with at least one solid, liquid or semi-liquid excipient or adjuvant.

13. Use of compounds of the formula II 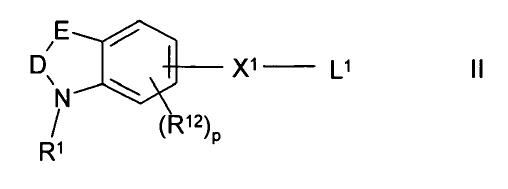 in which
L¹ denotes Cl, Br, I, OH, a reactively esterified OH group, selected from alkylsulfonyloxy groups having 1-6 C atoms and arylsulfon- yloxy groups having 6-10 C atoms, or a diazonium group, and R¹ D, E, R¹², p and X¹ have the meanings indicated in Claim 1,
for the preparation of compounds of the formula Ia according to Claim 1.

14. Use of compounds of the formula III 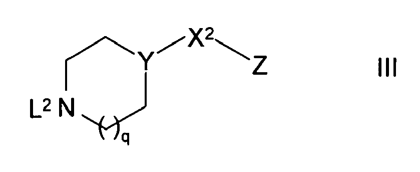 in which
L² denotes H or a metal ion, and q, Y, X² and Z have the meanings indicated in Claim 1,
for the preparation of compounds of the formula Ia according to Claim 1.

## Revendications

1. Composés de formule la 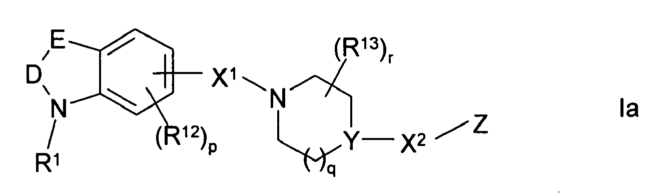 dans laquelle
R¹ représente H, A ou SO₂A,
A représente alkyle à chaîne linéaire ayant 1 à 4 atomes de C ou alkyle à chaîne ramifiée ayant 3 à 6 atomes de C, et
D-E représente R²C=CR⁴, où R² est choisi parmi H, A et cycloalkyle ayant 3 à 7 atomes de C, et R⁴ représente Hal, CH₂Hal, CH(Hal)₂, C(Hal)₃, NO₂, (CH₂)ₙCN, (CH₂)ₙCOOR⁶, (CH₂)ₙCON(R⁶)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙS(O)_{w}A, (CH₂)ₙOOCR⁶, (CH₂)ₙCOR⁶, (CH₂)ₙCO(CH₂)ₘAr, (CH₂)ₙCO(CH₂)ₘHét, (CH₂)ₙCOO(CH₂)ₘAr, (CH₂)ₙCOO(CH₂)ₘHét, (CH₂)ₙOR⁶, (CH₂)ₙO(CH₂)ₘAr, (CH₂)ₙO(CH₂)ₘHét, (CH₂)ₙSR⁶, (CH₂)ₙS(CH₂)ₘAr, (CH₂)ₙS(CH₂)ₘHét, (CH₂)ₙN(R⁶)(CH₂)ₘAr, (CH₂)ₙN(R⁶)(CH₂)ₘHét, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHét, (CH₂)ₙN(R⁶)SO₂(CH₂)ₘHét, (CH₂)ₙCON(R⁶)(CH₂)ₘAr, (CH₂₎ₙN(R⁶)CO(CH₂)ₘAr, (CH₂)ₙCON(R⁶)(CH₂)ₘHét, (CH₂)ₙN(R⁶)CO(CH₂)ₘHét, (CH₂)ₙN(R⁶)₂, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₘHét, (CH₂)ₙNR⁶COOR⁶, (CH₂)ₙNR⁶COO(CH₂)ₘAr, (CH₂)ₙNR⁶COO(CH₂)ₘHét, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHét, (CH₂)ₙN(R⁶)CH₂Hét, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶ ou (CH₂)ₙCHR⁶CH₂OR⁶, où
Hét représente un radical hétérocyclique mono- ou bicyclique saturé, insaturé ou aromatique qui est non substitué ou monosubstitué ou polysubstitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)₂, S(O)_{w}A et/ou OOCR⁶,
Ar représente un radical hydrocarboné aromatique ayant 6 à 14 atomes de carbone qui est non substitué ou monosubstitué ou polysubstitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂N(R⁶)2, S(O)_{w}A et/ou OOCR⁶,
w représente 0, 1, 2 ou 3, et
m représente 0, 1, 2, 3, 4 ou 5, et
n représente 0, 1, 2 ou 3,
X¹ représente (CHR⁷)g ou (CHR⁷)ₕ-Q-(CHR⁸)k, où
Q est choisi parmi O, S, N-R⁶, (O-CHR⁷)g, (CHR⁷-O)_{g}, CR⁹=CR¹⁰, (O-CHR⁹CHR¹⁰)_{g}, (CHR⁹CHR¹⁰-O)_{g}, C=O, C=S, C=NR⁶, CH(OR⁶), C(OR⁶)(OR⁶), C(=O)O, OC(=O), OC(=O)O, C(=O)N(R⁶), N(R⁶)C(=O), OC(=O)N(R⁶), N(R⁶)C(=O)O, CH=N-O, CH=N-NR⁶, OC(O)NR⁶, NR⁶C(O)O, S=O, SO₂, SO₂NR⁶ et NR⁶SO₂,
g représente 1, 2, 3, 4, 5 ou 6,
h représente 0, 1, 2, 3, 4, 5 ou 6,
k représente 1, 2 ou 3, et
R⁶ est choisi indépendamment parmi H, A ou cycloalkyle ayant 3 à 7 atomes de C,
R⁷, R⁸, R⁹ et R¹⁰ sont choisis indépendamment parmi les significa- tions indiquées pour R² et R⁴,
Y représente CH, N, COR¹¹, CSR¹¹, un carbocycle à liaison spiro non substitué ou substitué ayant 5 à 7 atomes de car- bone ou un hétérocycle à liaison spiro non substitué ou subs- titué choisi parmi les structures de formules 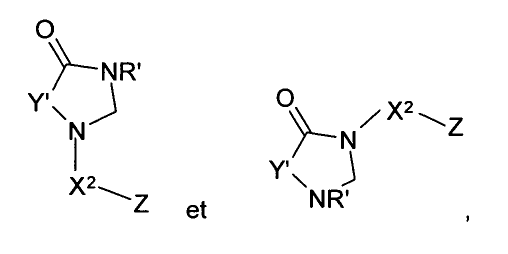 dans lesquelles R' représente H, A, (CH₂)ₙHét, (CH₂)ₙAr, cycloalkyle ayant 3 à 7 atomes de C ou SO₂A, et Y' repré- sente l'atome de C de liaison spiro de l'hétérocycle à liaison spiro,
R¹¹ représente H, A, (CH₂)ₙHét, (CH₂)ₙAr ou cycloalkyle ayant 3 à 7 atomes de C,
X² représente une liaison ou est choisi indépendamment parmi les significations indiquées pour X¹, et représente préféra- blement une liaison ou O, S, N-R₇, CH₂ ou CH₂CH₂,
p, q, r, représentent, indépendamment les uns des autres, 0, 1, 2 ou 3
et
Hal représente F, Cl, Br ou 1, et
R¹², R¹³ sont choisis, indépendamment l'un de l'autre, parmi les significations de R⁴ autres que H,
Z désigne H ou représente un carbocycle saturé, mono- ou polyéthyléniquement insaturé ayant 5 à 10 atomes de C, un carbocycle aromatique choisi parmi phényle et naphtyle, ou un hétérocycle saturé, mono- ou polyéthyléniquement insatu- ré ou aromatique choisi parmi furanyle, thiophényle, pyrrolyle, imidazolyle, pyrazolyle, triazolyle, tétrazolyle, thiazolyle, oxa- zolyle, isoxazolyle, oxadiazolyle, oxopyridyle, thiadiazolyle, isothiazolyle, pyridyle, pyridazyle, pyrazinyle, pyrimidyle, quinoléinyle, isoquinoléinyle, benzofuranyle, benzothio- phényle, benzo-1,4-dioxinyle, 2,3-dihydrobenzo-1,4-dioxinyle, benzothiadiazolyle, chroményle, 2-oxochroményle, indolyle et indazolyle, où le carbocycle ou l'hétérocycle peuvent être mono- ou polysubstitués, où les substituants sont choisis, indépendamment les uns des autres, parmi A, cycloalkyle ayant 3 à 7 atomes de C, Hal, CH₂Hal, CH(Hal)₂, C(Hal)₃, NO₂, (CH₂)ₙCN, (CH₂)ₙN(R⁶)_{2,} (CH₂)ₙN(R⁶)Ar, (CH₂)ₙN(R⁶)Hét, (CH₂)ₙN(Ar)₂, (CH₂)ₙN(Hét)₂, (CH₂)ₙCOOR⁶, (CH₂)ₙCOOAr, (CH₂)ₙCOOHét, (CH₂)ₙCON(R⁶)₂, (CH₂)ₙCON(R⁶)Ar, (CH₂)ₙCON(R⁶)Hét, (CH₂)ₙCON(Ar)₂, (CH₂)ₙCON(Hét)₂, (CH₂)ₙNR⁶COR⁶, (CH₂)ₙNR⁶CON(R⁶)₂, (CH₂)ₙNR⁶SO₂A, (CH₂)ₙSO₂N(R⁶)₂, (CH₂)ₙSO₂NR⁶(CH₂)ₘAr, (CH₂)ₙSO₂NR⁶(CH₂)ₘHét, (CH₂)ₙS(O)_{w}R⁶, (CH₂)ₙS(O)_{w}Ar, (CH₂)ₙS(O)_{w}Hét, (CH₂)ₙOOCR⁶, (CH₂)₂Hét, (CH₂)ₙAr, (CH₂)ₙCOR⁶, (CH₂)ₙCO(CH₂)ₘAr, (CH₂)ₙCO(CH₂)ₘHét, (CH₂)ₙCOO(CH₂)ₘAr, (CH₂)ₙCOO(CH₂)ₘHét, (CH₂)ₙOR⁶, (CH₂)ₙO(CH₂)ₘAr, (CH₂)ₙO(CH₂)ₘHét, (CH₂)ₙSR⁶, (CH₂)ₙS(CH₂)ₘAr, (CH₂)ₙS(CH₂)ₘHét, (CH₂)ₙN(R⁶)(CH₂)ₘAr, (CH₂)ₙN(R⁶)(CH₂)ₘHét, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHét, (CH₂)ₙN(R⁶)SO₂(CH₂)ₘHét, (CH₂)ₙCON(R⁶)(CH₂)ₘAr, (CH₂)ₙN(R⁶)CO(CH₂)ₘAr, (CH₂)ₙCON(R⁶)(CH₂)ₘHét, (CH₂)ₙN(R⁶)CO(CH₂)ₘHét, CH=N-OA, CH₂CH=N-OA, (CH₂)ₙNHOA, (CH₂)ₙCH=N-Hét, (CH₂)ₙOCOR⁶, (CH₂)ₙOC(O)N(R⁶)₂, (CH₂)ₙOC(O)NR⁶(CH₂)ₘAr, (CH₂)ₙOC(O)NR⁶(CH₂)ₘHét, (CH₂)ₙNR⁶COOR⁶, (CH₂)ₙNR⁶COO(CH₂)ₘAr, (CH₂)ₙNR⁶COO(CH₂)ₘHét, (CH₂)ₙN(R⁶)CH₂CH₂OR⁶, (CH₂)ₙN(R⁶)CH₂CH₂OCF₃, (CH₂)ₙN(R⁶)C(R⁶)HCOOR⁶, (CH₂)ₙN(R⁶)CH₂COHét, (CH₂)ₙN(R⁶)CH₂Hét, (CH₂)ₙN(R⁶)CH₂CH₂N(R⁶)CH₂COOR⁶, (CH₂)ₙN(R⁶)CH₂CH₂N(R⁶)₂, CH=CHCOOR⁶, CH=CHCH₂NR⁶Hét, CH=CHCH₂N(R⁶)_{2,} CH=CHCH₂OR⁶, (CH₂)ₙN(COOR⁶)COOR⁶, (CH₂)ₙN(CONH₂)COOR⁶, (CH₂)ₙN(CONH₂)CONH₂, (CH₂)ₙN(CH₂COOR⁶)COOR⁶, (CH₂)ₙN(CH₂CONH₂)COOR⁶, (CH₂)ₙN(CH₂CONH₂)CONH₂, (CH₂)ₙCHR⁶COR⁶, (CH₂)ₙCHR⁶COOR⁶, (CH₂)ₙCHR⁶CH₂OR⁶, (CH₂)ₙOCN et (CH₂)ₙNCO,
et les sels, solvats et stéréoisomères pharmaceutiquement utilisables et des mélanges de ceux-ci.

2. Composés de formule la selon la revendication 1, dans lesquels
R¹ désigne H ou SO₂A,
D-E représente R²C=CR⁴,
R² désigne H, alkyle ayant 1 à 3 atomes de C, alcoxyalkyle ayant 2 à 5 atomes de C, Hal, CH₂Hal, CH(Hal)₂, C(Hal)₃, NO₂, (CH₂)ₙCN, (CH₂)ₙCOOR⁶, (CH₂)ₙCON(R⁶)₂, (CH₂)ₙSO₂N(R⁶)₂ ou (CH₂)ₙS(O)_{w}R⁶,
R⁴ désigne H, alkyle ayant 1 à 3 atomes de C, alcoxyalkyle ayant 2 à 5 atomes de C, Hal, CH₂Hal, CH(Hal)₂, C(Hal)₃, NO₂, (CH₂)ₙCN, (CH₂)ₙCOOR⁶, (CH₂)ₙCON(R⁶)₂ (CH₂)ₙSO₂N(R⁶)₂ ou (CH₂)ₙS(O)_{w}R⁶ et
R⁶ désigne H ou A,
n représente 0 ou 1,
X¹ désigne (CHR⁷)_{g}, où g représente 2, 3 ou 4, ou désigne Q-(CHR⁸)ₖ, où k représente 1, 2 ou 3 et Q est choisi parmi O, S, N-R⁶, CONR⁶, C=O, C=S, S=O, SO₂, SO₂NR⁶ et NR⁶SO₂,
R⁷, R⁸ sont choisis, indépendamment l'un de l'autre, parmi H et A,
Y représente CH, COR¹¹ ou N,
R¹¹ désigne H ou A, et
X² désigne CH₂, CH₂CH₂, HCOH, O, S, N-R⁶, CONR⁶, C=O ou une liaison, et
R¹² est choisi indépendamment parmi A, Hal, CN, NO₂, OR⁶, N(R⁶)₂, COOR⁶, CON(R⁶)₂, COR⁶, SO₂N(R⁶)₂ et S(O)_{w}A.

3. Composés selon la revendication 1 ou 2, dans lesquels le groupement X²-Z est choisi parmi les groupements 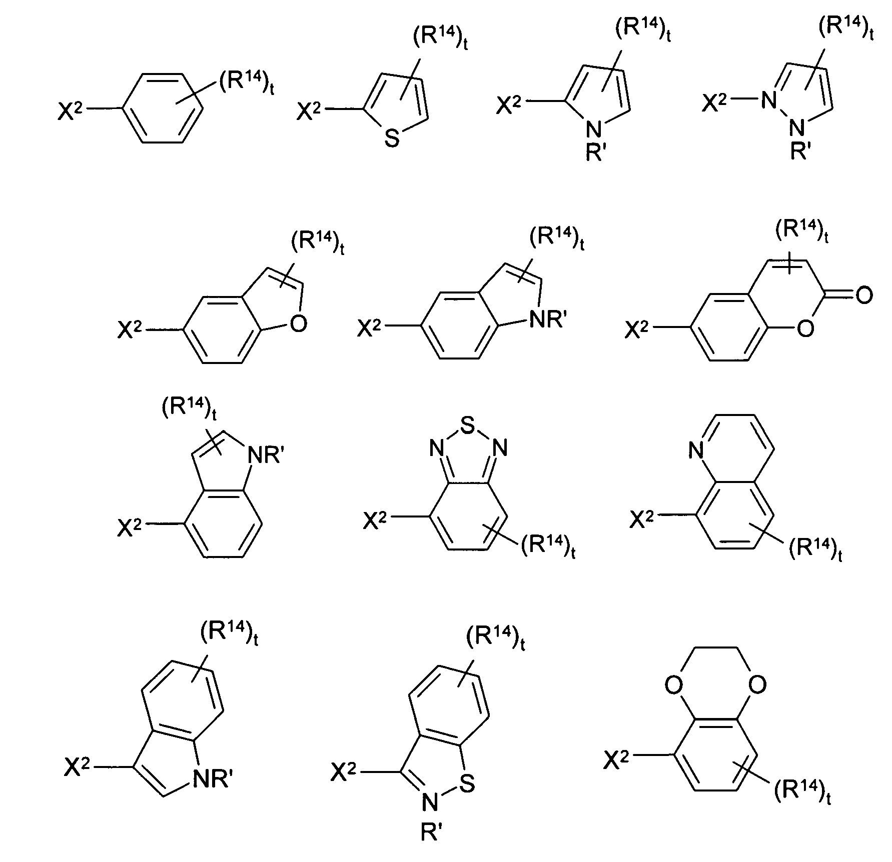 dans lesquels
R¹⁴ est choisi indépendamment parmi Hal, A, (CH₂)ₙHét, (CH₂)ₙAr, (CH₂)ₙCOO(CH₂)ₘAr, (CH₂)ₙCOO(CH₂)ₘHét, (CH₂)ₙOR⁶, (CH₂)ₙO(CH₂)ₘAr, (CH₂)ₙO(CH₂)ₘHét, (CH₂)ₙN(R⁶)(CH₂)ₘAr, (CH₂)ₙN(R⁶)(CH₂)ₘHét, (CH₂)ₙSO₂N(R⁶)(CH₂)ₘAr, (CH₂)ₙN(R⁶)SO₂(CH₂)ₘAr, (CH₂)ₙSO₂N(R⁶)(CH₂)ₘHét, (CH₂)ₙN(R⁶)SO₂(CH₂)ₘHét, (CH₂)nN(R⁶)₂, (CH₂)ₙNHOA, (CH₂)ₙ(R⁶)Hét, (CH₂)ₙOCOR⁶, (CH₂)ₙOC(O)N(R⁶)_{2,} (CH₂)ₙOC(O)NR⁶(CH₂)ₘAr, (CH₂)ₙOC(O)NR⁶(CH₂)ₘHét, (CH₂)ₙNR⁶COOR⁶, (CH₂)ₙNR⁶COO(CH₂)ₘAr, (CH₂)ₙNR⁶COO)(CH₂)ₘHét,
t représente 0, 1, 2 ou 3, et
R' représente H, A, (CH₂)ₙHét, (CH₂)ₙAr, cycloalkyle ayant 3 à 7 atomes de C ou SO₂A.

4. Composés selon la revendication 1 ou 2, dans lesquels le groupement X²-Z est choisi parmi les groupements 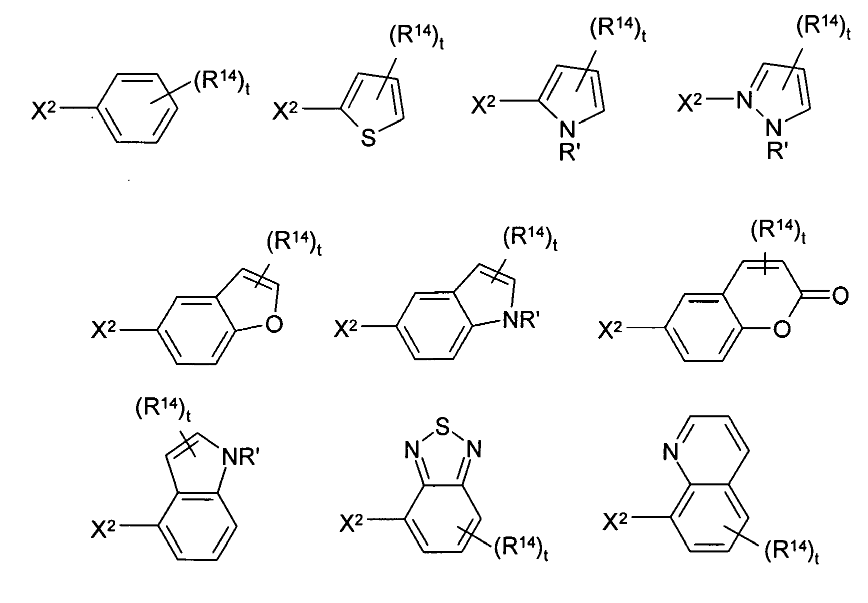 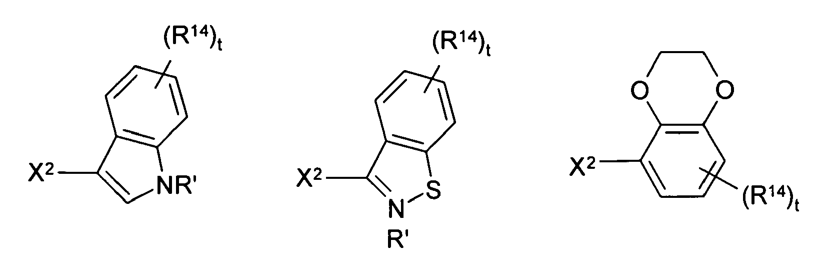 dans lesquels
R¹⁴ représente, indépendamment l'un de l'autre, Hal, NO₂, OR⁶, N(R⁶)_{2,} CN, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ et/ou C(NH)NOH,
w représente 0, 1, 2 ou 3,
t représente 0, 1, 2 ou 3, et
R' représente H, A, (CH₂)ₙHét, (CH₂)ₙAr, cycloalkyle ayant 3 à 7 atomes de C ou SO₂A.

5. Composés selon la revendication 1, 2, 3 ou 4, choisis parmi
a) le 6-{3-[4-(4-fluorobenzyl)-1-pipéridyl]propyl}-1H-indole-3-carbonitrile ;
b) le 6-{3-[4-(2,4-difluorobenzyl)-1-pipéridyl]propyl}-1H-indole-3-carbonitrile ;
c) le 6-{3-[4-(4-fluorophénoxy)-1-pipéridyl]propyl}-1H-indole-3-carbonitrile ;
d) le 4-{3-[4-(4-fluorobenzyl)-1-pipéridyl]propyl}-1H-indole-3-carbonitrile ;
e) le 4-{3-[4-(2,4-difluorobenzyl)-1-pipéridyl]propyl}-1H-indole-3-carbonitrile ;
f) le 4-{3-[4-(4-fluorophénoxy)-1-pipéridyl]propyl}-1H-indole-3-carbonitrile ;
g) le 5-{3-[4-(4-fluorophénoxy)-1-pipéridyl]propyl}-1H-indole-3-carbonitrile ;
h) le 5-{3-[4-(4-fluorobenzyl)-1-pipéridyl]propyl}-1H-indole-3-carbonitrile ;
i) le 5-{3-[4-(2,4-difluorobenzyl)-1-pipéridyl]propyl}-1H-indole-3-carbonitrile ;
j) le 5-{3-[4-(4-cyanophényl)pipérazin-1-yl]propyl}-1H-indole-3-carbonitrile ;
k) le 5-{4-[3-(3-cyano-1H-indol-6-yl)propyl]pipérazin-1-yl}benzo-furan-2-carboxamide ;
l) le 5-{3-[4-(2-oxo-2H-chromén-6-yl)pipérazin-1-yl]propyl}-1H-indole-3-carbonitrile ;
m) le 5-{4-[3-(3-cyano-1H-indol-4-yl)propyl]pipérazin-1-yl}benzo-furan-2-carboxamide ;
n) le 5-{4-[3-(3-cyano-1H-indol-5-yl)propyl]pipérazin-1-yl}benzo-furan-2-carboxamide ;
o) le 5-{3-[4-(1H-indol-4-yl)pipérazin-1-yl]propyl}-1-méthanesulfonyl-1H-indole-3-carbonitrile ;
p) le 5-[3-(4-oxo-1-phényl-1,3,8-triazaspiro[4.5]déc-8-yl)propyl]-1H-indole-3-carbonitrile ;
q) le 5-[3-(4-benzo-1,2,5-thiadiazol-4-ylpipérazin-1-yl)propyl]-1H-indole-3-carbonitrile ;
r) le 3-{1-[3-(3-cyano-1H-indol-5-yl)propyl]pipéridin-4-yl}-1H-indole-5-carboxamide ;
s) le 5-[3-(4-quinoléin-8-ylpipérazin-1-yl)propyl]-1H-indole-3-carbonitrile ;
t) le 5-{3-[4-(2,3-dihydrobenzo-1,4-dioxin-5-yl)pipérazin-1-yl]-propyl}-1H-indole-3-carbonitrile ;
u) le 1-méthanesulfonyl-5-[3-(4-oxo-1-phényl-1,3,8-triazaspiro[4.5]-déc-8-yl)propyl]-1H-indole-3-carbonitrile ;
v) le 5-{3-[4-(1H-indol-4-yl)pipérazin-1-yl]propyl}-1H-indole-3-carbonitrile ;
w) le 5-{3-[4-(1H-indol-3-yl)pipéridin-1-yl)propyl}-1H-indole-3-carbonitrile ;
x) le 5-{3-[4-(5-fluoro-1H-indol-3-yl)pipéridin-1-yl]propyl}-1H-indole-3-carbonitrile ;
y) le 3-{1-[3-(3-cyano-1H-indol-5-yl)propyl]pipéridin-4-yl}-1H-indole-5-carbonitrile ;
z) le 5-{3-[4-(6-fluoro-1H-indol-3-yl)pipéridin-1-yl]propyl}-1H-indole-3-carbonitrile ;
aa) le 5-{3-[4-(4-fluoro-1H-indol-3-yl)pipéridin-1-yl]propyl}-1H-indole-3-carbonitrile ;
bb) le 5-[3-(4-benzo[d]isothiazol-3-ylpipérazin-1-yl)propyl]-1H-indole-3-carbonitrile ;
cc) le 4-{1-[3-(3-cyano-1H-indol-6-yl)propyl]pipéridin-4-yloxy}benzamide ;
dd) le 6-{3-[4-(2-cyano-3-méthoxyphényl)pipérazin-1-yl]propyl}-1H-indole-3-carbonitrile ;
ee) le 6-{3-[4-(4-cyano-3-méthoxyphényl)pipérazin-1-yl]propyl}-1H-indole-3-carbonitrile ;
ff) le 6-{3-[4-(4-cyano-2-méthoxyphényl)pipérazin-1-yl]propyl}-1H-indole-3-carbonitrile ;
gg) le 4-[3-(4-pyrazol-1-ylméthyl-1-pipéridyl)propyl]-1H-indole-3-carbonitrile ;
hh) le N-(6-{4-[3-(3-cyano-1H-indol-5-yl)propyl]pipérazin-1-yl}-2-oxo-2H-chromén-3-yl)acétamide ;
ii) le 5-{3-[(pyridin-3-ylméthyl)amino]propyl}-1H-indole-3-carbonitrile ;
jj) le 5-{3-[4-(2,3-dihydrobenzo-1,4-dioxin-6-yl)pipérazin-1-yl]-propyl}-1H-indole-3-carbonitrile ;
kk) le 5-[3-(4-pyrimidin-2-ylpipérazin-1-yl)propyl]-1H-indole-3-carbonitrile ;
ll) le 5-{3-[4-(3-méthoxyphényl)-3-méthylpipérazin-1-yl]propyl}-1H-indole-3-carbonitrile ;
mm) le 5-{3-[4-(1-méthyl-1H-imidazo[4,5-c]pyridin-4-yl)pipérazin-1-yl]-propyl}-1H-indole-3-carbonitrile ;
nn) le N-(4-{1-[3-(3-cyano-1H-indol-5-yl)propyl]pipéridin-4-ylméthyl}-phényl)acétamide ;
oo) le 5-{3-[4-(4-pyridin-3-ylthiazol-2-yl)pipérazin-1-yl]propyl}-1H-indole-3-carbonitrile ;
pp) le 2-{4-[3-(3-cyano-1H-indol-5-yl)propyl]pipérazin-1-yl}thiazole-4-carboxylate d'éthyle ;
qq) le 5-{3-[3-(2-oxopyrrolidin-1-yl)propylamino]propyl}-1H-indole-3-carbonitrile ;
rr) le 5-{3-[4-(3-amino-2-oxo-2H-chromén-6-yl)pipérazin-1-yl]-propyl}-1H-indole-3-carbonitrile ;
ss) le (6-{4-[3-(3-cyano-1H-indol-5-yl)propyl]pipérazin-1-yl}-2-oxo-2H-chromén-3-yl)carbamate de méthyle ;
tt) le 2-{4-[3-(3-cyano-1H-indol-5-yl)propyl]pipérazin-1-yl}thiazole-4-carboxamide ;
uu) le 4-[3-(3-cyano-1H-indol-5-yl)propyl]pipérazine-1-thiocarboxamide ;
et les sels et solvats de ceux-ci.

6. Procédé de préparation de composés de formule la selon la revendication 1 et de sels de ceux-ci, **caractérisé en ce que**
a) un composé de formule II 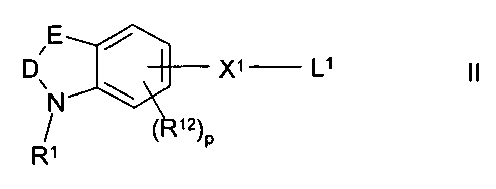 dans laquelle
L¹ désigne CI, Br, I, OH, un groupement OH estérifié de manière réactive, choisi parmi des groupements alkylsulfonyloxy ayant 1- 6 atomes de C et des groupements arylsulfonyloxy ayant 6-10 atomes de C, ou un groupement diazonium, et R¹, D, E, R¹², p et X¹ revêtent les significations indiquées selon la revendication 1,
b) est réagi avec un composé de formule III 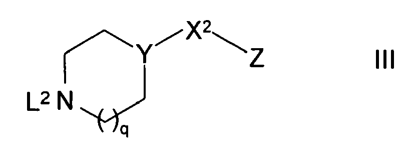 dans laquelle
L² désigne H ou un ion métallique, et q, Y, X² et Z revêtent les significations indiquées selon la revendication 1,
et éventuellement
c) le composé résultant de formule la est converti en l'un de ses sels par traitement par un acide.

7. Composition pharmaceutique, **caractérisée par** une teneur en au moins un composé de formule la selon la revendication 1 et/ou l'un de ses sels physiologiquement acceptables et/ou l'un de ses solvats.

8. Composés de formule la selon la revendication 1, et les sels et solvats physiologiquement acceptables de ceux-ci comme médicaments.

9. Utilisation des composés de formule la selon la revendication 1, pour la préparation d'un médicament destiné à la prophylaxie et/ou à la thérapie de maladies dans lesquelles la 5HT joue un rôle, **caractérisée en ce que** les maladies sont choisies dans le groupe constitué par la dépression, les accidents vasculaires cérébraux, l'ischémie cérébrale, les effets secondaires sur le système moteur extrapyramidal induits par des neuroleptiques et par la maladie de Parkinson, la maladie d'Alzheimer, la sclérose latérale amyotrophique, les traumatismes du cerveau et de la moelle épinière, les troubles obsessionnels-compulsifs, les troubles du sommeil, la dyskinésie tardive, les troubles d'apprentissage, les troubles de mémoire liés à l'âge, les troubles de l'alimentation et les dysfonctionnements sexuels.

10. Utilisation des composés de formule la selon la revendication 1 et/ou de sels ou solvats physiologiquement acceptables de ceux-ci, pour la préparation d'un médicament destiné à la prophylaxie et/ou au traitement de la schizophrénie, de la dépression, de la démence, de la maladie de Parkinson, de la maladie d'Alzheimer, de la démence à corps de Lewy, de la chorée de Huntington, du syndrome de Tourette, de l'anxiété, des déficiences de l'apprentissage et de la mémoire, des maladies neurodégénératives, des troubles cognitifs, ainsi que de la dépendance à la nicotine et des douleurs.

11. Composés de formule la selon la revendication 1, et/ou les sels physiologiquement acceptables de ceux-ci, pour une utilisation dans la lutte contre les maladies cérébrovasculaires, l'épilepsie, la schizophrénie, la maladie d'Alzheimer, la maladie de Parkinson ou la chorée de Huntington, l'ischémie cérébrale, l'infarctus ou les psychoses.

12. Procédé de préparation de compositions pharmaceutiques, **caractérisé en ce qu'**un composé de formule la selon la revendication 1 et/ou l'un de ses sels physiologiquement acceptables et/ou l'un de ses solvats est converti en une forme de dosage convenable conjointement avec au moins un excipient ou adjuvant solide, liquide ou semi-liquide.

13. Utilisation de composés de formule II 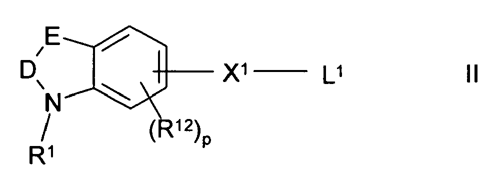 dans laquelle
L¹ désigne CI, Br, I, OH, un groupement OH estérifié de manière réactive, choisi parmi des groupements alkylsulfonyloxy ayant 1- 6 atomes de C et des groupements arylsulfonyloxy ayant 6-10 atomes de C, ou un groupement diazonium, et R¹, D, E, R¹², p et X¹ revêtent les significations indiquées selon la revendication 1,
pour la préparation de composés de formule la selon la revendication 1.

14. Utilisation de composés de formule III 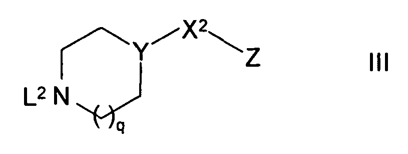 dans laquelle
L² désigne H ou un ion métallique, et q, Y, X² et Z revêtent les significations indiquées selon la revendication 1,
pour la préparation de composés de formule la selon la revendication 1.
